# EUROPÄISCHE PATENTANMELDUNG

(11) **EP 4 062 919 A1**
(43) Veröffentlichungstag der Anmeldung: **28.09.2022**
(21) Anmeldenummer: 22163510.5
(22) Anmeldetag: 22.03.2022
(51) Int. Cl.: A61K 31/728, A61K 33/14, A61P 43/00

(54) **EINE ODER MEHRERE ZUSAMMENSETZUNGEN AUF BASIS DES MENSCHLICHEN FRUCHTWASSERS ZUR ANWENDUNG IN EINEM VERFAHREN ZUR ENTERALEN ERNÄHRUNG, ZUR ENTERALEN ERNÄHRUNGSERGÄNZUNG UND/ODER ZUR BEHANDLUNG UND/ODER VERSORGUNG VON KAPILLARARMEN SCHICHTEN**

(30) Priorität: 24.03.2021 EP 21000086
(71) Anmelder: Prenatal International GmbH, 06126 Halle (Saale) (DE)
(72) Erfinder: Tchirikov, Michael, 06126 Halle (Saale) (DE)
(74) Vertreter: Mai Besier

(57) **Zusammenfassung**

Eine oder mehrere Zusammensetzungen zur Anwendung in einem Verfahren zur enteralen Ernährung, zur enteralen Ernährungsergänzung und/oder zur Behandlung von kapillararmen Schichten mit einer Kapillardichte von 0 bis 50/mm², wobei die eine der mehrere Zusammensetzungen zusammengenommen die folgenden Komponenten (pro 1000 ml)

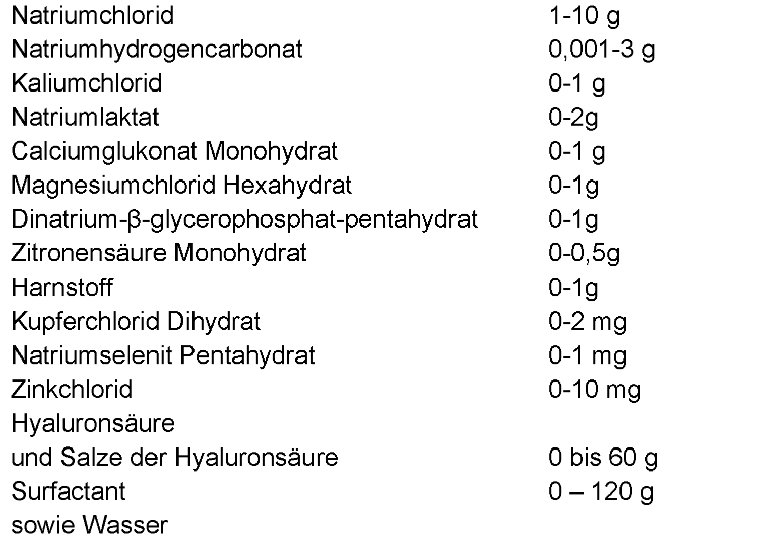

umfassen.

## Beschreibung

Die vorliegende Erfindung betrifft eine oder mehrere Zusammensetzungen auf Basis des menschlichen Fruchtwassers zur Anwendung bei einem Menschen oder einem Tier, insbesondere bei einem Menschen, in einem Verfahren zur enteralen Ernährung, zur enteralen Ernährungsergänzung und/oder zur Behandlung und/oder Versorgung von kapillararmen Schichten. Die Vorteile und Effekte der vorliegenden Erfindung werden vor allem bei der Behandlung des Sicca-Syndroms sowie bei der enteralen Ernährung, insbesondere extrem Frühgeborener, beobachtet.

Bereits in der 4. Schwangerschaftswoche umgibt das Fruchtwasser den menschlichen Embryo. In der Frühschwangerschaft gehören das Amnion- und Chorionepithel und die Nabelschnur zur Hauptbildungsstätte für das Fruchtwasser, wobei gleichzeitig eine aktive sekretorische und resorptive Tätigkeit der Fruchthüllen angenommen wird.

In der zweiten Hälfte der Schwangerschaft tritt die aktiv-sekretorische Leistung des Amnionepithels weiter in den Hintergrund und es verbleibt lediglich die Transsudation vom mütterlichen Plasma durch das Amnion- und Chorionepithel als wesentliche Bildungsart des Fruchtwassers (LIND et al. 1972, SEEDS). Im zweiten Trimester nehmen zusätzlich die fetalen Nieren eine wichtige Rolle als Entstehungsort für das Fruchtwasser ein; so fand PRITCHARD (1965) am Ende der Schwangerschaft eine Gesamturinmenge von 450 ml pro 24 Stunden, die in das Fruchtwasser abgegeben wird. HUTCHINSON et al. (1959) und PLENTL (1961) veröffentlichten Ergebnisse, dass die Nabelschnur 40 bis 50 ml Flüssigkeit pro Stunde in die Amnionhöhle abgibt. Mit der weiteren Ausreifung der fetalen Organanlagen kommt dann in der 2. Hälfte der Schwangerschaft auch noch das Tracheobronchialsystem als Mitbildungsort für Fruchtwasser in Frage. (Paper 1975).

Zusammengefast, sind in der 2. Hälfte der Schwangerschaft folgende Quellen für die Fruchtwasserbildung verantwortlich: die fetalen Nieren, Lungen und Trachealepithel, amniale Membran, Plazenta, fraglich die Nabelschnur und wahrscheinlich die fetale Schleimhaut und Haut, besonders vor dem Abschluss der Hornschichtbildung (Tchirikov et al. 2018; Gilbert et al. 1991; Beall et al. 2007).

Das Fruchtwasser hat während der Schwangerschaft mehrere Funktionen. Es dient als stoßpufferndes Schutzkissen und ermöglicht dem wachsenden Fötus schwerelose Bewegungen, hat eine wichtige Funktion in der Entwicklung der fetalen Lunge, Gastrointestinaltrakt und ernährt die kapillararmen oder kapillarlosen Schichten und Organe, wie z.B. amniale Membran, Nabelschnur, Kornea und äußere Schichten der Haut und Schleimhaut (Tchirikov et al. 2018; Lwigale 2015; Schmidt et al. 2010). (s. Mulvihill SJ et al. Trophic Effect of Amniotic Fluid on Fetal Gastrointestinal Development. J Surgical Research 1986;40:291-296).

Die oben genannten Organe und Schichten sind für die Entstehung, Entwicklung und weitere Differenzierung im Fruchtwasser genetisch über Millionen Jahren der Evolution des Menschen programmiert.

Die aktuelle Forschung befasst sich mit dem Nutzen (z.B. im Falle einer Ernährung oder Ernährungsergänzung) oder Reaktivierung und Unterstützung der Regenerationsprogramme von erkrankten, veralteten oder beschädigten Organen oder Schichten von Menschen und Tieren durch das Fruchtwasser ähnliche Komposition.

Über die Hypoosmolarität des Fruchtwassers (ca. 270-285 mOsm) besteht eine physiologisch bedingte osmotische Verschiebung der Wasserversorgung zum Gunsten von fetalen Oberflächen, Schleimhäuten, amnialer Membran, Plazenta und Nabelschnur (Gilbert und Brace 1993). Gilbert und Brace konnte im Tierversuch beweisen, dass das destillierte Wasser über die Haut, Schleimhaut und amniale Membran "intramembranöser Weg" aus der Amnionhölle von den Feten mit dem ligierten Ösophagus schnell aufgenommen wird (Gilbert und Brace 1993).

Die Amnionflüssigkeit wird vom Fetus durch das Schlucken (ca. 60-300 ml/kg/d) (Gilbert und Brace 1993; Beall et al. 2007) und über die intramembranöse Absorbtion der amnialen Membran (ca. 350-300 ml/kg/d) aufgenommen. Der getrunkene Volumenanteil wird vom fetalen Darm resorbiert. Ein Teil wird über den plazentaren Kreislauf an die mütterliche Blutbahn abgegeben (Beall et al. 2007). Mit der Entwicklung der Nieren wird ein zunehmender Anteil als fetaler Urin wieder in die Fruchtblase abgegeben. Fehlendes Fruchtwasser, z.B. nach dem klassischen Blasensprung, führt oft zu einer Lungenhypoplasie und Knochendeformationen (Tchirikov et al. 2018).

Das Fruchtwasser hat eine komplizierte Zusammensetzung. Hauptsächlich (>98%) besteht das Fruchtwasser aus dem Wasser und Elektrolyten. Das humane Fruchtwasser hat einen leicht alkalischen pH-Wert von 7,0-8,0 und eine Osmolatität von ca. 270-285 mOsm (Beall et al. 2007; Benzie et al. 1974, Tchirikov et al. 2018).

Zwischen der 22+0 Schwangerschaftswoche (SSW) und 34+0 Schwangerschaftswoche wird eine leichte Dynamik der Elektrolyten beobachtet. So nimmt die Natrium-Konzentration von 135,3 mmol/l in der 22+0 SSW auf 128,2 mmol/l leicht ab. In der 40+0 SSW liegt der Na⁺ Wert bei 125,6 mmol/l (Benzie RJ et al Am J Obstet Gynecol 1974). Die Chlorid-Ionen Konzentration hat ähnliche Tendenz (111.8 mmol/l in der 22+0 SSW auf 103,7 mmol/l in der 34+0 SSW). Die Kalium-Ionen Konzentration bleibt relativ bei 4,2±0,2 mml/l stabil. Die CO₂-Konzentratuion liegt zwischen der 15. und 31. SSW bei 20,8 bis 21,8 mmol/l (Benze RJ et al.). Die Glukose-Konzentration des menschlichen Fruchtwassers ist zwei bis dreifach geringer als im mütterlichen Plasma (rage 36,4 bis 49,8 mg/dl). Die Harnstoff-Konzentration nimmt während der Schwangerschaft von 4,2 mg/dl in der 22+0 SSW auf 6,2 mg(dl in der 34+0 SSW leicht zu. In einer sehr geringeren Konzentration sind andere Komponenten im Fruchtwasser präsent, wie z.B. Wachstumsfaktoren (TGA alpha, TGF beta-1. IGF-1, EPO, G-CSF, M-CSF; placental growth factor, angiogenic growth factors, beta 2-microglobulin), Proteine (lactoferrin, secretory leukocyte protease inhibitor, psoriasin, cathelicidin, alpha fetoprotein), Vitamine, Mikroelemente, andere Elemente, wie creatinine, bilirubin, hemoglobin, porphyrin, Aminosäuren, Hormone, Prostaglande, pepsinogen, adolase, amino-tripeptidase, carboxy peptidase, cathepsin, beta-glucuronidase, histaminase, phosphohexose isomerase, serotonin oxidase, alkaline phosphatase, alpha-keto-glutsric acid, piruvat acid, cholesterol, cholinesterase, acetone bodies, Hyaluonsäure und Surfactant (Nyman et al. 2013; Tchirikov et al. 2018; Dahl et al. 1983). Ab der 22.-24. Schwangerschaftswoche nimmt die Surfactant-Konzentration durch die Sekretion von Pneumozyten Typ-II signifikant zu. Surfactant spielt eine wichtige Rolle in der Entwicklung und Funktionalität der Lunge nach der Geburt und ist in der äußeren Schicht der Trennflüssigkeit nachgewiesen.

Während der embryonalen und fetalen Entwicklung spielt das Fruchtwasser für die kapillararmen Organen und deren äußeren Schichten eine essenzielle Rolle.

In Gegensatz zur Blutversorgung der meisten kapillarreichen menschlichen und tierischen Organe brauchen kapillararme Schichte, besonders Amnion und Kornea eine zusätzliche Quelle der Versorgung.

Als Referenzorgan gilt oft die Haut mit der Kapillardichte zwischen 55 bis 140 Kapillare/mm², (Mittelwert 90-100 Kapillare/mm²). Es hängt auch von der benutzen Methode stark ab. Bei Erkrankungen, z.B. Herz-Kreislauferkrankungen oder Psoriasis sinkt die Kapillardichtigkeit der Haut auf 40-50 Kapillare/mm² ab (Barcelous et al. 2018, Liang et al. 2019, Micali et al. 2021). Cornea und die amniale Membran haben normalerweise keine Kapillare. Damit können die Schichten mit einer Kapillardichte zwischen 0 bis 50/mm², bevorzugt zwischen 0 bis 30/mm² als kapillararm betrachtet werden.

Lichtmikroskopisch kann man während der Frühschwangerschaft lediglich eine Schicht im Bereich des Amnionepithels unterscheiden; im weiteren Verlauf der Entwicklung der Schwangerschaft finden sich nach BOURNE insgesamt 5 unterscheidbare Schichten.
a) Das kubisch bis zylindrische Amnionepithel: Die Zellen haben in der Regel einen einzigen Zellkern und liegen dicht der darunterliegenden Basalmembran auf. An ihrer freien, der Amnionhöhle zugewandten Seite erscheinen sie konvex. Im Inneren der Zellen finden sich Vakuolen verschiedener Größe. Zwischen den einzelnen Zellen lässt sich ein System von kleinsten interzellulären Kanälchen nachweisen.
b) Die Basalmembran besteht aus einer dünnen Schicht von retikulären Fasern, die in einem engen Kontakt mit den darüberliegenden Epithelzellen stehen.
c) Die Kompakta, die ebenfalls aus einem sehr dichten Netzwerk von retikulären Fasern besteht; sie stellt wohl die auf Zug am meisten belastbare Schicht dar.
d) Die Fibroblastenschicht, die sich aus einem lockeren Gewirk von Fibroblasten zusammensetzt, die in Retikulinfäserchen eingebettet sind.
e) Als letzte Schicht findet sich die Spongiosa, die sich aus den Resten der extraembryonalen Zölomhöhle zusammensetzt, die zwischen Chorion und Amnion zusammengepresst wurde. Insgesamt ergibt sich ein dreidimensionales Retikulinfasernetz, worin sich vereinzelt Fibroblasten finden lassen. In dieser Schicht kommt es häufig zu einer ödematösen Auflockerung, was für die Verdickung des Amnions verantwortlich ist. Gleichzeitig wird hier die Verschiebbarkeit des Amnions gegen das der Dezidua fest aufsitzende Chorion ermöglicht (Brusis et al. 1975).

Im gesamten Bereich des Amnions finden sich keine Blutgefäße; ebenso hat sich bis heute kein Hinweis für eine eigene Nervenversorgung ergeben.

Die amniale Membran ist bis zur 13.-16. Schwangerschaftswoche mit der choriallen Membran nicht verschmolzen. Die Ernährung der Amniozyten der Membran wird über die intramembranöse Absorbtion des Fruchtwassers sichergestellt ((Brace RA. 1997 und 2014, Tchirikov et al. 2018).

Ähnlich wird die äußere Schicht der Nabelschnur, die Cornea und teilweise die fetale Haut, Schleimhaut und Gastrointestinaltrakt über das Fruchtwasser versorgt.

Nach der Geburt bleibt das menschliche Epithelium der Cornea weiterhin nicht verhornt und wird nur über die Tränen versorgt.

Die Haut ist funktionell das vielseitigste Organ eines menschlichen oder tierischen Organismus. Sie dient als Hüllorgan der Abgrenzung von Innen und Außen, dem Schutz vor Umwelteinflüssen und der Wahrung einer Homöostase (inneres Gleichgewicht). Des Weiteren übernimmt die Haut wesentliche Funktionen im Bereich des Stoffwechsels, der Wärmeregulation und der Immunantwort; sie verfügt über vielfältige Anpassungs- und Abwehrmechanismen. Die äußere Haut gliedert sich in drei wesentliche Schichten: Epidermis (Oberhaut), Dermis und Subcutis. Dabei bilden Epidermis und Dermis zusammen die Cutis.

Als Schleimhaut, in der medizinischen Nomenklatur *Tunica mucosa* oder kurz Mukosa genannt, wird die Schutzschicht bezeichnet, die das Innere von Hohlorganen auskleidet. Im Gegensatz zur äußerlichen Haut besitzt sie keine Hornschicht und keine Haare. Wesentlich (und namensgebend) für Schleimhäute ist die Produktion von Schleimstoffen, den Mucinen.

Eine Schleimhaut besteht aus einer Epithelschicht (*Lamina epithelialis mucosae*)*,* einer epithelialen Muskelschicht (*Lamina muscularis mucosae*) und einer dazwischenliegenden Schicht aus Bindegewebe (*Lamina propria mucosae*)*.*

Das Epithel kann einschichtig (z. B. Darm) oder mehrschichtig (z. B. Mundhöhle) sein. Bei einigen Organen kann das Epithel oberflächlich auch eine spezifische Verhornung zeigen (z. B. Vormägen der Wiederkäuer). Häufig findet man auf den Epithelzellen Oberflächenvergrößerungen in Form von Mikrovilli, zum Teil auch Kinozilien und Stereozilien.

Die Eigenschicht enthält zumeist Drüsen (Drüsenschleimhaut), die die Schleimhaut feucht halten.

Schleimhäute dienen der mechanischen Abgrenzung der Organoberfläche. Viele Schleimhäute haben die Eigenschaft, durch aktive Transportproteine (z. B. Glucosetransporter) an der Schleimhautoberfläche Moleküle in eine bestimmte Richtung zu transportieren, und ermöglichen somit sowohl Sekretions- als auch Resorptionsprozesse. In der Eigenschicht finden sich häufig Lymphknötchen. Schleimhäute können Immunglobuline (vor allem IgA) absondern und haben so eine wichtige Schutzfunktion gegen eindringende Krankheitserreger.

Kapillaren sind die kleinsten Blutgefäße. Sie sind etwa 0,5 mm lang und haben einen Durchmesser von 5 bis 10 µm. Sie bilden ein feines Netzwerk in den Organen und Geweben des Körpers und ermöglichen den Stoffaustausch zwischen Blut und Gewebe. Der Durchmesser der Lungenkapillaren ist gerade so groß, dass rote Blutkörperchen hintereinander hindurchpassen.

Mit Sicca-Syndrom wird ein beschreibender (deskriptiver) Symptomkomplex bezeichnet, der im weitesten Sinne eine Benetzungsstörung verschiedener Organstrukturen der Augen, der Nase und des Mundraumes darstellt und so zu einer Austrocknung führt. Der Ausdruck Sicca-Syndrom dient als Bezeichnung für
∘ das "Trockene Auge" (*Keratoconjunctivitis sicca*)*,* auch als Syndrom des trockenen Auges bezeichnet: Ein durch Trockenheit der Augen gekennzeichnetes Krankheitsbild aus der Augenheilkunde. Typische Symptome sind Rötung und Brennen des Auges, einhergehend mit Fremdkörpergefühl, die durch eine Störung des Tränenfilms verursacht werden. Diese Störung des Tränenfilms kann auf unzureichender Produktion von Tränenflüssigkeit beruhen oder auf einem unvollständigen Aufbau des dreischichtigen Tränenfilms, u.a. der äußersten Surfactant-Schicht.
∘ Die Tränenflüssigkeit besteht aus einer wässrigen Mischung von Elektrolyten, Mikroelementen, Lipiden und Proteinen (Collin und Collin 2000; Millar und Schuett 2015; Rantamäki und Holopainen 2017). Die äußere Tränenschicht des Auges beinhalten Lipide, Surfactant, um u.a. das Verdampfen des Wassers der Tränenflüssigkeit zu vermeiden und die Tränenflüssigkeit zu stabilisieren. Ist der Fettfilm gestört und nicht mehr intakt, kann die darunterliegende wässrige Schicht schneller verdunsten.
∘ Surfactant macht 5-13% (im Mittel ca. 10%) der Fettschicht des Tränenfilms aus, wobei die Meibom-Drüsen nur ein Bruchteil davon synthetisieren (Cwiklik 2016).
∘ Die Reduktion der Surfactant-Konzentration in der Tränenflüssigkeit korreliert signifikant mit der Ausprägung des Sicca-Syndroms (trockene Augen). Die Surfactant-Proteine, wie Protein A, B und C sind sowohl im Lungen-Surfactant des Fruchtwassers als auch in der Tränenflüssigkeit präsent (Rantamäki und Holopainen 2017).
∘ Zur Therapie werden Lidrandhygiene, Tränenersatzmittel, liposomales Augenspray, Gel mit Hyaluronsäure und weitere Mittel eingesetzt. Wird die *Keratoconjunctivitis sicca* nicht behandelt, kann dies neben den anhaltenden, teilweise sehr belastenden Beschwerden zu einer ständigen Verstärkung der entzündlichen Prozesse am Auge führen, was zu einer vermehrten Trockenheit der Augen führt, wodurch die entzündlichen Prozesse weiter verstärkt werden können bis hin zu dauerhaften Schäden am Auge bei schweren Krankheitsverläufen.
∘ Eine entzündungsbedingte Trockenheit der Nase (*Rhinitis sicca*): Die *Rhinitis sicca* wird durch trockene Schleimhaut und deren Verkrustung gekennzeichnet. Die Atrophie der Schleimhaut und damit auch ihrer seromukösen Drüsen kann durch chronische Inhalation exogener Noxen, wie z. B. Kokain, zustande kommen oder infolge von Autoimmunerkrankungen wie dem *Sjögren*-Syndrom. Bei dieser Form der Rhinitis besteht eine Kontraindikation für die Anwendung α-sympathomimetischer Nasentropfen. Stattdessen werden Präparate zur Befeuchtung und Pflege der Nasenschleimhaut angewendet, wie Liposomsprays oder Nasensalben.
∘ Mundtrockenheit (*Xerostomie*)*,* d.h. die Trockenheit der Mundhöhle, die verschiedene Ursachen haben kann. Mundtrockenheit entsteht durch einen zu niedrigen Speichelfluss (Hyposalivation), welcher eine häufige Nebenwirkung von Arzneimitteln wie Anticholinergika, Diuretika oder Psychopharmaka ist. Ursachen können auch Erkrankungen wie die *Sialadenitis,* das *Sjögren-*Syndrom, das Sicca-Syndrom oder das Heerfordt-Syndrom sein. Der bei Mundtrockenheit entstehende hochvisköse Speichel kann Entzündungen in den Ausführungsgängen der Speicheldrüsen hervorrufen; besonders betroffen ist hierbei die Unterzungenspeicheldrüse (*Glandula sublingualis*). Die direkten (unangenehmen) Folgen sind die Trockenheit der Mundschleimhäute und Schluckbeschwerden. Damit ergeben sich Probleme beim Sprechen und beim Verzehr trockener Nahrung. Auch das Einschlafen kann erschwert sein. Chronisch Betroffene leiden meist hinsichtlich ihrer Zahngesundheit. Häufige Folge ist Karies in Abwesenheit der schützenden Begleitstoffe des normalen Speichelflusses. Der normale Mundspeichel enthält eine Reihe antimikrobiell wirksamer Bestandteile, u. a. Immunglobulin A (Antikörper), Lysozym (Enzym), Lactoferrin und Histatin (Protein). Eine systemische Therapie ist nicht bekannt. Symptomatische Therapie erfolgt durch Speichelersatz- bzw. Speichelergänzungsprodukte (benetzendes Gel, Aerosol, Mundwasser, Kaumittel), Mundauswischen mit feuchten Watteträgern, Trinken und Ausspülen mit Wasser, Tee (jedoch nicht austrocknendem Salbeitee), Kaffee, Säfte (insbesondere säuerliche, den Speichelfluss anregende), Auftragen von Vitaminpaste, Eiswürfel, Vernebler, ätherische Öle (Duftlampe mit Zitrusaroma).
∘ das *Sjögren*-Syndrom. (auch *Dacryo-Sialo-Adenopathia atrophicans* genannt): chronisch verlaufende Autoimmunerkrankung aus dem rheumatischen Formenkreis und der Gruppe der Kollagenosen, bei der bestimmte Immunzellen besonders die Speicheldrüsen und Tränendrüsen angreifen und zu weiteren entzündlichen Veränderungen an inneren Organen und am zentralen Nervensystem führen können. Das *Sjögren*-Syndrom manifestiert sich in morphologischen Veränderungen der Tränen- und Speicheldrüsen. Die Leitsymptome trockene Augen und Mundtrockenheit (*Xerostomie*) werden auch als Sicca-Syndrom bezeichnet. Es wird unterschieden zwischen primärem *Sjögren*-Syndrom (pSS) sowie sekundärem Sjögren-Syndrom (sSS), welches in Begleitung anderer Autoimmunerkrankungen auftreten kann, wie *Lupus erythematodes,* rheumatoider Arthritis oder systemischer Sklerose. Das anfängliche Auftreten einer Sicca-Symptomatik spricht für ein pSS.

Enterale Ernährung bedeutet Nahrungs- und Flüssigkeitszufuhr über Magen und Darm. Der Begriff wird überwiegend für die Sondenernährung mittels Magensonde, PEG- oder PEJ-Sonde, Jejunalsonde beziehungsweise JET-PEG verwendet.

Eine Magensonde (= Nasogastralsonde) beziehungsweise nasale Jejunalsonde ist ein Schlauch, der durch Nase oder Mund, Rachen und Speiseröhre in den Magen oder weiter bis in einen Teil des Dünndarms führt. Diese nasalen Sonden sind meist nur für einen kurzfristigen Einsatz konzipiert; ist eine längerfristige ernährungstherapeutische Intervention geplant (länger als vier Wochen), ist die Anlage einer perkutanen Sonde angezeigt.

Bei der perkutanen endoskopischen Gastrostomie (PEG) wird die Sonde - in der Regel endoskopisch geführt - direkt durch die Bauchdecke in den Magen gelegt.

Zur enteralen Ernährung geeignete Trinknahrungen und Nährstofflösungen enthalten die notwendigen Eiweiße, Kohlenhydrate, Fette, Vitamine, Mineralstoffe und Spurenelemente in optimaler Zusammensetzung. Sie müssen keimfrei und gebrauchsfertig sein. Der Nahrungsbehälter kann für eine Schwerkraftinfusion entweder direkt über ein Zuleitungssystem mit der Sonde verbunden werden, oder der Inhalt wird in ein passendes Beutelsystem umgefüllt.

Im Uterus trinken Feten zwischen 150 und 300 ml Fruchtwasser/kg (Gilbert WM, Brace RA. Amniotic fluid volume and normal flows to and from the amniotic cavity. Seminars in Perinatology 1993; 17(3): 150-7. Gilbert WM, Brace RA. Increase in fetal hydration during long-term intraamniotic isotonic saline infusion. American Journal of Obstetrics and Gynecology 1988; 159(6): 1413-7 [https://doi.org/10.1016/0002-9378(88)90566-2] [Gilbert WM, Cheung CY, Brace RA. Rapid intramembranous absorption into the fetal circulation of arginine vasopressin injected intraamniotically. American Journal of Obstetrics and Gynecology 1991; 164(4): 1013-1018; discussion 1018-1020 [https://doi.org/10.1016/0002-9378(91 )90576-d]

Durch die besonderen anatomischen Gegebenheiten des Kehlkopfes von Kleinkindern, wodurch Säuglinge noch gleichzeitig atmen und trinken können, gelangt Fruchtwasser sowohl in den Magen-Darm-Trakt als auch in die Lunge. Dies ist beim Fetus physiologisch bedingt und kann im Doppler-Ultraschall beobachtet werden. Das Fruchtwasser in der 24.-32. SSW beinhaltet eine hohe Konzentration von Surfactant (Robert Wenk 1981; Maya Bangyozova 2012).

Um den Zeitpunkt der Geburt sind Neugeborene besonders anfällig für die Entstehung von Entzündungen oder andere Erkrankungen. Dementsprechend geht dieser Zeitraum mit der höchsten Morbidität und Mortalität des gesamten Lebens einher (Gortner et al. 2012). Durch die extreme Unreife von Frühgeborenen erhöhen sich die Mortalitäts- und Morbiditätszahlen signifikant (Chen et al. 2016). Deswegen ist die medizinische Forschung für diese Kinder besonders wichtig. Infolge der Unreife des zentralen Nervensystems können des Weiteren Bradykardien und Apnoen auftreten. Ebenso sind intrakraniale Blutungen häufig. Diese drei Erscheinungen führen oft zu neurologischen Spätfolgen und einer erhöhten Sterblichkeit (Weyerstahl et al. 2013). Je besser diese größeren Komplikationen (major complications) behandelt werden können, desto geringer ist die Sterblichkeit bei den Neugeborenen. Das Auftreten der Hauptkomplikationen ist jedoch stark abhängig vom Gestationsalter (GA) zur Geburt. Je früher die Kinder zur Welt kommen, desto häufiger treten die Komplikationen auf und umso höher ist die Sterblichkeit auf Grund eines verkürzten Zeitabschnittes ohne Auftreten dieser (Chen et al. 2016).

Die Atemwege bei Frühgeborenen vor der 28. Schwangerschaftswoche (SSW) sind noch sehr unreif. Surfactant ist ein Stoffgemisch, aus Phospholipiden und Proteinen, das von den Pneumozyten Typ II produziert wird und oberflächenaktiv in der Lunge wirkt. Dadurch wird die Oberflächenspannung in den Lungenbläschen nach der Geburt reduziert (Hofmeyr et al. 2014). Ab der 22. SSW wird Surfactant in der Lunge des Fetus produziert (Gortner et al. 2012).

Bis zur 28. SSW ist die Konzentration des Surfactants noch mangelhaft. Somit erkranken jene Neugeborene häufig an einem Atemnotsyndrom. Aufgrund des mangelnden Surfactants ist die Oberflächenspannung in den Alveolen der Lunge zu hoch. Das führt in vielen Fällen zu Sättigungsabfällen und pulmonaler Hypertension. Es kann folgend zu einem Sauerstoffmangel des Gehirns, zu einem Pneumothorax oder anderen Komplikationen kommen. Als Spätfolge sind Fehlentwicklungen der Lunge und chronischen Lungenerkrankungen möglich (Gortner et al. 2012).

"In utero" wird Surfactant im Magen-Darm-Trakt aufgenommen und über die Blutbahn zu den Lungen transportiert und dann wieder in das Fruchtwasser abgegeben. Dadurch wird in einem Kreislauf immer wieder noch nicht abgebauter Surfactant mit dem Fruchtwasser vom Fetus getrunken. Daher ist die Konzentration des Surfactant im Fetus dauerhaft hoch und kann somit bis zum physiologischen Geburtstermin für eine gute Lungenentwicklung sorgen. Stark unreife fetale Leber, Pankreas und Darmtrakt sind bei extrem Frühgeborenen nicht in der Lage, eine suffiziente Menge der Phospholipide für die Surfactant-Produktion herzustellen. Das war wahrscheinlich von der Evolution nicht vorgesehen. Nach der Frühgeburt wird die enterale Versorgung mit dem Fruchtwasser und Surfactant akut komplett unterbrochen. Bisher wird bei der verfrühten Geburt dem extremen Frühgeborenen kein Fruchtwasser mit dem Surfactant enteral gegeben.

Um einen Surfactant-Mangel und RDS (respiratory disstress syndrome) nach der extremen Frühgeburt zu behandeln, wird kurz nach der Frühgeburt Surfactant über einen Katheter direkt in die Trachea gespritzt Richard A. Polin et al. ,Am Academic of Pediatrics 2014). Damit sollte die verbesserte Surfactant-Beschichtung von Alveolen erreicht werden, um eine Kollabierung von den kleinen Lungenbläschen nach dem Ausatmen zu verhindern. Insgesamt handelt sich bei Erwachsenen um ca. 300 Millionen Alveolen mit ca. 0,2 mm Durchmesser mit einer gesamten Fläche von ca. 100 m². Die Frühgeborenen haben signifikant weniger Alveolen. Nach dem Spritzen von z.B. 45 mg Surfactant (Alveofact, Lyomark Pharma, 45 mg/ml, 1,2 ml Aveofact-Lösung pro 1 kg Körpergewicht) oder 120 mg Curosurf, Chiesi GmbH, Suspension (1,5 ml) soll die intratracheal instillierte Flüssigkeit (1,2-1,5 ml) mit dem Surfactant aus der Trachea über das Bronchialsystems die Alveolen erreichen. Die Bronchien werden in ihrem Verlauf ab der Trachea immer kleiner bis sie in der Peripherie in den Alveolarsäcken, gebildet aus mehreren Lungenbläschen, enden. Der Bronchialbaum spaltet sich ca. 20-mal dichotom auf.

Je nach Stufe der Aufteilung unterscheidet man: Hauptbronchien → Lappenbronchien → Segmentbronchien → Subsegmentbronchien (Rami subsegmentales). Bis zum Durchmesser von ca. 1 mm erfolgen weitere Teilungen. Mit dem Verlust des Wandknorpels werden die bronchiale Röhrchen "Bronchiolen" genannt. Sie besitzen ein einschichtiges Flimmerepitel. Diese Bronchiolen teilen sich in 4 bis 5 Bronchioli terminales, die sich weiter in Brinchioli respiratori mit der Länge ca. 1 bis 3 mm und ca. 0,4 mm durchmessend teilen. Weiter kommen Ductus alveolares mit Alveolen (d=60-200µm). Mit den Bronchioli respiratorii beginnt der gasaustauschende Abschnitt der Lunge.

Die o.g. Beschreibung des langen Weges von der Trachea bis zu Alveolen stellt die Erklärung der therapeutischen Wirkung der intratrachealen Surfactant-Instillation in Frage. Surfactant kann mechanisch die Alveolen kaum erreichen. Dazu kommt die Entgegenwirkung der bronchialen Schleimhaut. Die Flimmerhärchen der Schleimhaut bewegen sich wellenartig in Richtung Rachen, sie schlagen durchgehend etwa 1000 Mal in der Minute. In der Luftröhre werden dabei Geschwindigkeiten von bis zu 1 cm pro Minute erreicht. Damit wird > 90% des intratracheal gespritzten Medikamentes mit dem Surfactant das Bronchialsystem verlassen und von dem Frühgeborenen verschluckt.

Wir gehen davon aus, dass das unreife Verdauungssystem des extrem frühgeborenen Kindes nicht in der Lage ist, verschlucktes Surfactant vollständig zu metabolisieren. Das verschluckte Surfactant begünstigt die Versorgung von Pneumozyten Typ II mit den Phospholipiden und dadurch die notwendige Surfactant-Synthese. Damit erklärt sich teilweise die positive therapeutische Wirkung der intratrachealen Surfactant-Instillation.

Die nekrotisierende Enterokolitis (NEC) ist die häufigste Ursache für das akute Abdomen bei Neugeborenen, besonders bei unreifen Frühgeborenen. Dabei kommt es meist in den ersten drei Lebenswochen zu Anzeichen einer transmuralen nekrotisierenden Entzündung der Darmwand. Diese beginnt zumeist im terminalen Ileum oder im Colon (Gortner et al. 2012) und kann verstreut oder auch kontinuierlich im gesamten Magen-Darm-Trakt vorkommen. Sie zeigt sich zum Beispiel mit einem geblähten Abdomen und schleimig-blutigen Stühlen.

Eine weitere Erkrankung extremer Frühgeborenen ist die spontane intestinale Perforation (SIP). (Mayr und Fasching 2018; Durell et al. 2017). Sie ist mit einer unreifen Funktionstüchtigkeit des Magen-Darm-Traktes und Problemen mit der Nahrungsverwertung begründet. Eine chirurgischen Intervention im Rahmen einer Laparotomie erhöht bei den Frühgeborenen < 26/0 SSW die Letalitäts- und Morbiditätsraten (Durell et al. 2017). Durell et al. beschreiben eine Mortalitätsrate von 30-31 % (Durell et al. 2017).

Die nekrotisierende Enterokolitis kann wie eine spontane intestinale Perforation in der Folge zu Hirnschädigungen bei den jungen Patienten führen. Schäden der weißen Substanz (white matter injury) können dabei im MRT gezeigt werden (Shin et al. 2016; Hinojosa-Rodríguez et al. 2017).

Durch die besonderen anatomischen Gegebenheiten des Kehlkopfes von Feten, gelangt Fruchtwasser sowohl in den Magen-Darm-Trakt als auch in die Lunge. Das ist beim Fetus physiologisch und kann im Doppler-Ultraschall beobachtet werden. Durch die mangelnde Reflexentwicklung der Frühgeborenen gelangt noch postnatal, dann pathologisch, Nahrung und Flüssigkeit in die Lunge, was zur Aspiration von Muttermilch oder anderer Säuglingsnahrung führen kann. Eine Aspiration führt zur kurzzeitigen Unterbrechungen der Sauerstoffversorgung des Gehirns, so dass neurologische Defizite oder Störungen bei Frühgeborenen oder als Spätfolgen gehäuft beobachtet werden (Espinheira et al. 2011). Analoge Auswirkungen kann die perinatale Asphyxie aufzeigen (Gortner et al. 2012).

Vorbeugend werden die betroffenen Frühgeborenen intubiert und beatmet (Weyerstahl et al. 2013; Gortner et al. 2012). Ein vollständiges Verhindern von Aspirationen ist jedoch nicht immer möglich, besonderes nach der Extubation. Nach der Entfernung des Tubus ist das Aufkommen von Aspirationen erhöht.

Das Fruchtwasser in der 24.-32. SSW beinhaltet eine relativ hohe Konzentration von Surfactant. "In utero" wird Surfactant im Magen-Darm-Trakt aufgenommen und über die Blutbahn über die Leber zur Lunge transportiert und dann wieder in das Fruchtwasser abgegeben. Der fetale Darm und die Leber haben intrauterin eine sehr eingeschränkte metabolische Funktion (Giancotti et al. 2019). Dadurch wird in einem Kreislauf immer wieder Surfactant mit dem Fruchtwasser vom Fetus getrunken und über Pneumozyten Typ-II über das tracheale System der Lunge zurück in das Fruchtwasser abgegeben. Daher ist die Konzentration des Surfactant im Fruchtwasser im III. Trimester dauerhaft hoch und kann somit bis zum physiologischen Geburtstermin für eine gute Lungenentwicklung sorgen.

Nach der Frühgeburt wird dem extremen Frühgeborenen kein Fruchtwasser mit dem Surfactant enteral gegeben. Der intrauterine physiologische Kreislauf der Surfactant-Metabolismus wird damit gestoppt und die intrauterine physiologische Lungenentwicklung vorzeitig beendet.

Wie zuvor dargestellt, bedarf es für die Herstellung von Surfactant Fettsäuren. Dabei entstammen maximal nur 50 % der Fettsäuren (FS) aus dem mütterlichen Plasma und nur zu einem geringen Anteil direkt aus der Plazenta (Coleman 1986). Über Lipoprotein Rezeptoren kann die Plazenta die mütterlichen Fette akkumulieren und an den Fetus weitergeben (Herrera et al. 2006). Für die Herstellung von Surfactant muss jedoch der Fetus selbst auch Lipide herstellen (Herrera et al. 2006). Unserer Abschätzung nach, stammen ca. 50% des fetalen Surfactants aus dem Fruchtwasser.

Zielparameter der enteralen Ernährung sind 110-135 kcal/kg/Tag Energie und 4,5 g/kg/Tag Protein. Die mittlere intrauterine Aufnahme von Zink zwischen 24 und 34 SSW wurde mit 850 µg/kg/Tag ermittelt.

Die Plazenta ist für die komplette Versorgung der Feten zuständig. Die Feten sind intrauterin nur für die enterale Einnahme des Fruchtwassers programmiert. Weder der Magen noch der Darm, die Pankreas oder die Leber sind für die Metabolisierung der Milch bei extrem Frühgeborenen < 1000 g, besonders < 750 g, vorbereitet. Die Einführung der Milch durch die Sonde führt bei den extrem Frühgeborenen nicht selten zu einem harten käseförmigen Stuhl und zur Darmperforation, NEC, Volvulus und "milk cord obstruction" Syndrom mit gravierenden Folgen (Durell et al. 2017; Viswanathan et al. 2015).

Bei extremer Frühgeburtlichkeit mit einem Geburtsgewicht < 1250g, besonders bei einem neonatalen Gewicht von weniger als 750 g (ca. 27. SSW), muss man in Frage stellen, ob den Neugeborenen mit einem extrem unreifen Magen-Darm-Leber-Pankreas-System Milch als Nahrung sofort nach der Geburt unverdünnt angeboten werden sollte. Die Feten sind intrauterin in diesem Gestationsalter nur für die Einnahme des Fruchtwassers mit Surfactant programmiert. Weder der Magen noch der Darm, die Pankreas oder die Leber sind nach der extremen Frühgeburt für die Verdauungsprozesse vorbereitet.

Viswanathan et al. (USA) konnten bei extrem Frühgeborenen < 750 g durch eine verzögerte Milchgabe um 14 Tage die Rate der NEC von 16.2 % auf 1.1 % signifikant reduzieren (Viswanathan et al. 2015). Auch die Morbiditätsrate wurde in der Studie dadurch von 29.5% auf 7.6 % reduziert (Viswanathan et al. 2015).

Eine unreife Leber kann und wahrscheinlich auch soll bei den Feten mit ≤750g die Surfactant-Fraktionen nicht metabolisieren. Damit können die Surfactant-Fraktionen die Pneumozyten Typ II fast unverändert erreichen. Surfactant wird in das Fruchtwasser extrahiert und von dem Fetus innerhalb von 2-4 Tagen ausgetrunken. Dann fängt der Surfactant-Kreis von vorne an. Durch die Frühgeburt wird er unterbrochen.

Im Mutterleib gibt es viele pathologische Zustände, bei denen das Fruchtwasser nicht ausreichend produziert wird, grobe Abweichungen von der physiologischen Zusammensetzung hat (nach dem Mekoniumabgang, Blutung) oder z.B. nach dem klassischen Blasensprung fehlt. Vom früheren vorzeitigen Blasensprung (engl.: Premature Preliminary Rupture of Membranes, PPROM) spricht man, wenn bereits vor der abgeschlossenen 37. Schwangerschaftswoche das Fruchtwasser aufgrund eines Risses in der Amnionhöhle abgeht.

Das Vorliegen eines Anhydramnion in der ersten Hälfte der Schwangerschaft, besonders vor der 24. SSW, ist mit der Entstehung einer Lungenhypoplasie stark korreliert. Die Sterblichkeitsrate ist in solchen Fällen über 80%. Die veränderte Zusammensetzung des Fruchtwassers durch das Mekonium oder Blut zerstört eine physiologische Ernährung der amnialen Membran und erhöht das Risiko eines vorzeitigen Blasensprunges. Eine Aspiration des Fruchtwassers mit Mekonium erhöht signifikant das Risiko eines RDS (resperatory disstress syndrome) und Sauerstoff-Unterversorgung des Neugeborenen mit dramatischen Folgen,
Tchirikov et al. haben eine Behandlung des PPROM mit einer kontinuierlichen Amnioinfusion mit einer Amnion Flush Solution (Serumwerk AG, Bernburg, Deutschland) beschrieben (Tchirikov et al., 2015, 2018). Damit konnte die PPROM-Entbindungsdauer auf 49 Tagen verlängert werden. Eine Kombination der Amnion Flush Solution mit dem Surfactant, Hyaluronsäure, Vitaminen, vervollständigen Mikroelementen wurde nicht beschrieben. Auch die Anwendung der Amnion Flush Solution beim Anhydramnion anderer Ursache wurde auch nicht erwähnt.

### Literaturverzeichnis:

Beall, M. H.; van den Wijngaard, J. P. H. M.; van Gemert, M. J. C.; Ross, M. G. (2007): Regulation of amniotic fluid volume. In: Placenta 28 (8-9), S. 824-832. DOI: 10.1016/j.placenta.2006.12.004.
Benzie RJ et al. Composition of the amniotic fluid and matarnal serum in pregnancy. Am J Obstet Gynecol 1974;119:798-810.
Bolisetty, Srinivas; Osborn, David; Schindler, Tim; Sinn, John; Deshpande, Girish; Wong, Chee Sing et al. (2020): Standardised neonatal parenteral nutrition formulations - Australasian neonatal parenteral nutrition consensus update 2017. In: BMC pediatrics 20 (1), S. 59. DOI: 10.1186/s12887-020-1958-9.
BOURGNE G.L. The human amnion and chorion. Loyd-Luke, London 1962 Bourgne G-L. The microscopic anatomy of the human amnion and chorion. Am. J. Ostet Gynecol 1960;79:1070.
Brace RA. Physiology of amniotic fluid volume regulation. Clin Obstet Gynecol 1997;40:280-289
2. Brace RA et al. Regulation of amniotic fluid volume: evolving concepts. In Zhang L and C.A. Ducsay (eds.), Advances in Fetal and Neonatal Physiology, Advances in Experimental Medicine and Biology. 2014; 814
3. Brace RA et al. Normal amniotic fluid volume changes throughout pregnancy. Am J Obstet Gynecol 1989;161:382-388
Brusis E, Nitsch B, Wengeler H. Fruchtwasser und Amnion. Klinik der Frauenheilkunde. 1975;Band IV, S.666-750.
Brusis et al. Fruchtwasser und Amnion. Klinik der Frauenheilkunde und Geburtshilfe. 1975; 4. Bald, 667-750.
Cancarini, A.; Fostinelli, J.; Napoli, L.; Gilberti, M. E.; Apostoli, P.; Semeraro, F. (2017): Trace elements and diabetes: Assessment of levels in tears and serum. In: Experimental eye research 154, S. 47-52. DOI: 10.1016/j.exer.2016.10.020. Chen, Fei; Bajwa, Nadia M.; Rimensberger, Peter C.; Posfay-Barbe, Klara M.; Pfister, Riccardo E. (2016): Thirteen-year mortality and morbidity in preterm infants in Switzerland. In: Archives of disease in childhood. Fetal and neonatal edition 101 (5), F377-83. DOI: 10.1136/archdischild-2015-308579.
Coleman, R. A. (1986): Placental metabolism and transport of lipid. In: Federation proceedings 45 (10), S. 2519-2523.
Collin, H. B.; Collin, S. P. (2000): The corneal surface of aquatic vertebrates: microstructures with optical and nutritional function? In: Philosophical transactions of the Royal Society of London. Series B, Biological sciences 355 (1401), S. 1171-1176. DOI: 10.1098/rstb.2000.0661.
Cwiklik, Lukasz (2016): Tear film lipid layer: A molecular level view. In: Biochimica et biophysica acta 1858 (10), S. 2421-2430. DOI: 10.1016/j.bbamem.2016.02.020.
Dahl, L.; Hopwood, J. J.; Laurent, U. B.; Lilja, K.; Tengblad, A. (1983): The concentration of hyaluronate in amniotic fluid. In: Biochemical medicine 30 (3), S. 280-283. DOI: 10.1016/0006-2944(83)90018-2.
Durell, Jonathan; Hall, Nigel J.; Drewett, Melanie; Paramanantham, Kujan; Burge, David (2017): Emergency laparotomy in infants born at <26 weeks gestation: a neonatal network-based cohort study of frequency, surgical pathology and outcomes. In: Archives of disease in childhood. Fetal and neonatal edition 102 (6), F504-F507. DOI: 10.1136/archdischild-2016-312195.
Espinheira, M. C.; Grilo, M.; Rocha, G.; Guedes, B.; Guimaräes, H. (2011): Meconium aspiration syndrome - the experience of a tertiary center. In: Revista portuguesa de pneumologia 17 (2), S. 71-76.
Gephart, Sheila M.; Hanson, Corrine; Wetzel, Christine M.; Fleiner, Michelle; Umberger, Erin; Martin, Laura et al. (2017): NEC-zero recommendations from scoping review of evidence to prevent and foster timely recognition of necrotizing enterocolitis. In: Maternal health, neonatology and perinatology 3, S. 23. DOI: 10.1186/s40748-017-0062-0.
Giancotti, Antonella; Monti, Marco; Nevi, Lorenzo; Safarikia, Samira; D'Ambrosio, Valentina; Brunelli, Roberto et al. (2019): Functions and the Emerging Role of the Foetal Liver into Regenerative Medicine. In: Cells 8 (8). DOI: 10.3390/cells8080914.
Gilbert, W. M.; Brace, R. A. (1993): Amniotic fluid volume and normal flows to and from the amniotic cavity. In: Seminars in perinatology 17 (3), S. 150-157.
Gilbert, W. M.; Cheung, C. Y.; Brace, R. A. (1991): Rapid intramembranous absorption into the fetal circulation of arginine vasopressin injected intraamniotically. In: American journal of obstetrics and gynecology 164 (4), 1013-8; discussion 1018-20.
Gortner, Ludwig; Meyer, Sascha; Sitzmann, Friedrich C. (2012): Pädiatrie. 304 Tabellen; [plus DVD mit Videofilmen]. Unter Mitarbeit von Ludwig Gortner, Sascha Meyer und Friedrich C. Sitzmann. 4., vollst. überarb. und erw. Aufl. Stuttgart: Thieme (Duale Reihe).
Herrera, E.; Amusquivar, E.; López-Soldado, I.; Ortega, H. (2006): Maternal lipid metabolism and placental lipid transfer. In: Hormone research 65 Suppl 3, S. 59-64. DOI: 10.1159/000091507.
Hinojosa-Rodríguez, Manuel; Harmony, Thalía; Carrillo-Prado, Cristina; van Horn, John Darrell; Irimia, Andrei; Torgerson, Carinna; Jacokes, Zachary (2017): Clinical neuroimaging in the preterm infant: Diagnosis and prognosis. In: NeuroImage. Clinical 16, S. 355-368. DOI: 10.1016/j.nicl.2017.08.015.
Hofmeyr, G. Justus; Eke, Ahizechukwu C.; Lawrie, Theresa A. (2014): Amnioinfusion for third trimester preterm premature rupture of membranes. In: The Cochrane database of systematic reviews (3), CD000942. DOI: 10.1002/14651858.CD000942.pub3.
HUTCHINSON DL et al. The role of the fetus in the water exchange of the amniotic fluid of normal and hydramniotic patients. J Clin Invest 1959;38:971. LIND T et al. The role of the fetus in the formation of the amniotic fluid. J Obstet Gynecol Brit. Cwlth 1972;79:289.
Lindower, Julie B. (2017): Water balance in the fetus and neonate. In: Seminars in fetal & neonatal medicine 22 (2), S. 71-75. DOI: 10.1016/j.siny.2017.01.002. Lwigale, Peter Y. (2015): Corneal Development: Different Cells from a Common Progenitor. In: Progress in molecular biology and translational science 134, S. 43-59. DOI: 10.1016/bs.pmbts.2015.04.003.
Mayr, Johannes; Fasching, Günter (Hg.) (2018): Akutes Abdomen im Kindes- und Jugendalter. Grundlagen - Diagnose - Erstversorgung - Therapie. Springer-Verlag GmbH. 1. Auflage 2018. Berlin: Springer Berlin.
Millar, Thomas J.; Schuett, Burkhardt S. (2015): The real reason for having a meibomian lipid layer covering the outer surface of the tear film - A review. In: Experimental eye research 137, S. 125-138. DOI: 10.1016/j.exer.2015.05.002. Mulvihill SJ et al. Trophic Effect of Amniotic Fluid on Fetal Gastrointestinal Development. J Surg Research 1986;40:291-296.
Nyman, Erika; Huss, Fredrik; Nyman, Torbjörn; Junker, Johan; Kratz, Gunnar (2013): Hyaluronic acid, an important factor in the wound healing properties of amniotic fluid: in vitro studies of re-epithelialisation in human skin wounds. In: Journal of plastic surgery and hand surgery 47 (2), S. 89-92. DOI: 10.3109/2000656X.2012.733169.
PRITCHARD JA Deglutination by normal and anencephalic fetuses. Obstet Gynecol (N.Y.) 1965; 25:289.
PRITCHARD JA Fetal swallowing and amniotic fluid volume. Obstet Gynecol (N.Y.) 1966;28:606 (1965)
Rantamäki, Antti H.; Holopainen, Juha M. (2017): The Effect of Phospholipids on Tear Film Lipid Layer Surface Activity. In: Investigative ophthalmology & visual science 58 (1), S. 149-154. DOI: 10.1167/iovs.16-20468.
Richard A. Polin, MD, FAAP, Waldemar A. Carlo. Surfactant Replacement Therapy for Preterm and Term Neonates With Respiratory Distress. PEDIATRICS (ISSN Numbers: Print, 0031-4005; Online, 1098-4275).
Schmidt, Robert F.; Lang, Florian; Heckmann, Manfred (Hg.) (2010): Physiologie des Menschen: Mit Pathophysiologie; mit 85 Tabellen; mit herausnehmbarem Repetitorium. 31., überarb. und aktualisierte Aufl. Heidelberg: Springer-Medizin-Verl. (Springer-Lehrbuch).
Shin, Seung Han; Kim, Ee-Kyung; Yoo, Hani; Choi, Young Hun; Kim, Saeyun; Lee, Byoung Kook et al. (2016): Surgical Necrotizing Enterocolitis versus Spontaneous Intestinal Perforation in White Matter Injury on Brain Magnetic Resonance Imaging. In: Neonatology 110 (2), S. 148-154. DOI: 10.1159/000444387.
Tchirikov, Michael; Schlabritz-Loutsevitch, Natalia; Maher, James; Buchmann, Jörg; Naberezhnev, Yuri; Winarno, Andreas S.; Seliger, Gregor (2018): Midtrimester preterm premature rupture of membranes (PPROM): etiology, diagnosis, classification, international recommendations of treatment options and outcome. In: Journal of perinatal medicine 46 (5), S. 465-488. DOI: 10.1515/jpm-2017-0027.
Viswanathan, S.; Merheb, R.; Wen, Xintong; Collin, M.; Groh-Wargo, S. (2017): Standardized slow enteral feeding protocol reduces necrotizing enterocolitis in micropremies. In: Journal of neonatal-perinatal medicine 10 (2), S. 171-180. DOI: 10.3233/NPM-171680.
Viswanathan, Sreekanth; McNelis, Kera; Super, Dennis; Einstadter, Douglas; Groh-Wargo, Sharon; Collin, Marc (2015): Standardized Slow Enteral Feeding Protocol and the Incidence of Necrotizing Enterocolitis in Extremely Low Birth Weight Infants. In: JPEN. Journal of parenteral and enteral nutrition 39 (6), S. 644-654. DOI: 10.1177/0148607114552848.
Weyerstahl, Thomas; Stauber, Manfred; Andergassen, Ulrich (2013): Gynäkologie und Geburtshilfe. [mit Filmen zu Spontangeburt und Sectio online]. Unter Mitarbeit von Thomas Weyerstahl, Manfred Stauber und Ulrich Andergassen. 4., vollst. überarb. Aufl. Stuttgart: Thieme (Duale Reihe).

WO 2012/093087 A1 offenbart eine wässrige Zusammensetzung zur Anwendung in einem Verfahren zur chirurgischen oder therapeutischen Behandlung des menschlichen oder tierischen Körpers oder in einem Diagnostizierverfahren, das am menschlichen oder tierischen Körper vorgenommen wird, wobei die Zusammensetzung osmotisch aktive Teilchen umfasst und weniger als 70 µl⁻¹ Amnionzellen enthält, wobei die Osmolarität der Zusammensetzung im Bereich von 240,0 mosm/l bis kleiner 308,0 mosm/l liegt und die Chloridionenkonzentration in der Zusammensetzung höchstens 130,0 mmol/l ist. Die wässrige Lösung beinhaltet keine Hyaluronsäure, unvollständige Spurenelemente und keine Vitamine.

Die Zusammensetzung kann zur Amnioinfusion eingesetzt werden. Weitere bevorzugte Anwendungsgebiete der Zusammensetzung schließen das Spülen von Körperhöhlen, bevorzugt das Ausspülen von eitrigen Höhlen, insbesondere in der Lunge, wie z. B. im Rahmen einer bronchoalveolären Lavage, sowie das Spülen von mit Häuten, insbesondere mit Schleimhäuten, überzogenen Körperbereichen ein. Hinweise auf eine Resorption der Zusammensetzung durch die Haut oder die Schleimhaut, durch kapillararmen Schichten oder durch die Darmschleimhaut sind der Druckschrift allerdings nicht zu entnehmen.

Eine Anwendung der Zusammensetzung in einem Verfahren zur enteralen Ernährung, zur enteralen Ernährungsergänzung und/oder zur Behandlung von kapillararmen Schichten mit einer Kapillardichte von 0 bis 50/mm² wird in dem o.g. Patent nicht erwähnt.

Von Serumwerk Bernburg AG (Deutschland) wird eine Amnion Flush Solution zur kontinuierlichen Amnioinfusion beim klassischen Blasensprung mit einem Oligo/ Anhydramnion folgender Zusammensetzung angeboten:

| | |
|---|---|
| 1000 ml Lösung | |
| Natriumchlorid | 6,172 g |
| Natriumhydrogencarbonat | 1,42 g |
| Kaliumchlorid) | 0,291 g |
| Natriumlaktat | 1,02 g |
| Calciumglukonat Monohydrat | 0,717 g |
| Magnesiumchlorid Hexahydrat | 0,116 g |
| Dinatrium-β-glycerophosphat-pentahydrat | 0,107 g |
| Zitronensäure Monohydrat | 0,074 g |
| Harnstoff | 0 bis 0,10 g |
| Kupferchlorid Dihydrat | 0,429 mg |
| Natriumselenit Pentahydrat | 0,039 mg |
| Zinkchlorid | 0,354 mg |
| Wasser für Injektionszwecke | ad 1,000 ml |

Diese Zusammensetzung enthält somit Na⁺ 132,2 mmol/l, K⁺ 3,90 mmol/l, Ca²⁺ 1,60 mmol/l, Mg²⁺ 0,57 mmol/l, Cl⁻ 110,7 mmol/l, Laktat 9,10 mmol/l, Gluconat 3,20 mmol/l, Hydrogencarbonat 16,90 mmol/l, Citrate 0,35 mmol/l, Phosphat 0,35, Kupfer 2,52 µmol/l, Zink 2,60 µmol/l, Selen 0,15 µmol/l, theoretische Osmolarität 278,9 mOsm/l, pH 7,0-8,0.

Als Anwendungsgebiet der Zusammensetzung wird in der Gebrauchsinformation angegeben:
"Die sterile Lösung wird für die kontinuierliche Amnioninfusion über ein subkutan implantiertes Portsystem oder einen intra-amnialen Katheter im Falle eines diagnostizierten Oligo-/Anhydramnion nach vorzeitigem Blasensprung ab der 22+0 Schwangerschaftswoche angewendet. Der Blasensprung kann dabei bereits vor der 22+0 Schwangerschaftswoche aufgetreten sein. Die Amnioninfusion dient der Verlängerung der Schwangerschaft, der Reduktion der Ausbreitung lokaler Infektionen durch Ausspülen von Bakterien und Inflammationsprodukten aus der Amnionhöhle ("flush-out Methode), der Verhinderung einer Lungenhypoplasie und der Wiederherstellung einer normalen Fruchtwassermenge unter stehlen Bedingungen."

Eine Anwendung der Zusammensetzung zur Behandlung eines menschlichen oder tierischen Körpers, insbesondere in einem Verfahren zur enteralen Ernährung, zur enteralen Ernährungsergänzung und/oder zur Behandlung von kapillararmen Schichten mit einer Kapillardichte von 0 bis 50/mm², ist nicht vorgesehen.

Die Patentanmeldung EP 0 998 916 A1 beschäftigt sich mit medizinischen Zusammensetzungen für Applikationen über die Schleimhaut. Die Zusammensetzungen sollen eine Osmolarität kleiner 290 mOsm/l aufweisen.

Als Schleimhaut werden die Darmschleimhaut, die Magenschleimhaut, die Nasenschleimhaut, die Tracheal- / Bronchial- / Lungenschleimhaut, die Schleimhaut der Mundhöhle, die Rektalschleimhaut und die Vaginalschleimhaut konkret genannt, wobei die Nasenschleimhaut am meisten bevorzugt wird.

Die klassischen Elektrolytlösungen mit einer Osmolarität im Bereich von 240,0 mosm/l bis 300 mosm/l weisen allerdings eine zu hohe Chloridionenkonzentration und einen pH-Wert im sauren unphysiologischen Bereich < 7 auf. Diese erhöht die Chlorid- und Na-Ionenkonzentration, welche aufgenommen werden. Dies kann, insbesondere bei Langzeit-Verabreichung, eine Schädigung der Organe des Patienten, vor allem seiner Haut, seiner Augen und seiner Verdauungsorgane, insbesondere seiner Nieren, bewirken. Weiterhin ist eine Veränderung der Programmierung des Patienten zu befürchten, die ggf. zu Problemen im weiteren Leben führen kann, auch wenn dies aufgrund fehlender entsprechender Daten bisher noch nicht beschrieben wurde.

Eine Plasma-Schwankung des NaCI-Gehaltes nach der extensiven Verwendung von den Plasma-Ersatz-Elektrolytlösungen führt zur zentralen pontinen und extrapontine Myelinolyse und zum osmotischen Demyelinisierungssyndrom. Bei den Feten ist die Myelinisierung noch nicht komplett abgeschlossen. Wegen einer sehr unreifen Blut-Hirn-Schranke ist die Elektrolytenschwankung im Blut besonders gefährlich. Eine dauerhafte Anwendung von Elektrolytenlösungen zur Amnioinfusion nach dem Blasensprung könnte den mangelhaften Effekt der Therapie in mehreren Studien erklären. So konnten Ono et al. (2020) trotzt der Verlängerung der Schwangerschaft keine Verbesserung des neonatalen Outcomes bei den Patienten mit dem vorzeitigen Blasensprung nach der Amnioinfusion mit Ringer-Lactat verzeichnen. In der Studie von Roberts et al. starben doppelt so viel Kinder nach der Geburt in der Gruppe der Amnioinfusion mit Hartmann-Lösung im Vergleich zur Kontrolle. Tchirikov et al. hat diesen negativen Effekt auf die Feten als "Salzgurken-Effekt" bezeichnet.

Präparate auf der Basis von Phospholipiden aus der Rinderlunge oder Schweinelunge sind für die Behandlung von Frühgeborenen mit AtemnotSyndrom seit Längerem auf dem Markt (ALVEOFACT^{®}, SURVANTA^{®}, Curosurf^{®}).

ALVEOFACT^{®} enthält, bezogen auf 1,2 ml, 50,76 mg - 60 mg Phospholipidfraktion aus Rinderlunge (Surfactant; entspricht 50 mg Gesamtphospholipide) sowie Natriumchlorid und Natriumhydrogencarbonat (Rote Liste 2000).

SURVANTA^{®} enthält, bezogen auf 8 ml, 72,8 mg - 211,2 mg Phospholipidfraktion aus Rinderlunge, 18,4 mg - 157,6 mg 1,2-Dipalmitoyl-sn-glycero(3)phosphocholin, 1,4 mg - 11,1 mg Palmitinsäure, 2,2 mg - 10,5 mg Glyceroltripalmitat (entspricht 50 mg Gesamtphospholipide) sowie Natriumchlorid und Natriumhydrogencarbonat oder Salzsäure (Rote Liste 2000).

Curosurf^{®} 1,5 ml Suspension, Chiesi GmbH, Hamburg, DE, Hersteller Chiesi Farmaceutici S. p.A., Parma, Italien, enthält Phospholipidfraktion aus Schweinelunge 120 mg, entsprechend 111 mg Gesamtphospholipide, Natriumchlorid, Natriumhydrogencarbonat, Wasser. Die Behandlung eines RDS-Syndroms soll innerhalb 48 St. nach Stellung der Diagnose begonnen werden, sonst verspricht die intratracheale Anwendung nach der Angabe des Herstellers kein Erfolg mehr (Beipackzettel, Gebrauchsinformation).

In Anbetracht des Standes der Technik war es daher Aufgabe der vorliegenden Erfindung, bessere Möglichkeiten zur Ernährung und/oder Behandlung eines menschlichen oder tierischen Körpers, bevorzugt zur Behandlung eines menschlichen oder tierischen Körpers, insbesondere zur Behandlung von Krankheiten eines menschlichen oder tierischen Auges, insbesondere zur Behandlung von Patienten mit Sicca-Syndrom, einer Störung der Meibomdrüsen und/oder mit einer Störung infolge von Kontaktlinsentragen, zur enteralen Ernährung oder zur enteralen Ernährungsergänzung eines menschlichen oder tierischen Körpers, zur Behandlung von mindestens einer kapillararmen Schicht, zur Behandlung von Patientinnen mit Oligo-Anhydramnion, insbesondere mit vorzeitigem Blasensprung (PPROM); zur Behandlung von Krankheiten des bronchopulmonalen Systems und/oder der Atemwege eines menschlichen oder tierischen Körpers; zur Behandlung von Hauterkrankungen eines menschlichen oder tierischen Körpers, insbesondere zur Behandlung von Amnion-Transplantaten aufzuzeigen. Gewünscht wurde insbesondere eine Lösung für die Nachteile und Probleme der bisherigen Verfahren sowie insbesondere Lösungen für kapillararme Schichten.

Gemäß einem ersten Aspekt der vorliegenden Erfindung wurden Möglichkeiten zur Behandlung von Krankheiten eines menschlichen oder tierischen Auges gesucht, die die mit der Krankheit verbundenen Symptome sehr schnell und vergleichsweise langanhaltend signifikant lindern und im besten Fall sogar dauerhaft heilen.

Wünschenswert war in diesem Zusammenhang insbesondere eine lange und ausreichende Wirkung in den Augen.

Darüber hinaus wurden Möglichkeiten zur Behandlung von Krankheiten eines menschlichen oder tierischen Auges gewünscht, die sehr gut handhabbar sind und die Sensorik des Auges nicht beeinträchtigen. Angestrebt wurden insbesondere
- eine sehr gute, möglichst sogar eine signifikant verbesserte Tränenfilmstabilität,
- ein sehr guter, möglichst sogar ein signifikant verminderter Oberflächenstress,
- eine sehr gute, möglichst sogar eine signifikant verbesserte Kontrastsensitivität und optische Qualität der Oberfläche sowie
- eine sehr gute, möglichst sogar signifikant gesteigerte Lebensqualität.

Gewünscht wurden vor allem Möglichkeiten zur Behandlung eines trockenen Auges, die eine signifikante Verbesserung der Symptome und der klinischen Zeichen bewirken. Dabei sollte sich nach vergleichsweise kurzer, beispielsweise zweiwöchiger Behandlungsdauer möglichst eine moderate bis vollständige Rückbildung der Symptome zeigen. Auch die Fluoreszein-Anfärbung der Hornhaut sollte möglichst signifikant reduziert werden. Darüber hinaus wurde ein möglichst dauerhafter Behandlungserfolg angestrebt, wobei die Beschwerden und klinischen Zeichen der Erkrankung auch nach Absetzen der Therapie möglichst für mehrere Wochen, im Idealfall dauerhaft, geringer oder sogar vollständig weg sind.

Darüber hinaus wurden möglichst eine Verbesserung der Keratopathie, erhöhte Werte im Schirmer-Test, eine Abnahme der Beschwerden (Schleiersehen, Augentrockenheit, Fremdkörpergefühl und Tränen) und eine Verringerung der Tränenersatztherapie gefordert, wobei diese klinische Besserung gleichzeitig möglichst mit einer Reduktion von Entzündungszellen und entzündlichen Markern an der Augenoberfläche sowie einer Zunahme von Becherzellen in der Bindehaut verbunden sein sollte.

Besonders wünschenswert waren Möglichkeiten zur Behandlung von Blepharitis und Meibomdrüsendysfunktion, wobei neben einer besseren Meibomdrüsenfunktion und Symptomatik auch eine Reduktion der bakteriellen Lidkantenbesiedelung und Normalisierung des Lipidprofils des Meibomdrüsensekrets gewünscht wurde.

Eine Anwendung eines kryokonservierten amnialen Membran in der Augenheilkunde und auch in der Hautheilkunde und Orthopädie ist seit Jahrzehnten ein etabliertes Verfahren (Thomasen et al. 2018, Ophtalmonologie). Ein geeignetes Spülmittel mit der Ernährungsfunktion für die amniale Membran wurde bis heute nicht etabliert. Besonders wünschenswert waren Möglichkeiten einer wässrigen Lösung aufgrund des Fruchtwassers zur Behandlung und Versorgung der transportierten amnialen Membran und des darunterliegenden Gewebes.

Gemäß einem weiteren Aspekt der vorliegenden Erfindung wurden Möglichkeiten zur Ernährung eines menschlichen oder tierischen Körpers gesucht, die es bei Erwachsenen wie bei Kindern ermöglichen
- einen gestörten Darm zu ergänzen,
- einen entzündeten Magen-Darm-Trakt zu spülen;
- einen entzündeten Darm ruhen zu lassen und/oder
- eine schwere Mangelernährung bei einem Kranken zu korrigieren, der sich nicht mehr ernähren kann oder will, sei es auf Grund einer schweren Erkrankung oder einer strapaziösen Behandlung.

Wünschenswert waren insbesondere die folgenden Eigenschaften:
- die zu findende Lösung sollte ähnlich zu herkömmlicherweise aufgenommener Nahrung oder dieser sogar überlegen sein;
- die zu findende Lösung sollte möglichst eine Erzeugung von Surfactant bewirken;
- die zu findende Lösung sollte möglichst eine Erzeugung nützlicher flüchtiger Fettsäuren bewirken;
- die zu findende Lösung sollte eine optimale physiologische Elektrolyten- und Spurenelementen-Plasma-Konzentration zu erreichen;
- die Neigung des Patienten zu Blähungen sollte möglichst merklich reduziert, im besten Fall sogar vollständig unterdrückt werden;
- die Darmbeweglichkeit des Patienten sollte möglichst signifikant verbessert werden;
- die zu findende Lösung sollte möglichst zu keinen Rückständen führen, die die Gesundheit des Patienten gefährden oder beeinträchtigen können oder zu einem Unbehagen beim Patienten führen können;
- die zu findende Lösung sollte möglichst vom Körper des Patienten sehr gut aufgenommen und resorbiert werden können;
- die zu findende Lösung sollte möglichst leicht realisiert und umgesetzt werden können;
- für die zu findende Lösung wurden eine möglichst einfache Lager- und Sterilisiermöglichkeit sowie eine möglichst gute Assimilierung von Bestandteilen gefordert;
- die zu findende Lösung sollte möglichst Probleme, die mit einer zu geringen Mineralienaufnahme verbunden sind, vermeiden.

Weiterhin wurden insbesondere Möglichkeiten zur Ernährung von Frühgeborenen gesucht, wobei möglichst eine Behandlung, Spülung der Darmoperation, und/oder Unterdrückung von nekrotisierender Enterokolitis, eine möglichst signifikante Verbesserung des Gesundheitszustands der Frühgeborenen und möglichst eine signifikante Senkung der Morbidität der Frühgeborenen angestrebt wurde. Insbesondere sollte eine Organunreife der Frühgeborenen möglichst schnell und möglichst vollständig behoben werden.

Gleichzeitig wurden folgende Vorteile angestrebt:
- Die Gesamtdauer der intensiven Betreuung sollte möglichst signifikant verkürzt werden und die Risiken einer Hirnblutung, NEC, einer bronchopulmonalen Dysplasie, Blindheit, eines cholestatischen Ikterus sollte bestmöglich reduziert werden.
- Die Kinder sollten bei der Entlassung möglichst ein höheres Gewicht und möglichst keine bleibenden Schäden aufgrund der Frühgeburt haben.

Weiterhin war es daher Aufgabe der vorliegenden Erfindung, bessere Möglichkeiten zur Behandlung von Patientinnen mit Oligo-Anhydramnion, insbesondere mit vorzeitigem Blasensprung (PPROM) aufzuzeigen. Gewünscht wurde insbesondere eine Lösung für die Nachteile und Probleme der bisherigen Amnioninfusionsverfahren. So sollten vor allem Wege aufgezeigt werden, wie gesundheitliche Risiken für den Fötus, insbesondere das Risiko einer Frühgeburt, möglichst vermieden werden können. Weiterhin wurde eine möglichst rasche und möglichst gute Lungenreifung gewünscht, um die Überlebenschancen des Fötus, selbst bei einer Frühgeburt, zu erhöhen. Darüber hinaus sollte eine Schädigung von Organen des Fötus, vor allem seines Gehirnes, seiner Haut, seiner Augen und seiner Verdauungsorgane, insbesondere seiner Nieren, möglichst vermieden werden. Wichtig war in diesem Zusammenhang auch, eine Änderung der fetalen Programmierung des Fötus nach Möglichkeit zu vermeiden. Schließlich sollte sich die erfindungsgemäße Lösung auch auf vergleichbar einfache Art und Weise kostengünstig realisieren lassen und aus moralischer, ethischer sowie rechtlicher Sicht unbedenklich sein.

Weiterhin war es daher Aufgabe der vorliegenden Erfindung eine fruchtwasserähnliche Lösung für das künstliche Gebärmutter- und Kiemensystem zu entwickeln (WO 2018/171905A1 und WO 2020/084125A1). Hier sollen insbesondere beim extrem frühgeborenen Kind die durch die Frühgeburt unterbrochene Reifungsprozesse in einem künstlichen Gebärmutter- und Kiemensystem möglichst gut fortgesetzt werden können.

Angestrebt wurden weiterhin bessere Möglichkeiten zur Behandlung von Krankheiten des bronchopulmonalen Systems und/oder der Atemwege eines menschlichen oder tierischen Körpers sowie bessere Möglichkeiten zur Behandlung von Hauterkrankungen eines menschlichen oder tierischen Körpers, insbesondere zur Versorgung von Haut und/oder Amnion-Transplantaten.

Weiterhin war es daher Aufgabe der vorliegenden Erfindung, Möglichkeiten zur Verbesserung künstlicher Gebärmutter- und Kiemensysteme aufzuzeigen, die beispielsweise in WO 2018/171905 A1 und WO 2020/084125 A1 beschrieben werden. Hier sollten insbesondere bei extrem frühgeborenen Kindern die durch die Frühgeburt unterbrochene Reifungsprozesse bestmöglich fortgesetzt werden.

Diese sowie weitere Aufgaben, die sich aus den in dieser Anmeldung diskutierten Zusammenhängen in naheliegender Weise ergeben, werden durch die eine oder mehrere Zusammensetzungen mit allen Merkmalen des vorliegenden Patentanspruchs 1 gelöst.

Die auf die Patentanspruch 1 rückbezogenen Unteransprüche beschreiben besonders vorteilhafte Ausführungsformen der einen oder mehreren Zusammensetzungen.

Die vorliegende Erfindung basiert auf der Feststellung, dass die erfindungsgemäße eine oder mehrere Zusammensetzungen zur Anwendung in einem Verfahren zur enteralen Ernährung, zur enteralen Ernährungsergänzung und/oder zur Behandlung und/oder Versorgung von kapillararmen Schichten besonders geeignet und befähigt sind. Es wurde insbesondere beobachtet, dass die eine oder mehreren Zusammensetzungen der vorliegenden Erfindung aufgrund der starken Ähnlichkeit zum menschlichen Fruchtwasser sehr gut über die Haut und über die Schleimhaut, insbesondere über mindestens eine kapillararme Schicht resorbiert und im Körper assimiliert werden. Es wurde eine komplette Remission einer *Keratoconjunctivitis sicca* bei mehreren Probanden festgestellt. Es wurde eine rasche Reduktion der O₂-Konzentration bei der invasiven Beatmung eines extrem Frühgeborenen durch die vermehrte Surfactant-Produktion beobachtet. Dies ermöglicht eine extrem schnelle und sehr gute Aufnahme und Assimilierung der Bestandteile der einen oder mehreren Zusammensetzungen gemäß der vorliegenden Erfindung, insbesondere von Surfactant. Basierend auf dieser neuen und überraschenden Erkenntnis wurden die vorstehend genannten sowie weitere sich in naheliegender Weise ergebende Aufgaben gelöst.

Gemäß einem besonders bevorzugten Aspekt der vorliegenden Erfindung werden eine oder mehrere Zusammensetzungen zur Anwendung in einem Verfahren zur Behandlung von kapillararmen Schichten mit einer Kapillardichte von 0 bis 50/mm², genauer gesagt zur Behandlung von Krankheiten des menschlichen oder tierischen Auges bereitgestellt. Dabei werden die Vorteile und Effekte der vorliegenden Erfindung insbesondere bei der Behandlung von Patienten mit Sicca-Syndrom, einer Störung der Meibomdrüsen und/oder mit einer Störung infolge von Kontaktlinsentragen beobachtet.

Erfindungsgemäß werden die mit der Krankheit verbundenen Symptome sehr schnell und vergleichsweise langanhaltend signifikant gelindert und im besten Fall sogar dauerhaft geheilt.

Obwohl eine Grundlage der einen oder mehreren Zusammensetzungen zur Behandlung von Krankheiten eines menschlichen oder tierischen Auges Wasser ist, können die Zusammensetzungen im Falle des Tropfens oder bei Applikation in Form eines Gels in die Augen leicht auf die Cornea aufgebracht werden und es ist nicht wahrscheinlich, dass sie aus den Augen fließen, sogar wenn die eine oder mehreren Zusammensetzungen durch Tränenflüssigkeit verdünnt werden. Dementsprechend werden Bestandteile der einen oder mehreren Zusammensetzungen lange und ausreichend in den Augen zurückgehalten.

Darüber hinaus zeichnen sich die eine oder mehreren Zusammensetzungen zur Behandlung von Krankheiten eines menschlichen oder tierischen Auges durch eine sehr gute Handhabbarkeit und Affinität zu den Augen (Gefühl im Falle des Tropfens oder bei Applikation in Form eines des Gels in die Augen) aus. Bei Verwendung der einen oder mehreren Zusammensetzungen zur Behandlung von Krankheiten eines menschlichen oder tierischen Auges werden insbesondere
- eine sehr gute, meist sogar signifikant verbesserte Tränenfilmstabilität,
- ein sehr guter, meist sogar signifikant verminderter Oberflächenstress,
- eine Halbierung der Blinzeln-Intensität,
- eine sehr gute, meist sogar signifikant verbesserte Kontrastsensitivität und optische Qualität der Oberfläche sowie
- eine sehr gute, meist sogar signifikant gesteigerte Lebensqualität beobachtet.

Bei Behandlung eines trockenen Auges mit der einen oder mehreren Zusammensetzungen ist in der Regel eine signifikante Verbesserung der Symptome und der klinischen Zeichen festzustellen. Nach vergleichsweise kurzer, beispielsweise zweiwöchiger Behandlungsdauer zeigt sich häufig eine moderate bis vollständige Rückbildung der Symptome. Auch die Fluoreszein-Anfärbung der Hornhaut reduziert sich häufig signifikant. Bemerkenswert ist weiterhin die Dauerhaftigkeit des Behandlungserfolgs, da die Beschwerden und klinischen Zeichen der Erkrankung auch nach Absetzen der Therapie für mehrere Wochen, im Idealfall dauerhaft, geringer, wenn nicht gar vollständig weg sind.

Darüber hinaus werden für die Verwendung der einen oder mehreren Zusammensetzungen zur Behandlung von Krankheiten eines menschlichen oder tierischen Auges häufig auch eine Verbesserung der Keratopathie, erhöhte Werte im Schirmer-Test, eine Abnahme der Beschwerden (Schleiersehen, Augentrockenheit, Fremdkörpergefühl und Tränen) berichtet. Diese klinische Besserung ist gleichzeitig meist mit einer Reduktion von Entzündungszellen und entzündlichen Markern an der Augenoberfläche sowie einer Zunahme von Becherzellen in der Bindehaut verbunden.

Besonders erfolgreich können die eine oder mehreren Zusammensetzungen zur Behandlung von Blepharitis und Meibomdrüsendysfunktion eingesetzt werden. Neben einer besseren Meibomdrüsenfunktion und Symptomatik fand sich eine Reduktion der bakteriellen Lidkantenbesiedelung und Normalisierung des Lipidprofils des Meibomdrüsensekrets.

Gemäß einem weiteren besonders bevorzugten Aspekt der vorliegenden Erfindung werden eine oder mehrere Zusammensetzungen zur Anwendung in einem Verfahren zur enteralen Ernährung eines menschlichen oder tierischen Körpers bereitgestellt.

Diese enterale Ernährung eines menschlichen oder tierischen Körpers ermöglicht bei Erwachsenen und Kindern
- einen gestörten Darm bestmöglich zu ergänzen,
- eine Surfactant-Produktion der Lunge bestmöglich zu unterstützen,
- einen entzündeten Darm bestmöglich ruhen zu lassen und/oder zu behandeln,
- einen erkrankten und/oder operierten Darm bestmöglich zu spülen,
- eine schwere Mangelernährung bei einem Kranken bestmöglich zu korrigieren, der sich nicht mehr ernähren kann oder will, sei es auf Grund einer schweren Erkrankung oder einer strapaziösen Behandlung.

Die Verwendung der einen oder mehrere Zusammensetzungen zur enteralen Ernährung und/oder Ernährungsergänzung eines menschlichen oder tierischen Körpers führt insbesondere zu den folgenden Vorteilen:
- die eine oder mehrere Zusammensetzungen sind sehr ähnlich zu herkömmlicherweise aufgenommener enteraler Nahrung eines Fetus;
- sie können insbesondere eine Erzeugung nützlicher flüchtiger Fettsäuren bewirken;
- die Neigung von Patienten zu Blähungen, ggf. zu Darmverstopfung durch im Magen zum Käse gewordener Milch, wird merklich reduziert, im besten Fall sogar vollständig unterdrückt;
- die Darmbeweglichkeit des Patienten wird signifikant verbessert;
- die Verabreichung der einen oder mehreren Zusammensetzungen führt zu keinen Rückständen, die die Gesundheit des Patienten gefährden oder beeinträchtigen können oder zu einem Unbehagen beim Patienten führen können;
- die eine oder mehreren Zusammensetzungen können vom Körper des Patienten sehr gut aufgenommen und resorbiert werden, da Löslichkeitsprobleme bestmöglich vermieden werden;
- die eine oder mehreren Zusammensetzungen sind zweckmäßigerweise flüssig und weisen vorzugsweise eine Viskosität bei 25°C im Bereich von 5 bis 100 mPas auf, um ihre Verabreichung weiter zu erleichtern;
- die eine oder mehreren Zusammensetzungen können auf einfachste Art und Weise gelagert und sterilisiert und assimiliert werden;
- durch die eine oder mehreren Zusammensetzungen wird die Mineralienaufnahme extrem begünstigt.

Weiterhin wird festgestellt, dass die frühe enterale Ernährung insbesondere für Frühgeborenen von Vorteil ist. Vor allem bei der Behandlung und/oder Unterdrückung von nekrotisierender Enterokolitis wird durch die enterale Nahrung gemäß der vorliegenden Erfindung der Gesundheitszustand der Frühgeborenen signifikant verbessert und die Morbidität der Frühgeborenen signifikant gesenkt.

Gleichzeitig ist ein früher Beginn der enteralen Ernährung von Frühgeborenen mit der einen oder mehreren Zusammensetzungen mit mehreren Vorteilen verbunden:
- Die Gesamtdauer der intensiven Betreuung wird in der Regel signifikant verkürzt und es kommt seltener zu Komplikationen, wie intrazerebrale Blutung, NEC, RDS, bronchopulmonale Dysplasie und einem cholestatischen Ikterus.
- Häufig haben die Kinder bei der Entlassung ein höheres Gewicht und deutliche seltener bleibende Schäden durch die Frühgeburt.

Gemäß einem weiteren besonders bevorzugten Aspekt der vorliegenden Erfindung werden eine oder mehrere Zusammensetzungen zur Anwendung in einem Verfahren zur Anwendung bei der Amnioninfusion bei einem menschlichen oder tierischen Körper bereitgestellt. Besonders bewährt hat sich die Verwendung der einen oder mehreren Zusammensetzungen zur Behandlung eines Oligo-Anhydramnion und zur Behandlung eines vorzeitigen Blasensprungs, wobei die Vorteile und Effekte der vorliegenden Erfindung insbesondere bei einer kontinuierlichen Amnioninfusion beobachtet werden.

Die Nachteile und Probleme bisheriger Amnioninfusionsverfahren werden überwunden. Die eine oder mehreren Zusammensetzungen sind für die Versorgung der kapillarfreien Schichten, wie Amnion, Nabelschnur und fetale Kornea besonders geeignet. Die Lösung ist überaus vorteilhaft für die intrauterine fetale Ernährung, ggf. Ernährungsergänzung, geeignet. So werden Möglichkeiten aufgezeigt, wie gesundheitliche Risiken für den Fötus, insbesondere das Risiko einer Frühgeburt, möglichst vermieden werden können. Weiterhin kann eine vergleichsweise schnelle und gute Lungenreifung u.a. durch die Surfactant-Gabe gewährleistet werden, welches wiederum die Überlebenschancen des Fötus, selbst bei einer Frühgeburt, deutlich erhöht. Darüber hinaus wird eine Schädigung von Organen des Fötus, vor allem seiner Haut, seiner Augen und seiner Verdauungsorgane, insbesondere seines Gehirnes und seiner Nieren, bestmöglich vermieden. Das Risiko einer Änderung der fetalen Programmierung des Fötus, besonders bei enteraler Einnahme, wird bestmöglich reduziert. Schließlich kann die erfindungsgemäße Lösung auch auf vergleichbar einfache Art und Weise kostengünstig realisiert werden und ist aus moralischer, ethischer sowie rechtlicher Sicht absolut unbedenklich.

Die Fruchtwasser-ähnliche Lösung der vorliegenden Erfindung ist auch für die Amnioinfusion in künstlichen Gebärmutter-Systemen, wie sie beispielsweise WO 2018/171905 A1 beschrieben werden, und auch für die Versorgung eines Oxygenator-Systems "Kiemen", wie es beispielsweise in WO 2020/084125A1 beschrieben wird, besonders geeignet.

Gemäß einem weiteren besonders bevorzugten Aspekt der vorliegenden Erfindung werden eine oder mehrere Zusammensetzungen zur Anwendung in einem Verfahren zur Behandlung von Krankheiten des bronchopulmonalen Systems und/oder der Atemwege eines menschlichen oder tierischen Körpers bereitgestellt. Die Behandlung von Patienten erfolgt vorzugsweise mittels Inhalation.

Gemäß einem weiteren besonders bevorzugten Aspekt der vorliegenden Erfindung werden eine oder mehrere Zusammensetzungen zur Anwendung in einem Verfahren zur Behandlung von Hauterkrankungen eines menschlichen oder tierischen Körpers, bevorzugt zur Behandlung von Patienten durch Auftragung der einen oder mehreren Zusammensetzungen auf die zu behandelnde Haut, insbesondere zur Behandlung von Amnion-Transplantaten bereitgestellt.

Gegenstand der vorliegenden Erfindung sind daher eine oder mehrere Zusammensetzungen zur enteralen Ernährung, zur enteralen Ernährungsergänzung und/oder zur Behandlung von kapillararmen Schichten mit einer Kapillardichte von 0 bis 50/mm².

Die Anwendung der erfindungsgemäßen einen oder mehrere Zusammensetzungen erfolgt zweckmäßigerweise in einem Verfahren zum in Kontakt bringen der einen oder mehreren Zusammensetzungen mit einem menschlichen oder tierischen Körper über mindestens eine Haut oder Schleimhaut, insbesondere über mindestens eine kapillararme Schicht.

Zu den Schleimhäuten gehören insbesondere die Darmschleimhaut, die Gebärmutterschleimhaut, die Magenschleimhaut, der Mukosablock, die Mundschleimhaut, die Nasenschleimhaut, die Vaginalschleimhaut, die Augenschleimhaut, Trachea und die Harnröhre), bevorzugt die Darmschleimhaut, die Magenschleimhaut, die Mundschleimhaut, die Nasenschleimhaut und die Augenschleimhaut, besonders bevorzugt die Darmschleimhaut, die Augenschleimhaut, kapillararme Schichten, wie Kornea, Amnion und Nabelschnur.

Besonders bevorzugt werden Verfahren zur Ernährung, Versorgung und/oder Befeuchtung eines menschlichen oder tierischen Körpers über mindestens eine kapillararme Schicht.

Kapillararme Schichten bezeichnen im Rahmen der vorliegenden Erfindung Schichten mit einer Kapillardichte von 0 bis 50/mm², bevorzugt Schichten mit einer Kapillardichte von 0 bis 30/mm², insbesondere kapillarlose Schichten.

Die Bestimmung der Kapillardichte kann auf an sich bekannte Weise, insbesondere mittels Dermoskopie, Videokapilloskopie (oder Kapillaroskopie), Fluoreszenzangiographie, Reflektions-Konfokalmikroskopie (RCM) oder Histopathologie erfolgen, die beispielsweise in den folgenden Publikationen beschrieben werden:
∘ M. Deng , Y. Xu, Z. Yu, X. Wang, Y. Cai, H. Zheng, W. Li , W. Zhang: Protective Effect of Fat Extract on UVB-Induced Photoaging In Vitro and In Vivo. Hindawi; Oxidative Medicine and Cellular Longevity; 2019; Article ID 6146942; 11 pages; https://doi.org/10.1155/2019/6146942
∘ E. Tibirica, E.G. Souza, A. De Lorenzo, G.M.M. Oliveira: Reduced systemic microvascular density and reactivity in individuals with early onset coronary artery disease. Microvascular Research; 97; 2015; 105-108
∘ G. Micali, A.E. Verzi, G. Broggi, R. Caltabiano, M.L. Musumeci, F. Lacarrubba: Evaluation of capillary density in psoriasis: An intrapatient study and literature review. PLOS ONE; https://doi.org/10.1371/journal.pone.0247835: March 10, 2021
∘ J. Liang, Y. Li, L. Chen, W. Xia, G. Wu, X. Tong, C. Su, J. He, X. Lin, J. Tao: Systemic microvascular rarefaction is correlated with dysfunction of late endothelial progenitor cells in mild hypertension: a substudy of EXCAVATION-CHN1. Liang et al. J Transl Med ; 2019 ; 17:368; https://doi.org/10.1186/s12967-019-2108-8
∘ Barcelosa, E. Tibirica, C. Lamas: Evaluation of microvascular endothelial function and capillary density in patients with infective endocarditis using laser speckle contrast imaging and video-capillaroscopy. Microvascular Research; 118; 2018; 61-68

Zu den für die Zwecke der vorliegenden Erfindung besonders bevorzugten kapillararmen Schichten gehören die Kornea, das Amnion, die Nabelschnur, die äußeren Schichten des Rückenmarkes bei z.B. Spina bifida, parietale und viszerale Peritoneum, transplantierte Haut oder Schleimhaut, insbesondere die Kornea.

Transplantate, wie z.B. Amnion, Kornea, Schleimhaut oder Haut haben nach der Transplantation keine Kapillare oder bei der Haut und Schleimhaut vorübergehend keine Kapillare und werden als kapillararmen Schichten definiert.

Im Rahmen einer ganz besonders bevorzugten Ausführungsform der vorliegenden Erfindung dienen die eine oder mehreren Zusammensetzungen zur Anwendung in einem Verfahren zur enteralen Ernährung, zur enteralen Ernährungsergänzung und/oder zur Behandlung und/oder Versorgung von kapillararmen Schichten.

Dabei führt die genannte erfindungsgemäße Anwendung der einen oder mehreren Zusammensetzungen zu einer zumindest teilweisen oder vollständigen Resorption der einen oder mehreren Zusammensetzungen in den Körper, bevorzugt eines Menschen oder eines Tieres, insbesondere eines Menschen. Weiterhin ist eine zumindest teilweise oder vollständige Aufnahme und Assimilierung von Bestandteilen der einen oder mehreren Zusammensetzungen in den Körper zu beobachten.

Infolge der erfindungsgemäßen Anwendung der einen oder mehreren Zusammensetzungen in einem Verfahren zur enteralen Ernährung, zur enteralen Ernährungsergänzung und/oder zur Behandlung und/oder Versorgung von kapillararmen Schichten sind daher entsprechende Änderungen in dem betreffenden Körper, bevorzugt eine Veränderung der Konzentration von Bestandteilen der einen oder mehreren Zusammensetzungen in dem betreffenden Körper, insbesondere in Körperflüssigkeiten, vor allem im Blut, festzustellen. Alternativ oder zusätzlich ist günstigerweise eine Linderung oder sogar vollständige Heilung der behandelten Symptome zu notieren.

Im Rahmen einer ersten besonders bevorzugten Variante betrifft die vorliegende Erfindung eine oder mehrere Zusammensetzungen zur Anwendung in einem Verfahren zur Behandlung von Krankheiten des menschlichen oder tierischen Auges.

Zweckmäßigerweise sind die eine oder mehreren Zusammensetzungen zur Behandlung von Krankheiten eines menschlichen oder tierischen Auges als Augentropfen (*Oculoguttae*) oder als Gel formuliert. Darreichungsformen zum Eintropfen in den Bindehautsack oder auf die Hornhaut des Auges oder zur Applikation als Gel werden besonders bevorzugt.

Vorzugsweise sind die eine oder mehreren Zusammensetzungen zur Behandlung von Krankheiten eines menschlichen oder tierischen Auges steril, da das Auge ein empfindliches Sinnesorgan ist, welches nicht gut durchblutet ist und dessen Regenerationsmöglichkeiten begrenzt sind.

Um die bestmögliche Wirksamkeit, Verträglichkeit und Reizlosigkeit bei der Anwendung der einen oder mehreren Zusammensetzungen zur Behandlung von Krankheiten eines menschlichen oder tierischen Auges zu erzielen, sind der pH-Wert, die Tonizität und die Viskosität der einen oder mehreren Zusammensetzungen an die physiologischen Parameter des Tränenfilms so weit wie möglich angepasst.

Die eine oder mehreren Zusammensetzungen zur Behandlung von Krankheiten eines menschlichen oder tierischen Auges weisen zweckmäßigerweise einen pH-Wert im Bereich von 6,0 bis 10,0, bevorzugt im Bereich von 6,5 bis 9,0, besonders bevorzugt im Bereich von 6,75 bis 8,5 auf. Zur Einstellung können Puffersysteme, insbesondere zum Beispiel Natriumacetat/Borsäure, Phosphat- oder Acetat-Puffer eingesetzt werden.

Nach dem Einbringen der einen oder mehreren Zusammensetzungen in das Auge wird der pH-Wert günstigerweise durch die Pufferwirkung der Tränenflüssigkeit auf den physiologischen pH-Wert angeglichen.

Gemäß einer bevorzugten Variante der vorliegenden Erfindung werden die eine oder mehreren Zusammensetzungen in Augentropffläschchen aus speziellem Glas (bevorzugt Braunglas Glasart I) oder in Kunststoffbehältnissen (Ophtiolen) abgefüllt. Optionen gibt es für den Einmalgebrauch (Eindosis-Ophtiole, EDO) oder für den Mehrfachgebrauch (Mehrdosis-Ophtiole, MDO). Mehrdosenbehältnisse ermöglichen eine mehrmalige tropfenweise Applikation der Zubereitung und enthalten zweckmäßigerweise maximal ein Volumen von 10-100 ml.

Gemäß einer besonders bevorzugten Variante der vorliegenden Erfindung können Surfactant, Hyaluronsäure, Vitamine und einige Mikroelemente kurz vor der Anwendung in die eine oder mehreren Zusammensetzungen eingeführt werden. Surfactant wird zweckmäßigerweise kurz vor der Anwendung in eine oder mehrere Zusammensetzungen eingeführt.

Um mikrobielle Verunreinigung nach Anbruch zu unterdrücken, werden bei eigenen Varianten die eine oder mehreren Zusammensetzungen zur Behandlung von Krankheiten eines menschlichen oder tierischen Auges in Mehrdosisbehältnissen zweckmäßigerweise konserviert, sofern die Behältniskonstruktion das Eindringen vom Keimen nicht verhindert.

Bevorzugte Konservierungsmittel sind:
Thiomersal, bevorzugt 0,002 %;
organische Quecksilberverbindungen, insbesondere Phenylquecksilber; Benzalkoniumchlorid, bevorzugt 0,002-0,02 %;
Chlorhexidin, bevorzugt 0,005-0,01 %;
Benzylalkohol, bevorzugt 0,5-1 %.

Mehrdosenbehältnisse, die eine mikrobielle Kontamination der Zubereitung nach Anbruch verhindern, so dass Konservierungsstoffe erlässlich sind, sind zum Beispiel das Primärpack-System ABAK^{®}- oder das COMOD^{®}-System.

Eine oder mehreren Zusammensetzungen zur Behandlung von Krankheiten eines menschlichen oder tierischen Auges in Form von Suspensionen oder eines Gels sind vorzugsweise leicht zu dispergieren und die Dispersion bleibt zweckmäßigerweise genügend lange stabil, um die genaue Entnahme der Dosis sicherzustellen. Suspensionszusammensetzungen entsprechen zweckmäßigerweise der Prüfung auf Teilchengröße gemäß dem Europäischen Arzneibuch.

Im Rahmen einer weiteren Ausführungsform der vorliegenden Erfindung sind die eine oder mehreren Zusammensetzungen zur Behandlung von Krankheiten eines menschlichen oder tierischen Auges als ophthalmologische Salbe oder als Gel formuliert. Eine oder mehrere Zusammensetzungen zur Behandlung von Krankheiten eines menschlichen oder tierischen Auges in Form von Augentropfen werden allerdings erfindungsgemäß besonders bevorzugt.

Im Rahmen einer besonders bevorzugten Ausführungsform der vorliegenden Erfindung umfassen die eine oder mehreren Zusammensetzungen zur Behandlung von Krankheiten eines menschlichen oder tierischen Auges viskositätserhöhende Stoffe, bevorzugt Cellulosederivate, günstigerweise Hypromellose, Methylcellulose; Hyaluronsäure, Celluloseacetatphthalat oder Poloxamere, insbesondere Hyaluronsäure, um die die Kontaktzeit der einen oder mehreren Zusammensetzungen mit dem Auge zu verlängern.

Im Rahmen einer besonders bevorzugten Ausführungsform der vorliegenden Erfindung beträgt die Viskosität der einen oder mehreren Zusammensetzungen zur Behandlung von Krankheiten eines menschlichen oder tierischen Auges bei 25°C vorzugsweise 5 bis 100 MPas und bevorzugt 10 bis 70 MPas. Zweckmäßigerweise wird die Viskosität der einen oder mehreren Zusammensetzungen unter Verwendung eines Rotationsviskometers RE-110SL (hergestellt durch Toki Sangyo Co., Ltd.) gemessen. Wenn die Viskosität der einen oder mehreren Zusammensetzungen zur Behandlung von Krankheiten eines menschlichen oder tierischen Auges zu groß wird, sind die Vorteile der vorliegenden Erfindung, insbesondere die Leichtigkeit der Handhabung und die Affinität zu den Augen (Gefühl im Falle des Eintropfens in die Augen oder bei Applikation als Gel oder Salbe) weniger stark. Andererseits ist die Zeitdauer der Einwirkung der einen oder mehreren Zusammensetzungen zur Behandlung von Krankheiten eines menschlichen oder tierischen Auges geringer, wenn die Viskosität der einen oder mehreren Zusammensetzungen kleiner 5 mPas ist.

Die eine oder mehreren Zusammensetzungen zur Behandlung von Krankheiten eines menschlichen oder tierischen Auges der vorliegenden Erfindung werden z. B. durch Mischen einer vorbestimmten Menge der Bestandteile mit sterilem, destilliertem Wasser als einer Grundlage und Unterwerfung der Lösung unter eine Sterilisationsbehandlung, hergestellt.

Es ist möglich, weiter Isotoniemittel, Puffersubstanzen, pH-Einsteller, Löslichkeitsverbesserer, Stabilisatoren, Antioxidantien und Antiseptika mit der einen oder mehreren Zusammensetzungen der vorliegenden Erfindung zu mischen.

Das Isotoniemittel schließt vorzugsweise D-Mannitol, Glucose und Glycerin mit ein.

Die Puffersubstanz schließt vorzugsweise Natriumdihydrogenphosphat, Natriumhydrogenphosphat, Kaliumdihydrogenphosphat, Kaliumhydrogenphosphat, Borsäure, Natriumborat, Zitronensäure, Weinsäure und/oder Natriumtartrat mit ein.

Der pH-Einsteller schließt vorzugsweise Säure, insbesondere Salzsäure, Essigsäure, und Base, insbesondere Natriumhydroxid, Kaliumhydroxid mit ein.

Der Löslichkeitsverbesserer schließt Polyoxyethylenglykolether, insbesondere Natriumcarboxymethylcellulose, Polyoxyethylenlaurylether und Polyoxyethylenoleylether, Polyethylenglykol-Fettsäureester höherer Ordnung, insbesondere Polyethylenglykolmonolaurat und Polyethylenglykolmonooleat und Polyoxyethylen-Fettsäureester, insbesondere Polyoxyethylensorbitanmonolaurat und Polyoxyethylensorbitanmonooleat mit ein.

Der Stabilisator schließt vorzugsweise Hydroxypropylmethylcellulose, Polyvinylalkohol, Carboxymethylcellulose, Hydroxyethylcellulose, Glycerin und/oder EDTA mit ein.

Das Antioxidans schließt vorzugsweise Natriumbisulfit, Natriumthiosulfat und/oder Ascorbinsäure mit ein.

Das Antiseptikum schließt vorzugsweise Chlorbutanol, Benzalkoniumchlorid, Cetylpyridiniumchlorid, Thimerosal, Phenethylalkohol, Methylparaben und/oder Propylparaben mit ein.

Der pH der einen oder mehrere Zusammensetzungen der vorliegenden Erfindung liegt vorzugsweise bei 4 bis 10 und bevorzugt bei ungefähr 5 bis 9.

Es wird vorgezogen, dass die eine oder mehrere Zusammensetzungen zur Behandlung von Krankheiten eines menschlichen oder tierischen Auges der vorliegenden Erfindung und die Tränenflüssigkeit isotonisch sind. Es wird besonders vorgezogen, dass der pH der einen oder mehrere Zusammensetzungen zur Behandlung von Krankheiten eines menschlichen oder tierischen Auges der vorliegenden Erfindung auf einen Bereich von ungefähr 5,5 bis 8,5 und vorzugsweise von ungefähr 6,5 bis 8,0 eingestellt wird.

Die eine oder mehreren Zusammensetzungen zur Behandlung von Krankheiten eines menschlichen oder tierischen Auges der vorliegenden Erfindung werden auf die gleiche Weise verwendet, wie in dem Falle von herkömmlich bekannten Mittel zur Behandlung von Krankheiten eines menschlichen oder tierischen Auges. Bevorzugt werden die eine oder mehreren Zusammensetzungen zur Behandlung von Krankheiten eines menschlichen oder tierischen Auges aus einem geeigneten Augentropfenbehälter in die Augen getropft oder unter Verwendung einer Sprühvorrichtung in die Augen gesprüht.

Im Rahmen einer besonders bevorzugten Variante wird die Behandlung von Krankheiten eines menschlichen oder tierischen Auges mit der erfindungsgemäßen einen oder mehreren Zusammensetzungen mit einer medikamentösen Therapie kombiniert.

In diesem Zusammenhang werden
- Tränenersatzstoffe, zweckmäßigerweise umfassend oberflächenaktive Stoffe, Polyvinylalkohol, Polyvinylpyrrolidon, Cellulosederivate, Hyaluronsäuren, Carbomere, Muzinanaloga, Osmoprotektiva, Lipide, insbesondere Triglyzeride, Phospholipide und/oder Rizinusöl, semifluorierte Alkane,
- immunmodulierende Stoffe, insbesondere Steroiden, Cyclosporin A, Lifitegrast und Omega-3-Fettsäuren,
- Serum-Augentropfen, die als Tränenersatz und anti-inflammatorisch wirken,
- tränenstimulierende Substanzen, insbesondere Cyclosporin,
- Mucolytika, insbesondere Acetylcystein,
- Kortikosteroide,
- Makrolide, insbesondere Azythromyin
- Omegafettsäuren
besonders bevorzugt.

Im Rahmen einer weiteren besonders bevorzugten Variante werden die eine oder mehreren Zusammensetzungen der vorliegenden Erfindung zur Behandlung von Krankheiten auf die gleiche Weise verwendet, wie in dem Falle von herkömmlich bekannten Mittel zur Behandlung von Krankheiten eines menschlichen oder tierischen Auges, Haut, Schleimhaut nach der Verbrennung oder z.B. Kornea, Haut und Amnion-Transplantation.

Für Hautverbrennungen werden die eine oder mehreren Zusammensetzungen der vorliegenden Erfindung vorzugsweise als Lösung oder Gel verwendet. Auch eine Dauerinfusion, Verbleiben in der Lösung (Badewanne) und Spülen der verbrannten Haut sind besonders vorteilhaft.

Im Rahmen einer weiteren besonders bevorzugten Variante betrifft die vorliegende Erfindung eine oder mehrere Zusammensetzungen zur Anwendung in einem Verfahren zur enteralen Ernährung eines menschlichen oder tierischen Körpers.

Die eine oder mehrere Zusammensetzungen für die enterale Ernährung werden zweckmäßigerweise vorab mit einer beliebigen dem Fachmann bekannten Technik sterilisiert, und bevorzugt in sterilen Schachteln, Beuteln oder Flaschen abgepackt.

Die Zubereitung der einen oder mehreren Zusammensetzungen für die enterale Ernährung kann mit jeder beliebigen konventionellen Technik erfolgen, insbesondere durch Mischen der verschiedenen Zutaten. Die eine oder mehreren Zusammensetzungen der vorliegenden Erfindung sind zweckmäßigerweise mit der mütterlichen Milch kompatibel. Die eine oder mehreren Zusammensetzungen vorliegenden Erfindung sind zweckmäßigerweise leicht mit Hilfe einer Sonde ohne oder mit der Milch verabreichbar, insbesondere durch dosiertes Spritzen, einfache Schwerkraft oder durch Pumpen. Sie weisen vorzugsweise eine geringe Viskosität bei 25°C, bevorzugt im Bereich von 5 bis 100 mPas, insbesondere von weniger als 40 mPas auf.

Für den Fall, dass die eine oder mehreren Zusammensetzungen nicht vollkommen homogen sind und beispielsweise als wässrige Suspensionen oder Emulsionen vorliegen, werden sie möglichst frisch, d. h. möglichst direkt vor ihrer Anwendung hergestellt. Dementsprechend werden Zusammenstellungen, umfassend die Einzelkomponenten der erfindungsgemäßen wässrigen Zusammensetzungen in mindestens zwei unterschiedlichen Fraktionen, im Rahmen der vorliegenden Erfindung ebenfalls unter Schutz gestellt.

Im Rahmen einer besonders bevorzugten Variante der vorliegenden Erfindung werden die Konzentrationen der Bestandteile der Zusammensetzungen während ihrer vorzugsweisen kontinuierlichen Verabreichung mit der Zeit geändert, um dem jeweiligen Alter des Patienten Rechnung zu tragen. So kann die Surfactant-Konzentration, insbesondere die Phospholipid-Konzentration ggf. erhöht werden, um die Lungenreifung zu beschleunigen. Weiterhin kann die Konzentration von einem oder mehreren Surfactant-Proteinen erhöht werden, um die Lungenreifung zu fördern.

Die Dauer der enteralen Ernährung kann von einigen Tagen bis zu vier Wochen, mehreren Monaten oder gar Jahren reichen.

Nach einer Einstellung im Krankenhausumgebung, in Medizin, Chirurgie und/oder Reanimation, kann die künstliche Ernährung heutzutage bei Fällen, die eine sehr lang andauernde Behandlung erfordern, zu Hause fortgesetzt werden. Zweckmäßigerweise werden durch die enterale Ernährung alle lebensnotwendigen Nährstoffe zugeführt, die der Patient nicht mehr auf dem üblichen oralen Weg aufnehmen kann. Die enterale Ernährung umfasst daher bevorzugt Zucker, Fette, Proteine, Mineralien, Spurenelemente und Vitamine.

Die enterale Ernährung ist der einfachste und physiologischste Weg der Ernährungsunterstützung. Der Verabreichungsort ist im Allgemeinen intragastrisch, mit Hilfe einer über die Nase eingeführten Sonde. Dabei erfolgt die enterale Ernährung vorzugsweise langsam, die bevorzugte Verabreichungsgeschwindigkeit liegt im Bereich von 0,1 bis 10 m/min, insbesondere im Bereich von 1 bis 3 ml/min. Weiterhin erfolgt die enterale Ernährung zweckmäßigerweise über einen längeren Zeitraum, bevorzugt innerhalb von 12 bis 24 Stunden pro Tag, und günstigerweise kontinuierlich.

Im Falle von ausgedehnten Darmschäden, die die Aufnahmefunktionen in Frage stellen, sind die verwendeten Nahrungsmittel, insbesondere Aminosäuren und/oder Monosaccharide, zweckmäßigerweise vorverdaut.

Die erfindungsmäße Zusammensetzung ist für die enterale Ernährung von
- Frühgeborenen mit einem Körpergewicht kleiner 1500 g,
- Frühgeborenen mit einem Körpergewicht von mindestens 1500 g,
- Säuglingen im Alter von 0 bis 2 Monaten,
- Säuglingen im Alter von 3 bis 5 Monaten,
- Säuglingen im Alter von 6 bis 11 Monaten,
- Kinder im Alter von 1 bis 3 Jahren,
- Kinder im Alter von 4 bis 6 Jahren,
- Kinder im Alter von 7 bis 9 Jahren,
- Kinder im Alter von 10 bis 12 Jahren,
- Kinder im Alter von 13 bis 14 Jahren,
- Jugendliche im Alter von 15 bis 18 Jahre,
- Erwachsene im Alter von 19 bis 35 Jahren,
- Erwachsene im Alter von 36 bis 50 Jahren,
- Erwachsene im Alter von 51 bis 65 Jahren und von
- Erwachsene im Alter über 65 Jahre besonders geeignet.

Die Vorteile der vorliegenden Erfindung sind vor allem bei der enteralen Ernährung von Frühgeborenen, Säuglingen im Alter von 0 bis 11 Monaten, Kinder im Alter von 1 bis 14 Jahren, Jugendlichen im Alter von 15 bis 18 Jahren, bevorzugt bei der enteralen Ernährung von Frühgeborenen, Säuglingen im Alter von 0 bis 11 Monaten, Kinder im Alter von 1 bis 14 Jahren, besonders bevorzugt Frühgeborenen, Säuglingen im Alter von 0 bis 11 Monaten zu beobachten. Sehr gute Ergebnissee werden vor allem bei der enteralen Ernährung von Frühgeborenen und Säuglingen im Alter von 0 bis 5 Monaten, vor allem bei Frühgeborenen, insbesondere bei Frühgeborenen mit einem Körpergewicht kleiner 1500 g, besonders kleiner als 750 g, festgestellt.

Die Vorteile der vorliegenden Erfindung sind vor allem bei der enteralen Ernährung von Frühgeborenen in einer Mischung mit der mütterlichen Milch zu beobachten.

Im Rahmen einer weiteren besonders bevorzugten Variante betrifft die vorliegende Erfindung eine oder mehrere Zusammensetzungen zur Anwendung in einem Verfahren zur Anwendung bei der Amnioninfusion bei einem menschlichen oder tierischen Körper, wobei mindestens eine der Zusammensetzungen vorzugsweise mindestens ein Antibiotikum umfasst.

Ein besonders bevorzugtes Anwendungsgebiet ist die Behandlung von Patientinnen mit Oligo-Anhydramnion, insbesondere die Behandlung von Patientinnen mit vorzeitigem Blasensprung (PPROM), vor allem von Schwangeren im zweiten Trimester (13. Woche bis 24. Woche) und dritten Trimester (25. Woche bis 40. Woche), insbesondere in der 23. bis 35. Woche.

Die Amnioinfusion mit der einen oder mehreren Zusammensetzungen kann auch bei Oligo/Anhydramnion anderer Ursache vorteilhaft verwendet werden (insbesondere bei Nierenagenesie, infravesikale Obstruktion, Niereninsuffizienz, Anhydramnion unklarer Ursache).

Die Amnioinfusion mit der einen oder mehrere Zusammensetzungen kann weiterhin zum Fruchtwasser-Austausch beim blutverfärbten Fruchtwasser beim feto-fetalen Transfusionssyndrom im Rahmen einer fetoskopischen Laser-Operation vorteilhaft verwendet werden. Der Fruchtwasser-Austausch kann auch mit der wässrigen Komposition bei Mekonium- oder Blut-Verunreinigung des Fruchtwassers vorteilhaft durchgeführt werden.

Die Verabreichung der einen oder mehreren Zusammensetzungen bei der Amnioninfusion kann einmalig, periodisch oder auch kontinuierlich erfolgen, wobei die Vorteile der vorliegenden Erfindung insbesondere bei einer kontinuierlichen Verabreichung zu beobachten sind.

Ansonsten kann die Verabreichung auf an sich bekannte Weise durchgeführt werden. Um Wiederholungen zu vermeiden, wird in diesem Zusammenhang auf die bekannten Amnioninfusionsverfahren, insbesondere auf die einleitend genannten Veröffentlichungen, verwiesen.

Für die kontinuierliche Amnioninfusion ist die Verwendung eines subkutan implantierten Portsystems oder eines intrauterinen Katheters (insbesondere gemäß US 9,839,767B2, EP 2614851, CN 27303360) empfehlenswert. Entsprechende Systeme werden in der Literatur beschrieben (Tchirikov et al. , Eur J Obstet Gynecol Reprod Biol. 2010; 152:30-33 und Tchirikov et al. J Perinat Med 2018 26;46(5):465-488.), deren Offenbarung hierin durch Bezugnahme explizit mit aufgenommen wird.

Die Infusionsgeschwindigkeit ist bei kontinuierlicher Amnioninfusion vorzugsweise größer 1,0 ml/h, bevorzugt größer 10,0 ml/h, besonders bevorzugt größer 50,0 ml/h, insbesondere größer 75,0 ml/h. Weiterhin ist sie vorzugsweise kleiner 1000,0 ml/h, bevorzugt kleiner 500,0 ml/h, besonders bevorzugt kleiner 250 ml/h, insbesondere kleiner 125,0 ml/h.

Die Dauer der kontinuierlichen Amnioninfusion unterliegt grundsätzlich keinen besonderen Beschränkungen. Allerdings kommen die Vorteile der vorliegenden Erfindung insbesondere bei Langzeitverabreichung der einen oder mehreren Zusammensetzungen zum Tragen. Besonders vorteilhaft ist die erfindungsgemäße Vorgehensweise für kontinuierliche Amnioninfusionen mit einer Dauer von mindestens 30 min, bevorzugt mindestens 1h, zweckmäßigerweise mindestens 2h, günstigerweise mindestens 4h, besonders bevorzugt mindestens 12h, noch mehr bevorzugt mindestens 24h, vorteilhafterweise mindestens 3 Tage, vorzugsweise mindestens eine Woche, ganz besonders bevorzugt mindestens 3 Wochen, insbesondere mindestens 6 Wochen. Dabei sind gelegentliche Unterbrechungen der kontinuierlichen Verabreichung möglich. Diese sollten aber zweckmäßigerweise nicht länger als 100% der Zeit, bevorzugt nicht länger als 50% der Zeit, seit der letzten Unterbrechung dauern.

Im Rahmen einer weiteren besonders bevorzugten Variante betrifft die vorliegende Erfindung eine oder mehrere Zusammensetzungen zur Anwendung in einem Verfahren zur Behandlung von Krankheiten des bronchopulmonalen Systems und/oder der Atemwege eines menschlichen oder tierischen Körpers, bevorzugt zur Behandlung von Patienten mittels Inhalation.

Im Rahmen noch einer weiteren besonders bevorzugten Variante betrifft die vorliegende Erfindung eine oder mehrere Zusammensetzungen zur Anwendung in einem Verfahren zur Behandlung von Hauterkrankungen eines menschlichen oder tierischen Körpers, vorzugsweise zur Behandlung von Patienten durch Auftragung der einen oder mehreren Zusammensetzungen auf die zu behandelnde Haut, insbesondere zur Behandlung von Amnion-Transplantaten.

Erfindungsgemäß umfassen die eine der mehrere Zusammensetzungen zusammengenommen die folgenden Komponenten (pro 1000 ml)

| | |
|---|---|
| Natriumchlorid | 1-10 g |
| Natriumhydrogencarbonat | 0,001-3 g |
| Kaliumchlorid | 0-1 g |
| Natrium laktat | 0-2g |
| Calciumglukonat Monohydrat | 0-3 g, bevorzugt 0,001-3 g, besonders bevorzugt 0,3-2 g, insbesondere 0,5-1 g, |
| Magnesiumchlorid Hexahydrat | 0-1g |
| Dinatrium-β-glycerophosphat-pentahydrat | 0-1g |
| Zitronensäure Monohydrat | 0-0,5g |
| Harnstoff | 0-1g |
| Kupferchlorid Dihydrat | 0-2 mg |
| Natriumselenit Pentahydrat | 0-1 mg |
| Zinkchlorid | 0-10 mg |
| Hyaluronsäure | |
| und Salze der Hyaluronsäure | 0 bis 60 g |
| Surfactant | 0- 120 g |
| sowie Wasser. | |

Die Osmolarität in allen Zusammensetzungen zusammengenommen ist zweckmäßigerweise kleiner 320,0 mosm/l. Bevorzugt ist sie kleiner 315,0 mosm/l, zweckmäßigerweise kleiner 310,0 mosm/l, besonders bevorzugt kleiner 308,0 mosm/l, zweckmäßigerweise höchstens 307,0 mosm/l, günstigerweise höchstens 306,0 mosm/l, insbesondere höchstens 305,0 mosm/l.

Weiterhin ist die Osmolarität in allen Zusammensetzungen zusammengenommen ist mindestens 240,0 mosm/l, bevorzugt mindestens 250,0 mosm/l, insbesondere mindestens 260,0 mosm/l, um eine Lyse von Zellen nach Möglichkeit zu vermeiden.

Besonders bevorzugt liegt die Osmolarität in allen Zusammensetzungen zusammengenommen im Bereich von 240,0 mosm/l bis kleiner 320,0 mosm/l, bevorzugt im Bereich von 250,0 mosm/l bis 310,0 mosm/l, insbesondere im Bereich von 260,0 mosm/l bis 305,0 mosm/l.

Die Chloridionenkonzentration in allen Zusammensetzungen zusammengenommen ist vorzugsweise höchstens 150,0 mmol/l. Zweckmäßigerweise ist die Chloridionenkonzentration höchstens 130,0 mmol/l. Bevorzugt ist sie kleiner 130,0 mmol/l, besonders bevorzugt kleiner 125,0 mmol/l, insbesondere höchstens 120,0 mmol/l.

Weiterhin ist die Chloridionenkonzentration in allen Zusammensetzungen zusammengenommen vorzugsweise mindestens 90,0 mmol/l, bevorzugt mindestens 100,0 mmol/l, insbesondere mindestens 105,0 mmol/l und liegt zweckmäßigerweise im Bereich von 90,0 mmol/l bis 175,0 mmol/l, bevorzugt im Bereich von 100,0 mmol/l bis 150,0 mmol/l, insbesondere im Bereich von 105,0 mmol/l bis 150,0 mmol/l.

Besonders bevorzugt liegt die Chloridionenkonzentration in allen Zusammensetzungen zusammengenommen im Bereich von 100,0 mmol/l bis 120,0 mmol/l, insbesondere im Bereich von 105,0 mmol/l bis 115,0 mmol/l.

Die Natriumionenkonzentration in allen Zusammensetzungen zusammengenommen ist vorzugsweise kleiner 175,0 mmol/l, zweckmäßigerweise kleiner 154,0 mmol/l. Bevorzugt ist sie kleiner 150,0 mmol/l, insbesondere höchstens 145,0 mmol/l. Andererseits ist sie vorzugsweise mindestens 120,0 mmol/l, zweckmäßigerweise größer 125,0 mmol/l, bevorzugt mindestens 130,0 mmol/l, und liegt zweckmäßigerweise im Bereich von 120,0 mmol/l bis 175,0 mmol/l, bevorzugt im Bereich von 130,0 mmol/l bis 145,0 mmol/l, insbesondere im Bereich von 130,0 mmol/l bis 140,0 mmol/l.

Die Kaliumionenkonzentration in allen Zusammensetzungen zusammengenommen liegt vorzugsweise im Bereich von 3,5 mmol/l bis 25 mmol/l, zweckmäßigerweise im Bereich von 3,5 mmol/l bis 5,2 mmol/l, insbesondere im Bereich von 3,5 mmol/l bis 4,5 mmol/l.

Die Calciumionenkonzentration in allen Zusammensetzungen zusammengenommen ist zweckmäßigerweise kleiner 8,0 mmol/l, bevorzugt kleiner 6,0 mmol/l, besonders bevorzugt kleiner 4,0 mmol/l, und liegt vorzugsweise im Bereich von 0,5 mmol/l bis 3,0 mmol, bevorzugt im Bereich von 0,5 mmol/l bis 2,5 mmol/l, insbesondere im Bereich von 1,5 mmol/l bis kleiner 2,3 mmol/l. Die Calciumionen werden den Zusammensetzungen vorzugsweise als Salz, bevorzugt als Calciumglukonat, insbesondere als Calciumglukonat Monohydrat zugesetzt.

Die Magnesiumionenkonzentration in allen Zusammensetzungen zusammengenommen liegt vorzugsweise im Bereich von 0,0 mmol/l bis 2,0 mmol/l, bevorzugt im Bereich von 0,1 mmol/l bis 1,5 mmol/l, besonders bevorzugt im Bereich von 0,1 mmol/l bis 1,0 mmol/l, insbesondere im Bereich von 0,1 mmol/l bis kleiner 0,8 mmol/l. Die Magnesiumionen werden den Zusammensetzungen vorzugsweise als Salz, bevorzugt als Magnesiumchlorid, insbesondere als Magnesiumchlorid Hexahydrat zugesetzt.

Die Harnstoffkonzentration in allen Zusammensetzungen zusammengenommen ist vorzugsweise im Bereich von 0,0 mg/dl bis 500,0 mg/dl, insbesondere im Bereich von 0,0 mg/dl bis 10,0 mg/dl.

Die Harnsäurekonzentration in allen Zusammensetzungen zusammengenommen ist vorzugsweise im Bereich von 0,0 mg/dl bis 100,0 mg/dl, insbesondere im Bereich von 0,0 mg/dl bis 5,0 mg/dl.

Die Phosphationenkonzentration in allen Zusammensetzungen zusammengenommen liegt zweckmäßigerweise im Bereich von 0,0 mg/dl bis 500,0 mg/dl, vorzugsweise im Bereich von 0,0 mg/dl bis 8,0 mg/dl, insbesondere im Bereich von 0,1 mg/dl bis 7,0 mg/dl. Die Phosphationen werden den Zusammensetzungen vorzugsweise als Salz, bevorzugt als Dinatrium-β-glycerophosphat, insbesondere als Dinatrium-β-glycerophosphat-pentahydrat zugesetzt.

Die Kohlenhydratkonzentration in allen Zusammensetzungen zusammengenommen liegt vorzugsweise im Bereich von 0,0 g/l bis 8,0 g/l.

Die Glucosekonzentration in allen Zusammensetzungen zusammengenommen liegt vorzugsweise im Bereich von 0,0 mg/dl bis 8,0 g/l, insbesondere im Bereich von 0,0 mg/dl bis 340,0 mg/dl. Sie ist bevorzugt kleiner 300,0 mg/dl, günstigerweise kleiner 200,0 mg/dl, zweckmäßigerweise kleiner 100,0 mg/dl, insbesondere kleiner 60,0 mg/dl. Im Rahmen einer ganz besonders bevorzugten Ausführungsform der vorliegenden Erfindung enthalten alle Zusammensetzungen zusammengenommen weniger als 50,0 mg/dl, günstigerweise weniger als 25,0 mg/dl, zweckmäßigerweise weniger als 10,0 mg/dl, insbesondere keine, Glucose.

Die Konzentration der Laktatsalze in allen Zusammensetzungen zusammengenommen liegt vorzugsweise im Bereich von 0,0 mmol/l bis 100,0 mmol/l, insbesondere im Bereich von 0,0 mmol/l bis 20,0 mmol/l.

Die Konzentration der Glukonatsalze in allen Zusammensetzungen zusammengenommen liegt vorzugsweise im Bereich von 0,0 mmol/l bis 100,0 mmol/l, insbesondere im Bereich von 0,0 mmol/l bis 20,0 mmol/l.

Die Konzentration der Citratsalze in allen Zusammensetzungen zusammengenommen liegt vorzugsweise im Bereich von 0,0 mg/l bis 100,0 mg/l.

Die Hydrogencarbonationenkonzentration in allen Zusammensetzungen zusammengenommen liegt vorzugsweise im Bereich von 5,0 mmol/l bis 100,0 mmol/l, bevorzugt im Bereich von 5,0 mmol/l bis 50,0 mmol/l, günstigerweise im Bereich von 5,0 mmol/l bis kleiner 24,0 mmol/l, insbesondere im Bereich von 10,0 mmol/l bis 20,0 mmol/l.

Die Gesamt-Eiweißkonzentration in allen Zusammensetzungen zusammengenommen liegt vorzugsweise im Bereich von 0,0 g/l bis 15,0 g/l, insbesondere im Bereich von 0,0 g/l bis 10,0 g/l.

Die Albuminkonzentration in allen Zusammensetzungen zusammengenommen liegt vorzugsweise im Bereich von 0,0 g/dl bis 15,0 g/dl, zweckmäßigerweise im Bereich von 0,0 g/dl bis 5,0 g/dl, insbesondere im Bereich von 0,0 g/dl bis kleiner 2,0 g/dl.

Der Anteil von Spurenelementen in allen Zusammensetzungen zusammengenommen liegt vorzugsweise im Bereich von 0,0 g/l bis 2,0 g/l.

Die Kupferionenkonzentration in allen Zusammensetzungen zusammengenommen liegt vorzugsweise im Bereich von 0,0 mg/l bis 2,0 mg/l, bevorzugt im Bereich von 0,3 mg/l bis 1 mg/l, insbesondere im Bereich von 0,4 mg/l bis 1 mg/l.

Die Selenionenkonzentration in allen Zusammensetzungen zusammengenommen liegt vorzugsweise im Bereich von 0,0 mg/dl bis 1 mg/l, bevorzugt im Bereich von 0,01 mg/l bis 0,5 mg/l, insbesondere im Bereich von 0,02 mg/l bis 0,2 mg/l.

Die Zinkionenkonzentration in allen Zusammensetzungen zusammengenommen liegt vorzugsweise im Bereich von 0,0 mg/l bis 10 mg/l, zweckmäßigerweise im Bereich von 0,0 mg/l bis 2 mg/l, bevorzugt im Bereich von 0,1 mg/l bis 0,5 mg/l.

Die Eisenionenkonzentration in allen Zusammensetzungen zusammengenommen liegt vorzugsweise im Bereich von 0,0 µg/dl bis 500,0 µg/dl.

Die Chromionenkonzentration in allen Zusammensetzungen zusammengenommen liegt vorzugsweise im Bereich von 0,0 µg/dl bis 150,0 µg/dl.

Die Aluminiumionenkonzentration in allen Zusammensetzungen zusammengenommen liegt vorzugsweise im Bereich von 0,0 µg/dl bis 200,0 µg/dl.

Die Cobaltionenkonzentration in allen Zusammensetzungen zusammengenommen liegt vorzugsweise im Bereich von 0,0 µg/dl bis 10,0 µg/dl.

Die Nickelionenkonzentration in allen Zusammensetzungen zusammengenommen liegt vorzugsweise im Bereich von 0,0 µg/dl bis 7,0 µg/dl.

Die Manganionenkonzentration in allen Zusammensetzungen zusammengenommen liegt vorzugsweise im Bereich von 0,0 µg/dl bis 15,0 µg/dl.

Die Vanadiumionenkonzentration in allen Zusammensetzungen zusammengenommen liegt vorzugsweise im Bereich von 0,0 µg/dl bis 45,0 µg/dl.

Die Arsenionenkonzentration in allen Zusammensetzungen zusammengenommen liegt vorzugsweise im Bereich von 0,0 µg/dl bis 10,0 µg/dl.

Die Rubidiumionenkonzentration in allen Zusammensetzungen zusammengenommen liegt vorzugsweise im Bereich von 0,0 µg/dl bis 20,0 µg/dl.

Die Bariumionenkonzentration in allen Zusammensetzungen zusammengenommen liegt vorzugsweise im Bereich von 0,0 µg/dl bis 35,0 µg/dl.

Die Vitamin C-Konzentration in allen Zusammensetzungen zusammengenommen liegt vorzugsweise im Bereich von 0,0 mg/l bis 20 g/l.

Die Vitamin A-Konzentration in allen Zusammensetzungen zusammengenommen liegt vorzugsweise im Bereich von 0,0 mg/l bis 1500,0 mg/l.

Die Vitamin E-Konzentration in allen Zusammensetzungen zusammengenommen liegt vorzugsweise im Bereich von 0,0 mg/l bis 75,0 g/l.

Die Vitamin K-Konzentration in allen Zusammensetzungen zusammengenommen liegt vorzugsweise im Bereich von 0,0 mg/l bis 100,0 mg/l.

Die Vitamin B1-Konzentration in allen Zusammensetzungen zusammengenommen liegt vorzugsweise im Bereich von 0,0 mg/l bis 20,0 mg/l.

Die Vitamin B2-Konzentration in allen Zusammensetzungen zusammengenommen liegt vorzugsweise im Bereich von 0,0 mg/l bis 20,0 mg/l.

Die Vitamin B3-Konzentration in allen Zusammensetzungen zusammengenommen liegt vorzugsweise im Bereich von 0,0 mg/l bis 90,0 mg/l.

Die Vitamin B5-Konzentration in allen Zusammensetzungen zusammengenommen liegt vorzugsweise im Bereich von 0,0 mg/l bis 25,0 mg/l.

Die Vitamin B7-Konzentration in allen Zusammensetzungen zusammengenommen liegt vorzugsweise im Bereich von 0,0 mg/l bis 5,0 mg/l.

Die Vitamin B9-Konzentration in allen Zusammensetzungen zusammengenommen liegt vorzugsweise im Bereich von 0,0 mg/l bis 7,0 mg/l.

Die Vitamin B12-Konzentration in allen Zusammensetzungen zusammengenommen liegt vorzugsweise im Bereich von 0,0 mg/l bis 5,0 mg/l.

Die Vitamin D-Konzentration in allen Zusammensetzungen zusammengenommen liegt vorzugsweise im Bereich von 0,0 mg/l bis 5,0 mg/l.

Die Hyaloronsäure-Konzentration in allen Zusammensetzungen zusammengenommen liegt vorzugsweise im Bereich von 0,0 mg/l bis 60 g/l

Die Hyaloronat-Konzentration in allen Zusammensetzungen zusammengenommen liegt vorzugsweise im Bereich von 0,0 mg/l bis 60 g/l.

Im Rahmen der vorliegenden Erfindung enthält die mindestens eine der Zusammensetzungen vorzugsweise mindestens ein Surfactant.

Der Begriff "Surfactant" bezeichnet erfindungsgemäß oberflächenaktive Substanzen, die in der menschlichen Lunge zu finden sind. Diese werden normalerweise von spezialisierten Lungenzellen (Pneumozyten Typ II) gebildet und schließen insbesondere Phospholipide und Surfactant-Proteine ein.

Im Rahmen einer besonders bevorzugten Ausführungsform der vorliegenden Erfindung enthält mindestens eine der Zusammensetzungen mindestens ein Phospholipid. Im Rahmen einer zweiten besonders bevorzugten Ausführungsform der vorliegenden Erfindung enthält mindestens eine der Zusammensetzungen mindestens ein Surfactant-Protein. Im Rahmen einer weiteren besonders bevorzugten Ausführungsform der vorliegenden Erfindung enthält mindestens eine der Zusammensetzungen mindestens ein Phospholipid und mindestens ein Surfactant-Protein.

Erfindungsgemäß besonders bevorzugte Phospholipide (Phosphatide) schließen fettähnliche Triglyceride ein, die zwei langkettige Fettsäuren und ein Phosphorsäurerest, an den vorzugsweise noch eine Base gebunden ist, enthalten. Ganz besonders bevorzugt werden solche Verbindungen, die in tierischen und pflanzlichen Zellen, vor allem im Gehirn, im Herzen, in der Leber, im Eidotter sowie in der Sojabohne, vorkommen.

Für die Zwecke der vorliegenden Erfindung ganz besonders vorteilhafte Phospholipide schließen O-Phosphatidyl-ethanolamin und O-Phosphatidylcholin ein. Die Stereochemie dieser Verbindungen unterliegt dabei keinen besonderen Beschränkungen. Besonders günstig ist jedoch die Verwendung des 1-sn-Phosphatidylethanolamins und des 3-sn-Phosphatidylcholins, insbesondere des 2,3-Diacyl-sn-glycero-1-Phosphoethanolamins (früher Kephalin genannt) und des 1,2-Diacyl-sn-glycero-3-Phosphocholins (früher Lecithin genannt). Der Einsatz von O-Phosphatidylcholin, bevorzugt von 3-sn-Phosphatidylcholin, insbesondere von 1,2-Diacyl-sn-glycero-3-Phosphocholin hat sich dabei ganz besonders bewährt, wobei Dipalmitoylphosphatidylcholine besonders bevorzugt werden.

Sphingolipide sind eine weitere Klasse erfindungsgemäß besonders bevorzugter Phospholipide. Hierzu gehören insbesondere Verbindungen, bei denen das Glycerin durch einen ungesättigten Aminoalkohol, bevorzugt Sphingosin, ersetzt ist. Sphingosinphosphate werden üblicherweise auch als Sphingomyelin bezeichnet.

Im Rahmen einer besonders bevorzugten Variante der vorliegenden Erfindung enthält mindestens eine der Zusammensetzungen ein Gemisch aus Phosphatiden, das neben dem mindestens einen Phospholipid eine oder mehrere gesättigte oder ungesättigte Fettsäuren, besonders bevorzugt Palmitin-, Stearin-, Öl-, Linol- und/oder Linolensäure, enthält.

Das Gewichtsverhältnis von Phospholipid zu Surfactant-Protein liegt in den erfindungsgemäßen Zusammensetzungen günstigerweise im Bereich von 5:1 bis 15:1, bevorzugt im Bereich von 8:1 bis 12:1, insbesondere im Bereich von 9:1 bis 11:1.

Für die Zwecke der vorliegenden Erfindung besonders bevorzugte Surfactant-Proteine umfassen biophysische Surfactant-Proteine, insbesondere Surfactant-Protein-B und Surfactant-Protein-C, immunologische Surfactant-Proteine, insbesondere Surfactant-Protein-A und Surfactant-Protein-D, sowie regulatorische Surfactant-Proteine.

Die Gesamtlipidkonzentration allen Zusammensetzungen zusammengenommen liegt vorzugsweise im Bereich von 0,0 mg/l bis 350 g/l, zweckmäßigerweise im Bereich von 0,0 mg/l bis 35 g/l, bevorzugt im Bereich von 1,0 mg/l bis 5 g/l, besonders bevorzugt im Bereich von 25,0 mg/l bis 1 g/l, insbesondere im Bereich von 50,0 mg/l bis 2400 mg/dl.

Die Phosphatidylcholinkonzentration in allen Zusammensetzungen zusammengenommen liegt vorzugsweise im Bereich von 0,0 mg/l bis 35 g/l, bevorzugt im Bereich von 1,0 mg/l bis 5 g/l, besonders bevorzugt im Bereich von 25,0 mg/l bis 1 g/l, insbesondere im Bereich von 50,0 mg/l bis 2400 mg/dl. Der Gewichtsanteil von Phosphatidylcholin, bezogen auf alle Lipide, ist vorzugsweise größer 50,0 Gew.-%, bevorzugt größer 70,0 Gew.-% und liegt ganz besonders bevorzugt im Bereich von 75,0 Gew.-% bis 85,0 Gew.-%.

Die Sphingomyelinkonzentration in allen Zusammensetzungen zusammengenommen liegt vorzugsweise im Bereich von 0,0 mg/l bis 35 g/l, bevorzugt im Bereich von 1,0 mg/l bis 5 g/l, besonders bevorzugt im Bereich von 25,0 mg/l bis 1 g/l, insbesondere im Bereich von 50,0 mg/l bis 2400 mg/dl. Zweckmäßigerweise wird sie analog zum natürlichen Fruchtwasser an das Alter des Fötus angepasst, wobei bei jüngeren Föten eine niedrigere Konzentration und bei älteren Föten eine höhere Konzentration gewählt wird.

Die Surfactant Protein A-Konzentration in allen Zusammensetzungen zusammengenommen liegt vorzugsweise im Bereich von 0,0 mg/l bis 5000,0 mg/l, bevorzugt im Bereich von 1,0 mg/l bis 1000,0 mg/l, besonders bevorzugt im Bereich von 2,0 mg/l bis 500,0 mg/l, insbesondere im Bereich von 3,0 mg/l bis 500,0 mg/l.

Die Surfactant Protein B-Konzentration in allen Zusammensetzungen zusammengenommen liegt vorzugsweise im Bereich von 0,0 mg/l bis 1000,0 mg/l, bevorzugt im Bereich von 0,1 mg/l bis 500,0 mg/l, besonders bevorzugt im Bereich von 0,2 mg/l bis 100,0 mg/l, insbesondere im Bereich von 0,3 mg/l bis 80,0 mg/l.

Die Surfactant Protein D-Konzentration in allen Zusammensetzungen zusammengenommen liegt vorzugsweise im Bereich von 0,0 mg/l bis 1000,0 mg/l, bevorzugt im Bereich von 1,0 mg/l bis 500,0 mg/l, besonders bevorzugt im Bereich von 2,0 mg/l bis 300,0 mg/l, insbesondere im Bereich von 3,0 mg/l bis 100,0 mg/l.

Der pH-Wert aller Zusammensetzungen zusammengenommen, gemessen bei 20°C, liegt vorzugsweise im Bereich von 6,5 bis 9,0, bevorzugt im Bereich von 7,0 bis 8,6, insbesondere im Bereich von 7,5 bis 8,5.

Die Lipoproteinkonzentration aller Zusammensetzungen zusammengenommen liegt vorzugsweise im Bereich von 0,0 mg/l bis 1,0 g/l, bevorzugt im Bereich von 1,0 mg/l bis 500,0 mg/l, besonders bevorzugt im Bereich von 5,0 mg/l bis 250,0 mg/l, insbesondere im Bereich von 50,0 mg/l bis 125,0 mg/l.

Besonders bewährt hat sich in diesem Zusammenhang die Verwendung von Zusammensetzungen, deren Gesamtlipidanteil aller Phospholipide für alle Zusammensetzungen zusammengenommen größer 35 g/l, bevorzugt größer 10g/l, besonders bevorzugt größer 5 g/l, insbesondere größer 240 mg/l ist.

Weiterhin enthalten mindestens einer der Zusammensetzungen Wasser. Dessen Anteil, jeweils bezogen auf das Gesamtgewicht aller Zusammensetzungen, beträgt vorzugsweise mindestens 50,0 Gew.-%, bevorzugt mindestens 70,0 Gew.-%, insbesondere mindestens 90,0 Gew.-%lm Rahmen einer besonders bevorzugten Variante der vorliegenden Erfindung enthalten Zusammensetzungen keine weiteren Lösungsmittel.

Der Anteil von weiteren Komponenten als den in dieser Anmeldung explizit genannten essentiellen und optionalen Bestandteilen ist vorzugsweise kleiner 5,0 Gew.-%, bevorzugt kleiner 2,5 Gew.-%, besonders bevorzugt kleiner 1,0 Gew.-%, insbesondere kleiner 0,5 Gew.-%, jeweils bezogen auf das Gesamtgewicht aller Zusammensetzungen. Im Rahmen einer besonders vorteilhaften Variante der vorliegenden Erfindung bestehen die Zusammensetzungen ausschließlich aus den in dieser Anmeldung explizit genannten essentiellen und optionalen Komponenten.

Im Rahmen der vorliegenden Erfindung enthalten alle Zusammensetzungen zusammengenommen zweckmäßigerweise weniger als 70 µl⁻¹, vorzugsweise weniger als 50 µl⁻¹, bevorzugt weniger als 25 µl⁻¹, günstigerweise weniger als 10 µl⁻¹, besonders bevorzugt weniger als
5,0 µl⁻¹, ganz besonders bevorzugt weniger als 1,0 µl⁻¹, insbesondere keine, Amnionzellen.

Weiterhin enthalten alle Zusammensetzungen zusammengenommen vorzugsweise weniger als 750 µl⁻¹, vorteilhafterweise weniger als 500 µl⁻¹, bevorzugt weniger als 250 µl⁻¹, günstigerweise weniger als 100 µl⁻¹, besonders bevorzugt weniger als 50,0 µl⁻¹, ganz besonders bevorzugt weniger als 25,0 µl⁻¹, noch mehr bevorzugt weniger als 10,0 µl⁻¹, zweckmäßigerweise weniger als 1,0 µl⁻¹, insbesondere keine, fetale Zellen.

Die Herstellung der einen oder mehreren Zusammensetzungen kann auf an sich bekannte Weise durch Mischen von Komponenten in den jeweiligen Einsatzmengen erfolgen. Alternativ ist auch das Auflösen von Zusammensetzungen, die die gewünschten Komponenten in den gewünschten Mengenverhältnissen enthalten, in Wasser oder die Verdünnung von wässrigen Konzentraten auf die gewünschte Konzentration denkbar.

Ansonsten sollte auf eine möglichst sterile Herstellung der einen oder mehreren Zusammensetzungen geachtet werden, damit die resultierenden Zusammensetzungen auch nach längerer Lagerung, von beispielsweise einem Jahr oder länger, möglichst frei von vermehrungsfähigen Keimen, insbesondere von Mikroorganismen und Viren, sind.

Im Rahmen einer besonders bevorzugten Variante der vorliegenden Erfindung umfassen die eine oder mehrere Zusammensetzungen, insbesondere zur Anwendung bei der Amnioninfusion bei einem menschlichen oder tierischen Körper, weiterhin mindestens ein Antibiotikum, bevorzugt ein bakteriostatisches und/oder ein bakterizides Antibiotikum, zweckmäßigerweise ein β-Lactam-Antibiotikum, ein Glykopeptid, ein Polyketid, ein Glycylcyclin, ein Makrolid-Antibiotikum, ein Ketolid, ein Lincosamid, ein Aminoglykosid-Antibiotikum, ein Polypeptid-Antibiotikum, ein Lipopeptid-Antibiotikum, ein Epoxid-Antibiotikum, ein Chinolon-Antibiotikum, ein Streptogramin, ein Sulfonamid, ein Oxazolidinon, ein Ansamycin und/oder ein Nitroimidazol, besonders bevorzugt ein Makrolid-Antibiotikum, zweckmäßigerweise Clarithromycin und/oder Azithromycin insbesondere Clarithromycin.

Eine für die Zwecke der vorliegenden Erfindung ganz besonders geeignete Zusammensetzung "Top" umfasst Natriumchlorid, Natriumhydrogencarbonat, Kaliumchlorid, Natriumlaktat, Calciumglukonat, Magnesiumchlorid, Dinatrium-β-glycerophosphat, Zitrinensäure, ggf. Harnstoff, Kupferclorid, Natriumselenit, Zinkchlorid sowie Wasser. Die Zusammensetzung dieser Zusammensetzung entspricht vorzugsweise der von Amnion Flush Solution (Serumwerk AG, Bernburg, Deutschland).

Diese Zusammensetzung wird zweckmäßigerweise in Kombination mit einem Surfactant, idealerweise handelt es ich hierbei um Phospholipide aus Schweinelunge oder Rinderlunge, insbesondere SURVANTA, CUROSURF oder Alveofact, in einem Verfahren zur enteralen Ernährung, zur enteralen Ernährungsergänzung und/oder zur Behandlung und/oder Versorgung von kapillararmen Schichten, bevorzugt zur Behandlung von Krankheiten des menschlichen oder tierischen Auges, insbesondere zur Behandlung von Patienten mit Sicca-Syndrom, einer Störung der Meibomdrüsen und/oder mit einer Störung infolge von Kontaktlinsentragen; zur enteralen Ernährung eines menschlichen oder tierischen Körpers; zur Anwendung bei der Amnioninfusion bei einem menschlichen oder tierischen Körper, bevorzugt zur Behandlung eines Oligo-Anhydramnion, insbesondere eines vorzeitigen Blasensprungs, vorzugsweise zur Anwendung bei einer kontinuierlichen Amnioninfusion; zur Behandlung von Krankheiten des bronchopulmonalen Systems und/oder der Atemwege eines menschlichen oder tierischen Körpers, bevorzugt zur Behandlung von Patienten mittels Inhalation; zur Behandlung von Hauterkrankungen eines menschlichen oder tierischen Körpers, vorzugsweise zur Behandlung von Patienten durch Auftragung der einen oder mehreren Zusammensetzungen auf die zu behandelnde Haut, insbesondere zur Behandlung von Amnion-Transplantaten eingesetzt.

Das Mengenverhältnis der Zusammensetzung "Top", insbesondere Amnion Flush Solution (Serumwerk, Deutschland), zum Surfactant, idealerweise handelt es sich hierbei um Phospholipide aus Rinderlunge oder Schweinelunge, insbesondere SURVANTA, CUROSURF oder ALVEOFACT, liegt vorzugsweise im Bereich von 1000 ml zu 1 mg bis 35 g Surfactant, bevorzugt im Bereich von 1000 ml zu 25 mg bis 5 g Surfactant, insbesondere im Bereich von 1000 ml zu 90 mg bis 2400 mg Surfactant.

Im Rahmen einer ganz besonders bevorzugten Ausführungsform der vorliegenden Erfindung werden eine oder mehrere Zusammensetzungen, bevorzugt die Zusammensetzung "Top", insbesondere Amnion Flush Solution (Serumwerk, Deutschland), zusammen mit Hyaloronsäure eingesetzt. Weiterhin wird vorzugsweise Surfactant ergänzt, idealerweise handelt es ich hierbei um Phospholipide aus Rinderlunge, insbesondere SURVANTA, CUROSURF oder ALVEOFACT.

### Bevorzugte Varianten:

### Variante 1

| | |
|---|---|
| Natriumchlorid | 6,172 g |
| Natriumhydrogencarbonat | 1,42 g |
| Kaliumchlorid) | 0,291 g |
| Natriumlaktat | 1,02 g |
| Calciumglukonat Monohydrat | 0,717 g |
| Magnesiumchlorid Hexahydrat | 0,116 g |
| Dinatrium-β-glycerophosphat-pentahydrat | 0,107 g |
| Zitronensäure Monohydrat | 0,074 g |
| Harnstoff | 0 bis 0,10 g |
| Kupferchlorid Dihydrat | 0,429 bis 1 mg |
| Natriumselenit Pentahydrat | 0,039 bis 1,0 mg |
| Zinkchlorid | 0,354 bis 10 mg |
| Surfactant | 0 - 120 g |
| Hyaluronsäure und/oder Salze der | |
| Hyaluronsäure | 0 bis 100 g |
| Wasser für Injektionszwecke | ad 1,000 ml |

### Variante 2

| | |
|---|---|
| Natriumchlorid | 6,172 g |
| Natriumhydrogencarbonat | 1,42 g |
| Kaliumchlorid) | 0,291 g |
| Natriumlaktat | 1,02 g |
| Calciumglukonat Monohydrat | 0,717 g |
| Magnesiumchlorid Hexahydrat | 0,116 g |
| Dinatrium-β-glycerophosphat-pentahydrat | 0,107 g |
| Zitronensäure Monohydrat | 0,074 g |
| Harnstoff | 0 bis 0,10 g |
| Kupferchlorid Dihydrat | 0,429 bis 1 mg |
| Natriumselenit Pentahydrat | 0,039 bis 1,0 mg |
| Zinkchlorid | 0,354 bis 10 mg |
| Surfactant | 0 - 120 g |
| Hyaluronsäure und/oder Salze der | |
| Hyaluronsäure | 0-100 g |
| Wasser für Injektionszwecke | ad 1,000 ml |

### Variante 3

| | |
|---|---|
| Natriumchlorid | 6,172 g |
| Natriumhydrogencarbonat | 1,42 g |
| Kaliumchlorid) | 0,291 g |
| Natriumlaktat | 1,02 g |
| Calciumglukonat Monohydrat | 0,717 g |
| Magnesiumchlorid Hexahydrat | 0,116 g |
| Dinatrium-β-glycerophosphat-pentahydrat | 0,107 g |
| Zitronensäure Monohydrat | 0,074 g |
| Harnstoff | 0 bis 0,10 g |
| Kupferchlorid Dihydrat | 0,429 bis 1 mg |
| Natriumselenit Pentahydrat | 0,039 bis 1,0 mg |
| Zinkchlorid | 0,354 bis 10 mg |
| Surfactant | 0 - 120 g |
| und / oder | |
| Curosurf (Chiesi GmbH) | 120 - 12000 mg |
| Wasser für Injektionszwecke | ad 1,000 ml |

### Variante 4

| | |
|---|---|
| Natriumchlorid | 6,172 g |
| Natriumhydrogencarbonat | 1,42 g |
| Kaliumchlorid) | 0,291 g |
| Natriumlaktat | 1,02 g |
| Calciumglukonat Monohydrat | 0,717 g |
| Magnesiumchlorid Hexahydrat | 0,116 g |
| Dinatrium-β-glycerophosphat-pentahydrat | 0,107 g |
| Zitronensäure Monohydrat | 0,074 g |
| Harnstoff | 0 bis 0,10 g |
| Kupferchlorid Dihydrat | 0,429 bis 1 mg |
| Natriumselenit Pentahydrat | 0,039 bis 1 mg |
| Zinkchlorid | 0,354 bis 10 mg |
| Hyaluronsäure | |
| und/oder Salze der Hyaluronsäure | 0 bis 100 g |
| Wasser für Injektionszwecke | ad 1,000 ml |

### Verpackung: bevorzugt 1ml bis 1000 ml

Surfactant und ggf. Hyaluronsäure oder Salze der Hyaluronsäure können zur Grund-Lösung von der Anwendung eingegeben werden.

Im Folgenden wird die vorliegende Erfindung durch Beispiele und Vergleichsbeispiele weiter veranschaulicht, ohne dass hierdurch eine Beschränkung des Erfindungsgedankens erfolgen soll.

Die Bestimmung der Kapillardichte erfolgte mittels Videokapilloskopie.

**Beispiel 1:** Versorgung der Cornea (kapillarlose Schicht) und Augenlieder bei Patienten mit Sicca-Syndrom (Trockene Augen) oder mit einer Dysfunktion von Meibom Drüsen (Tarsaldrüsen) und Augen-Dysfunktion bei Kontaktlinsen-Träger.

### Test 1a (Lösung-Variante 3)

Amnion Flush Solution (100 ml; Serumwerk AG, Bernburg, Deutschland) mit Surfactant (Alveofact 45 mg; Lyomark Pharma GmbH, Oberhaching, Deutschland) und 0,1% Natrumhyaluronat wurden bei 3 Probanden mit einem ausgeprägten langjährigen Sicca-Syndrom und bei zwei Probanden mit einer relativen "Unverträglichkeit" der Kontaktlinsen als Augentropfen 2 bis 3 Tropfen in jedes Auge (0,1 bis 0,5 ml)), 6- bis 8-mal täglich über 2 Wochen gegeben. Bei allen Probanden wurde bereits innerhalb der ersten 3 Tage der Behandlung eine signifikante Verbesserung der Augenfunktion und vollständiges Verschwinden der Augenbeschwerden festgestellt. Die Ergänzung der Cornea-Ernährung zeigte keine Nebenwirkungen innerhalb der Testphase und im folgenden Monat. Insgesamt wurden die Augentropfen sehr gut vertragen.

### Test 1b

Bei 3 weiteren Patienten mit Sicca-Syndrom wurde Surfactant CUROSURF 120 mg (Chiesi GmbH) mit 100 ml Amnion Flush Solution (Serumwerk AG, Bernburg, Deutschland) und 0,1% Natrumhyaluronat erfolgreich über 2 Wochen verwendet.

### Test 1c

Bei 2 weiteren Patienten wurde die Variante 2: Amnion Flush Solution (100 ml; Serumwerk AG, Bernburg, Deutschland) mit Surfactant (Alveofact 45 mg; Lyomark Pharma GmbH, Oberhaching, Deutschland) und 0.5 g Hyaluronsäure 3mal täglich und auch abends vor dem Einschlafen erfolgreich verwendet. Durch die zusätzliche Anwendung der Hyaluronsäure entstand ein Serum, das zur besseren Affinität und auch Haftung an der Kornea-Oberfläche signifikant beigetragen hat. Die Symptome des Sicca-Syndroms verschwanden bereits unter der Therapie am nächsten Tag.

### Test 2

Die Augentropfen gemäß Beispiel 1, Test 1a,b, Lösung-Variante A und B mit 0,1% Natrumhyaluronat wurden bei 2 reifen Neugeborenen für die Behandlung einer Konjunktivitis nach der Credéschen-Augenprophylaxe nach der Geburt erfolgreich verwendet. Die Rötung verschwand innerhalb von 2 Tagen.

### Beispiel 2

In einem Heilversuch konnte bei einem extrem Frühgeborenen < 750 g statt eines kompletten Verzichts auf die enterale Versorgung für 14 Tagen, die Fortsetzung der physiologischen intrauterinen enteralen Fruchtwassereinnahme mit Surfactant gefolgt durch die sanfte Umstellung auf die Milchernährung ab dem 3. Tag erfolgreich implementieren werden.

### Test 1 (enterale Ernährung)

Eine Schwangere in der 24. Schwangerschaftswoche wurde von ihrer Frauenärztin wegen eines vorzeitigen hohen Blasensprunges eingewiesen. In der Klinik wurde die PPROM Behandlung nach den aktuellen DGGG-Leitlinien durchgeführt. Leider musste die Patientin frühzeitig per Kaiserschnitt entbunden werden. Das männliche Frühgeborene hatte ein Geburtsgewicht von 580 g. Das Frühgeborene erhielt über die Magensonde 12 ml Lösung (12h postnatal, 1 ml alle 2 St., Amnion Flush Solution, Serumwerk AG, Bernburg, Deutschland mit 45 mg Surfactant Lyomark Pharma GmbH, Oberhaching, Deutschland). Am 2. Tag wurden die Flüssigkeitsmengen entsprechend auf 24 ml Lösung angepasst die Ernährung bestand aus 8 ml der mütterlichen Milch und 16 ml der erfindungsgemäßen Lösung mit Surfactant. Ab dem 3. Tag wurde die Lösung mit der mütterlichen Milch 12 ml kombiniert (insgesamt 36 ml Lösung). Ab dem 8. Lebenstag bestand die Nahrung aus einer Hälfte der Milch und einer Hälfte des künstlichen Fruchtwassers. An Tag 12 bestand die Nahrung nur noch zu einem Drittel aus Amnion Flush Solution und 45 mg Surfactant und 2/3 der mütterlichen Milch. Am 15. Tag hat das Neugeborene nur noch 90 ml Milch erhalten (160-180 ml/kg/d). Die parenterale Ernährung wurde laborchemisch mit der Messung von Blutgasanalyse, Blutbild, Blutglukose, Elektrolyte, Kreatinin, Harnstoff, Leberwerte, Triglyzeride,_Cholesterin und Protein überwacht._Das Kind konnte aus der Klinik nach dem Abschluss der Therapie und Erreichen der Reife ohne pathologischen Befund im guten allgemeinen Zustand entlassen werden.

### Test 2

Nach der Geburt konnte der neugeborene Junge wegen Surfactant-Mangel über 2 Tage lang durch die zusätzliche Sauerstoffgabe (bis 50% O₂) nicht dauerhaft stabilisiert werden. Das Kind hat enteral 20 ml Amnion Flush Solution, Serumwerk AG und 120 mg Surfactant über eine Magensonde erhalten. Bereits 2 Stunden später ist die Sättigung auf 99% angestiegen. Die Sauerstoffkonzentration wurde auf 21% (O₂ Luftkonzentration) gesenkt. Die 2. enterale Applikation erfolgte am nächsten Tag. Ein Tag später wurde das Kind im Wohlbefinden nach Hause entlassen. Ein Jahr später ist das Kind weiterhin kerngesund.

Beispiel 3: Ernährung des Amnions (kapillarlose Schicht), der Nabelschnur (kapillarlose Schicht), der fetalen Schleimhaut, der Haut und enterale Versorgung des Fetus nach dem vorzeitigen Blasensprung mit Anhydramnion

Amnion Flush Solution (1000 ml) mit Surfactant (45 mg) wurde bei 2 Patienten mit einem vorzeitigen Blasensprung mit Anhydramion getestet.

### Test 1

Bei der ersten Patientin lag der vorzeitige Blasensprung in der 22. Schwangerschaftswoche vor. Sonographisch zeigte sich ein Anhydramnion bei einem fetalen Schätzgewicht von 485 g (< 5. Perzentile). Nach ausführlicher Aufklärung der Patientin, interdisziplinärem Konsens wurde ein prenatal KatheterSystem zur kontinuierlichen intraamnialen Auffüllung angelegt. Es wurde Amnion Flush Solution (Serumwerk, Deutschland) kombiniert mit Surfactant (45 mg/l) und Clarithromycin (500 mg/d) über einen Infusiomaten (2400 ml/d) verwendet. Die Patientin wurde systemisch mit Ampicillin und Clarithromycin behandelt.

Die Schwangerschaft konnte bis zur 26+5 v. SSW prolongiert werden. Aufgrund einer verstärkten vaginalen Blutung erfolgte bei Verdacht auf Plazentalösung die Schnittentbindung. Eutrophes Mädchen Gewicht: 920 g (P.53) Länge: 34 cm (P.40) Kopfumfang: 24,4 cm (P40) pH-Wert der Nabelschnurarterie: 7,37 pH-Wert der Nabelschnurvene: 7,44 APGAR: 7/6/8. Das Kind konnte nach initialer Stabilisierung der Atmung mit einer prophylaktischen Surfactantgabe und Anlage einer pharyngealen Atemhilfe und unauffälligen Infektionsparametern (CRP <1,0 mg/l; IL-6: 23,4 pg/ml) kreislaufstabil in unsere Neonatologie verlegt werden. Das Mädchen entwickelte sich bis auf zwei Infektionsepisoden im Rahmen einer Frühgeburtlichkeit und einen offenen *Ductus arteriosus* unauffällig. Entlassung nach 43 Behandlungstagen im guten allgemeinen Zustand.

### Test 2

Bei der zweiten 32-jährigen Patientin mit dem vorzeitigen Blasensprung in der 27. Schwangerschaftswoche wurde die kontinuierliche Amnioinfusion mit dem Surfactant nach dem o.g. Schema in der 28 Schwangerschaftswoche gestartet. Die Schwangerschaft konnte um weitere 22 Tage bis zur 32. SSW prolongiert werden. Das Kind wog bei der Geburt 1700g, APGAR 6/8/8, arterieller Nabelschnur-pH Wert 7.35. Das Kind konnte ebenso nach dem Erreichen der Reife gesund nach Hause entlassen werden.

Beispiel 4 Versorgung der Schleimhäute der Atemwege und Lunge

### Test 1

Bei 4 Probanden (2 Erwachsene und 2 Kinder (3 und 7-Jährige) mit einer Bronchitis wurde 20 ml der Amnion Flush Solution ((Serumwerk AG, Bernburg, Deutschland) mit 45 mg Surfactant (Lyomark Pharma GmbH, Oberhaching, Deutschland, ein Erwachender und ein Kind) und mit CUROSURF 120 mg (Chiesi GmbH) mit 100 ml Amnion Flush Solution (Serumwerk AG, Bernburg, Deutschland, ein Erwachender und ein Kind) mit einem Inhalationsgerät (Pari Junior BOY S PARI GmbH, Starnberg, Deutschland) 3-mal täglich über 15 min als Nebel appliziert. Bereits nach der ersten Anwendung milderten sich die Hustensymptome signifikant.

### Beispiel 5

### Test 1

Bei 2 Probanden mit einer Hautverbrennung II° und III° (kapillararme Schicht nach der Verbrennung mit einer Kapillardichte < 50/mm²) < 5% der Gesamtoberfläche wurde das Serum bestehend aus 100 ml Amnion Flush Solution (Seromwerk AG, Bernburg, Deutschland, 3% Hyaluronsäure und 120 mg Surfactant (Curosurf, Chiesi GmbH, Italien) behandelt. Das Serum wurde auf die verletzte Oberfläche 4-5mal tgl. über 1 Woche aufgetragen. Es kam beim Probanden mit der Hautverbrennung II° bereits nach 24h zu einer deutlichen Rückbildung von der Rötung und Schmerzen. Komplette Heilung ohne Narbenbildung wurde in 10 Tagen festgestellt. Die Hautregeneration im Bereich der Blasen verlief schmerzlos und ohne sekundäre Entzündung.

### Test 2

Bei einem 3-jährigen Kind mit der Verbrennung III° (kapillararme Schicht nach der Verbrennung mit einer Kapillardichte < 30/mm²) wurde die Variante 4 Amnion Flush Solution 500 ml mit 1 g Natrumhyaluronat auf die verletzte Haut 3mal tgl. über 1 Woche lang aufgetragen. Es kam zur Verbesserung und zu deutlicher Schmerzlinderung nach 2 Tagen der Serumanwendung. Eine komplette Heilung mit einer sehr dezenten Narbenbildung konnte nach bereits 2 Wochen festgestellt werden.

Die wesentlichen Aspekte der vorliegenden Erfindung werden in den folgenden Bestimmungen zusammengefasst, ohne dass hierdurch eine Beschränkung des Erfindungsgedankens erfolgen soll.
A1) Eine oder mehrere Zusammensetzungen zur Anwendung in einem Verfahren zur enteralen Ernährung, zur enteralen Ernährungsergänzung und/oder zur Behandlung von kapillararmen Schichten mit einer Kapillardichte von 0 bis 50/mm², dadurch gekennzeichnet, dass die eine der mehrere Zusammensetzungen zusammengenommen die folgenden Komponenten (pro 1000 ml)

| | |
|---|---|
| Natriumchlorid | 1-10 g |
| Natriumhydrogencarbonat | 0,001-3 g |
| Kaliumchlorid | 0-1 g |
| Natrium laktat | 0-2g |
| Calciumglukonat Monohydrat | 0-1 g |
| Magnesiumchlorid Hexahydrat | 0-1g |
| Dinatrium-β-glycerophosphat-pentahydrat | 0-1g |
| Zitronensäure Monohydrat | 0-0,5g |
| Harnstoff | 0-1g |
| Kupferchlorid Dihydrat | 0-2 mg |
| Natriumselenit Pentahydrat | 0-1 mg |
| Zinkchlorid | 0-10 mg |
| Hyaluronsäure | |
| und Salze der Hyaluronsäure | 0 bis 60 g |
| Surfactant | 0 - 120 g |
| sowie Wasser | |
| umfassen. | |

A2) Eine oder mehrere Zusammensetzungen nach Bestimmung A1) zur Behandlung von kapillararmen Schichten mit einer Kapillardichte von 0 bis 30/mm².
A3) Eine oder mehrere Zusammensetzungen nach Bestimmung A1) oder A2) zur Behandlung von kapillarlosen Schichten.
A4) Eine oder mehrere Zusammensetzungen nach mindestens einer der vorangehenden Bestimmungen, dadurch gekennzeichnet, dass mindestens eine der Zusammensetzungen Hyaluronsäure oder Salze der Hyaluronsäure enthält.
A5) Eine oder mehrere Zusammensetzungen nach mindestens einer der vorangehenden Bestimmungen, dadurch gekennzeichnet, dass mindestens eine der Zusammensetzungen Surfactant enthält.
A6) Eine oder mehrere Zusammensetzungen nach mindestens einer der vorangehenden Bestimmungen, dadurch gekennzeichnet, dass die Zusammensetzungen, bezogen auf das Gesamtgewicht aller Zusammensetzungen, zusammengenommen die folgenden Komponenten
o 120-175 mmol/l Natrium,
o 3,5-40 mmol/l Kalium,
o 0,5-2,5 mmol/l Calcium,
o 0-2 mmol/l Magnesium,
o 100-150 mmol/l Chlorid,
∘ 0-500 mg/l Harnstoff,
∘ 0-100 mg/l Harnsäure,
∘ 0-500 mg/l Phosphat,
∘ 0-8 g/l Kohlenhydrate,
∘ 0-8 g/l Glucose,
∘ 0-100 mmol/l Laktat,
o 0-100 mmol/l Gluconat,
o 0-100 mg/l Citrat,
∘ 5-100 mmol/l Hydrogencarbonat,
∘ 0-1.0 g/l Dinatrium-β-glycerophosphat-pentahydrat
∘ 0-15 g/l Gesamt-Eiweiß,
o 0-15 g/l Albumin,
∘ 0-2 g/l Spurenelemente,
∘ 0-2 mg/l Kupfer,
∘ 0-1 mg/l Selen,
∘ 0-5 mg/l Eisen,
∘ 0-1,5 mg/l Chrom,
∘ 0-2 mg/l Aluminium,
∘ 0-0,1 mg/l Cobalt,
∘ 0-70 mg/l Nickel,
∘ 0-1,5 mg/l Mangan,
∘ 0-4,5 mg/l Vanadium,
∘ 0-0,1 mg/l Arsen,
∘ 0-0,2 mg/l Rubidium
∘ 0-0,35 mg/l Barium,
∘ 0-10 mg/l Zink,
∘ 0-20 g/l Vitamin C,
∘ 0-1500 mg/l Vitamin A,
∘ 0-75 g/l Vitamin E
∘ 0-100 mg/l Vitamin K,
∘ 0-20 mg/l Vitamin B1,
∘ 0-20 mg/l Vitamin B2,
∘ 0-90 mg/l Vitamin B3,
∘ 0-25 mg/l Vitamin B5,
∘ 0-5 mg/l Vitamin B7,
∘ 0-7 mg/l Vitamin B9,
∘ 0-5 mg/l Vitamin B12,
∘ 0-5 mg/l Vitamin D,
∘ 0-60 g/l Hyaluronsäure und Salze der Hyaluronsäure,
∘ 0-120 g/l Surfactant,
∘ 0-350 g/l Fettsäuren
∘ Wasser umfassen und
zusammen vorzugsweise einen pH im Bereich von 7 bis 8 aufweisen.
A7) Eine oder mehrere Zusammensetzungen nach mindestens einer der vorangehenden Bestimmungen, dadurch gekennzeichnet, dass mindestens eine der Zusammensetzung Glucose, Dextrose, Phospholipide, Surfactant, Lipide, Aminosäuren, Wachstumsfaktoren, Albumin, Proteine, Spurenelemente und/oder Vitamine umfasst.
A8) Eine oder mehrere Zusammensetzungen nach mindestens einer der vorangehenden Bestimmungen, dadurch gekennzeichnet, dass mindestens eine der Zusammensetzungen ein Antibiotikum umfasst.
A9) Eine oder mehrere Zusammensetzungen nach Bestimmung A9), dadurch gekennzeichnet, dass die Zusammensetzung ein β-Lactam-Antibiotikum, ein Glykopeptid, ein Polyketid, ein Tetracyclin, ein Glycylcyclin, ein Makrolid-Antibiotikum, ein Ketolid, ein Lincosamid, ein Aminoglykosid-Antibiotikum, ein Polypeptid-Antibiotikum, ein Lipopeptid-Antibiotikum, ein Epoxid-Antibiotikum, ein Chinolon-Antibiotikum, ein Streptogramin, ein Sulfonamid, ein Oxazolidinon, ein Ansamycin und/oder ein Nitroimidazol oder Azithromycin umfasst.
A10) Eine oder mehrere Zusammensetzungen nach mindestens einer der vorangehenden Bestimmungen, dadurch gekennzeichnet, dass die eine oder mehrere Zusammensetzungen als Serum oder Gel verwendet werden.
A11) Eine oder mehrere Zusammensetzungen nach mindestens einer der vorangehenden Bestimmungen zur Behandlung von Krankheiten des menschlichen oder tierischen Auges, insbesondere zur Behandlung von Patienten mit Sicca-Syndrom, einer Störung der Meibomdrüsen und/oder mit einer Störung infolge von Kontaktlinsentragen; zur enteralen Ernährung eines menschlichen oder tierischen Körpers; zur Anwendung bei der Amnioninfusion bei einem menschlichen oder tierischen Körper, bevorzugt zur Behandlung eines Oligo-Anhydramnion, insbesondere eines vorzeitigen Blasensprungs, vorzugsweise zur Anwendung bei einer kontinuierlichen Amnioninfusion; zur Behandlung von Krankheiten des bronchopulmonalen Systems und/oder der Atemwege eines menschlichen oder tierischen Körpers, bevorzugt zur Behandlung von Patienten mittels Inhalation, insbesondere zur Behandlung eines RDS-Syndroms; zur Behandlung von Hauterkrankungen eines menschlichen oder tierischen Körpers, vorzugsweise zur Behandlung von Patienten durch Auftragung der einen oder mehreren Zusammensetzungen auf die zu behandelnde Haut, insbesondere zur Versorgung von Amnion-Transplantaten.
A12) Eine oder mehrere Zusammensetzungen nach mindestens einen der vorangehenden Bestimmungen zur Behandlung von kapillararmen Schichten in einem künstlichen Gebärmutter-System.
A13) Eine oder mehrere Zusammensetzungen nach mindestens einen der vorangehenden Bestimmungen zur Behandlung von kapillararmen Schichten, insbesondere Amnion und Kornea, vor und nach der einer Transplantation.
B1) Eine oder mehrere Zusammensetzungen zur Anwendung in einem Verfahren zur Behandlung von kapillararmen Schichten mit einer Kapillardichte von 0 bis 50/mm² zur Behandlung von Krankheiten des menschlichen oder tierischen Auges, dadurch gekennzeichnet, dass die eine der mehrere Zusammensetzungen zusammengenommen die folgenden Komponenten (pro 1000 ml)

| | |
|---|---|
| Natriumchlorid | 1-10 g |
| Natriumhydrogencarbonat | 0,001-3 g |
| Kaliumchlorid | 0-1 g |
| Natrium laktat | 0-2g |
| Calciumglukonat Monohydrat | 0-1 g |
| Magnesiumchlorid Hexahydrat | 0-1g |
| Dinatrium-β-glycerophosphat-pentahydrat | 0-1g |
| Zitronensäure Monohydrat | 0-0,5g |
| Harnstoff | 0-1g |
| Kupferchlorid Dihydrat | 0-2 mg |
| Natriumselenit Pentahydrat | 0-1 mg |
| Zinkchlorid | 0-10 mg |
| Hyaluronsäure | |
| und Salze der Hyaluronsäure | 0 bis 60 g |
| Surfactant | 0 - 120 g |
| sowie Wasser | |
| umfassen. | |

B2) Eine oder mehrere Zusammensetzungen nach Bestimmung A1) zur Behandlung von kapillararmen Schichten mit einer Kapillardichte von 0 bis 30/mm².
B3) Eine oder mehrere Zusammensetzungen nach Bestimmung A1) oder A2) zur Behandlung von kapillarlosen Schichten.
B4) Eine oder mehrere Zusammensetzungen nach mindestens einer der vorangehenden Bestimmungen B1) bis B3) zur Behandlung von Patienten mit Sicca-Syndrom, einer Störung der Meibomdrüsen und/oder mit einer Störung infolge von Kontaktlinsentragen.
B5) Eine oder mehrere Zusammensetzungen nach mindestens einer der vorangehenden Bestimmungen B1) bis B4), dadurch gekennzeichnet, dass mindestens eine der Zusammensetzungen Hyaluronsäure oder Salze der Hyaluronsäure enthält.
B6) Eine oder mehrere Zusammensetzungen nach mindestens einer der vorangehenden Bestimmungen B1) bis B5), dadurch gekennzeichnet, dass mindestens eine der Zusammensetzungen Surfactant enthält.
B7) Eine oder mehrere Zusammensetzungen nach mindestens einer der vorangehenden Bestimmungen B1) bis B6), dadurch gekennzeichnet, dass die Zusammensetzungen, bezogen auf das Gesamtgewicht aller Zusammensetzungen, zusammengenommen die folgenden Komponenten
∘ 120-175 mmol/l Natrium,
∘ 3,5-40 mmol/l Kalium,
∘ 0,5-2,5 mmol/l Calcium,
∘ 0-2 mmol/l Magnesium,
∘ 100-150 mmol/l Chlorid,
∘ 0-500 mg/l Harnstoff,
∘ 0-100 mg/l Harnsäure,
∘ 0-500 mg/l Phosphat,
∘ 0-8 g/l Kohlenhydrate,
∘ 0-8 g/l Glucose,
∘ 0-100 mmol/l Laktat,
∘ 0-100 mmol/l Gluconat,
∘ 0-100 mg/l Citrat,
∘ 5-100 mmol/l Hydrogencarbonat,
∘ 0-1.0 g/l Dinatrium-ß-glycerophosphat-pentahydrat
∘ 0-15 g/l Gesamt-Eiweiß,
∘ 0-15 g/l Albumin,
∘ 0-2 g/l Spurenelemente,
∘ 0-2 mg/l Kupfer,
∘ 0-1 mg/l Selen,
∘ 0-5 mg/l Eisen,
∘ 0-1,5 mg/l Chrom,
∘ 0-2 mg/l Aluminium,
∘ 0-0,1 mg/l Cobalt,
∘ 0-70 mg/l Nickel,
∘ 0-1,5 mg/l Mangan,
∘ 0-4,5 mg/l Vanadium,
∘ 0-0,1 mg/l Arsen,
∘ 0-0,2 mg/l Rubidium
∘ 0-0,35 mg/l Barium,
∘ 0-10 mg/l Zink,
∘ 0-20 g/l Vitamin C,
∘ 0-1500 mg/l Vitamin A,
∘ 0-75 g/l Vitamin E
∘ 0-100 mg/l Vitamin K,
∘ 0-20 mg/l Vitamin B1,
∘ 0-20 mg/l Vitamin B2,
∘ 0-90 mg/l Vitamin B3,
∘ 0-25 mg/l Vitamin B5,
∘ 0-5 mg/l Vitamin B7,
∘ 0-7 mg/l Vitamin B9,
∘ 0-5 mg/l Vitamin B12,
∘ 0-5 mg/l Vitamin D,
∘ 0-60 g/l Hyaluronsäure und Salze der Hyaluronsäure,
∘ 0-120 g/l Surfactant,
∘ 0-350 g/l Fettsäuren
∘ Wasser umfassen und
zusammen vorzugsweise einen pH im Bereich von 7 bis 8 aufweisen.
B8) Eine oder mehrere Zusammensetzungen nach mindestens einer der vorangehenden Bestimmungen B1) bis B7), dadurch gekennzeichnet, dass mindestens eine der Zusammensetzung Glucose, Dextrose, Phospholipide, Surfactant, Lipide, Aminosäuren, Wachstumsfaktoren, Albumin, Proteine, Spurenelemente und/oder Vitamine umfasst.
B9) Eine oder mehrere Zusammensetzungen nach mindestens einer der vorangehenden Bestimmungen B1) bis B8), dadurch gekennzeichnet, dass die eine oder mehrere Zusammensetzungen als Serum oder Gel verwendet werden.
C1) Eine oder mehrere Zusammensetzungen zur Anwendung in einem Verfahren zur enteralen Ernährung oder zur enteralen Ernährungsergänzung eines menschlichen oder tierischen Körpers, dadurch gekennzeichnet, dass die eine der mehrere Zusammensetzungen zusammengenommen die folgenden Komponenten (pro 1000 ml)

| | |
|---|---|
| Natriumchlorid | 1-10 g |
| Natriumhydrogencarbonat | 0,001-3 g |
| Kaliumchlorid | 0-1 g |
| Natrium laktat | 0-2g |
| Calciumglukonat Monohydrat | 0-1 g |
| Magnesiumchlorid Hexahydrat | 0-1g |
| Dinatrium-β-glycerophosphat-pentahydrat | 0-1g |
| Zitronensäure Monohydrat | 0-0,5g |
| Harnstoff | 0-1g |
| Kupferchlorid Dihydrat | 0-2 mg |
| Natriumselenit Pentahydrat | 0-1 mg |
| Zinkchlorid | 0-10 mg |
| Surfactant | 0 - 120 g |
| sowie Wasser | |
| umfassen. | |

C2) Eine oder mehrere Zusammensetzungen nach Bestimmung C1), dadurch gekennzeichnet, dass mindestens eine der Zusammensetzungen Surfactant enthält.
C3) Eine oder mehrere Zusammensetzungen nach Bestimmung C1) oder C2), dadurch gekennzeichnet, dass die Zusammensetzungen, bezogen auf das Gesamtgewicht aller Zusammensetzungen, zusammengenommen die folgenden Komponenten
∘ 120-175 mmol/l Natrium,
∘ 3,5-40 mmol/l Kalium,
∘ 0,5-2,5 mmol/l Calcium,
∘ 0-2 mmol/l Magnesium,
∘ 100-150 mmol/l Chlorid,
∘ 0-500 mg/l Harnstoff,
∘ 0-100 mg/l Harnsäure,
∘ 0-500 mg/l Phosphat,
∘ 0-8 g/l Kohlenhydrate,
∘ 0-8 g/l Glucose,
∘ 0-100 mmol/l Laktat,
∘ 0-100 mmol/l Gluconat,
∘ 0-100 mg/l Citrat,
∘ 5-100 mmol/l Hydrogencarbonat,
∘ 0-1.0 g/l Dinatrium-ß-glycerophosphat-pentahydrat
∘ 0-15 g/l Gesamt-Eiweiß,
∘ 0-15 g/l Albumin,
∘ 0-2 g/l Spurenelemente,
∘ 0-2 mg/l Kupfer,
∘ 0-1 mg/l Selen,
∘ 0-5 mg/l Eisen,
∘ 0-1,5 mg/l Chrom,
∘ 0-2 mg/l Aluminium,
∘ 0-0,1 mg/l Cobalt,
∘ 0-70 mg/l Nickel,
∘ 0-1,5 mg/l Mangan,
∘ 0-4,5 mg/l Vanadium,
∘ 0-0,1 mg/l Arsen,
∘ 0-0,2 mg/l Rubidium
∘ 0-0,35 mg/l Barium,
∘ 0-10 mg/l Zink,
∘ 0-20 g/l Vitamin C,
∘ 0-1500 mg/l Vitamin A,
∘ 0-75 g/l Vitamin E
∘ 0-100 mg/l Vitamin K,
∘ 0-20 mg/l Vitamin B1,
∘ 0-20 mg/l Vitamin B2,
∘ 0-90 mg/l Vitamin B3,
∘ 0-25 mg/l Vitamin B5,
∘ 0-5 mg/l Vitamin B7,
∘ 0-7 mg/l Vitamin B9,
∘ 0-5 mg/l Vitamin B12,
∘ 0-5 mg/l Vitamin D,
∘ 0-120 g/l Surfactant,
∘ 0-350 g/l Fettsäuren
∘ Wasser umfassen und
zusammen vorzugsweise einen pH im Bereich von 7 bis 8 aufweisen.
C4) Eine oder mehrere Zusammensetzungen nach mindestens einer der vorangehenden Bestimmungen C1) bis C3), dadurch gekennzeichnet, dass mindestens eine der Zusammensetzung Glucose, Dextrose, Phospholipide, Surfactant, Lipide, Aminosäuren, Wachstumsfaktoren, Albumin, Proteine, Spurenelemente und/oder Vitamine umfasst.
C5) Eine oder mehrere Zusammensetzungen nach mindestens einer der vorangehenden Bestimmungen C1) bis C4), dadurch gekennzeichnet, dass die eine oder mehrere Zusammensetzungen als Serum oder Gel verwendet werden.
D1) Eine oder mehrere Zusammensetzungen zur Anwendung in einem Verfahren zur Behandlung von kapillararmen Schichten mit einer Kapillardichte von 0 bis 50/mm² zur Anwendung bei der Amnioninfusion bei einem menschlichen oder tierischen Körper, dadurch gekennzeichnet, dass die eine der mehrere Zusammensetzungen zusammengenommen die folgenden Komponenten (pro 1000 ml)

| | |
|---|---|
| Natriumchlorid | 1-10 g |
| Natriumhydrogencarbonat | 0,001-3 g |
| Kaliumchlorid | 0-1 g |
| Natrium laktat | 0-2g |
| Calciumglukonat Monohydrat | 0-1 g |
| Magnesiumchlorid Hexahydrat | 0-1g |
| Dinatrium-β-glycerophosphat-pentahydrat | 0-1g |
| Zitronensäure Monohydrat | 0-0,5g |
| Harnstoff | 0-1g |
| Kupferchlorid Dihydrat | 0-2 mg |
| Natriumselenit Pentahydrat | 0-1 mg |
| Zinkchlorid | 0-10 mg |
| Surfactant | 0 - 120 g |
| sowie Wasser | |
| umfassen. | |

D2) Eine oder mehrere Zusammensetzungen nach Bestimmung D1) zur Behandlung von kapillararmen Schichten mit einer Kapillardichte von 0 bis 30/mm².
D3) Eine oder mehrere Zusammensetzungen nach Bestimmung D1) oder D2) zur Behandlung von kapillarlosen Schichten.
D4) Eine oder mehrere Zusammensetzungen nach mindestens einer der vorangehenden Bestimmungen D1) bis D3) zur Behandlung eines Oligo-Anhydramnion.
D5) Eine oder mehrere Zusammensetzungen nach mindestens einer der vorangehenden Bestimmungen D1) bis D4) zur Behandlung eines vorzeitigen Blasensprungs.
D6) Eine oder mehrere Zusammensetzungen nach mindestens einer der vorangehenden Bestimmungen D1) bis D5) zur Anwendung bei einer kontinuierlichen Amnioninfusion.
D7) Eine oder mehrere Zusammensetzungen nach mindestens einer der vorangehenden Bestimmungen D1) bis D6), dadurch gekennzeichnet, dass mindestens eine der Zusammensetzungen Surfactant enthält.
D8) Eine oder mehrere Zusammensetzungen nach mindestens einer der vorangehenden Bestimmungen D1) bis D7), dadurch gekennzeichnet, dass die Zusammensetzungen, bezogen auf das Gesamtgewicht aller Zusammensetzungen, zusammengenommen die folgenden Komponenten
∘ 120-175 mmol/l Natrium,
∘ 3,5-40 mmol/l Kalium,
∘ 0,5-2,5 mmol/l Calcium,
∘ 0-2 mmol/l Magnesium,
∘ 100-150 mmol/l Chlorid,
∘ 0-500 mg/l Harnstoff,
∘ 0-100 mg/l Harnsäure,
∘ 0-500 mg/l Phosphat,
∘ 0-8 g/l Kohlenhydrate,
∘ 0-8 g/l Glucose,
∘ 0-100 mmol/l Laktat,
∘ 0-100 mmol/l Gluconat,
∘ 0-100 mg/l Citrat,
∘ 5-100 mmol/l Hydrogencarbonat,
∘ 0-1.0 g/l Dinatrium-ß-glycerophosphat-pentahydrat
∘ 0-15 g/l Gesamt-Eiweiß,
∘ 0-15 g/l Albumin,
∘ 0-2 g/l Spurenelemente,
∘ 0-2 mg/l Kupfer,
∘ 0-1 mg/l Selen,
∘ 0-5 mg/l Eisen,
∘ 0-1,5 mg/l Chrom,
∘ 0-2 mg/l Aluminium,
∘ 0-0,1 mg/l Cobalt,
∘ 0-70 mg/l Nickel,
∘ 0-1,5 mg/l Mangan,
∘ 0-4,5 mg/l Vanadium,
∘ 0-0,1 mg/l Arsen,
∘ 0-0,2 mg/l Rubidium
∘ 0-0,35 mg/l Barium,
∘ 0-10 mg/l Zink,
∘ 0-20 g/l Vitamin C,
∘ 0-1500 mg/l Vitamin A,
∘ 0-75 g/l Vitamin E
∘ 0-100 mg/l Vitamin K,
∘ 0-20 mg/l Vitamin B1,
∘ 0-20 mg/l Vitamin B2,
∘ 0-90 mg/l Vitamin B3,
∘ 0-25 mg/l Vitamin B5,
∘ 0-5 mg/l Vitamin B7,
∘ 0-7 mg/l Vitamin B9,
∘ 0-5 mg/l Vitamin B12,
∘ 0-5 mg/l Vitamin D,
∘ 0-120 g/l Surfactant,
∘ 0-350 g/l Fettsäuren
∘ Wasser umfassen und
zusammen einen pH im Bereich von 7 bis 8 aufweisen.
D9) Eine oder mehrere Zusammensetzungen nach mindestens einer der vorangehenden Bestimmungen D1) bis D8), dadurch gekennzeichnet, dass mindestens eine der Zusammensetzung Glucose, Dextrose, Phospholipide, Surfactant, Lipide, Aminosäuren, Wachstumsfaktoren, Albumin, Proteine, Spurenelemente und/oder Vitamine umfasst.
D10) Eine oder mehrere Zusammensetzungen nach mindestens einer der vorangehenden Bestimmungen D1) bis D9), dadurch gekennzeichnet, dass mindestens eine der Zusammensetzungen ein Antibiotikum umfasst.
D11) Eine oder mehrere Zusammensetzungen nach Bestimmung D10), dadurch gekennzeichnet, dass die Zusammensetzung ein β-Lactam-Antibiotikum, ein Glykopeptid, ein Polyketid, ein Tetracyclin, ein Glycylcyclin, ein Makrolid-Antibiotikum, ein Ketolid, ein Lincosamid, ein Aminoglykosid-Antibiotikum, ein Polypeptid-Antibiotikum, ein Lipopeptid-Antibiotikum, ein Epoxid-Antibiotikum, ein Chinolon-Antibiotikum, ein Streptogramin, ein Sulfonamid, ein Oxazolidinon, ein Ansamycin und/oder ein Nitroimidazol oder Azithromycin umfasst.
D12) Eine oder mehrere Zusammensetzungen nach mindestens einer der vorangehenden Bestimmungen D1) bis D11), dadurch gekennzeichnet, dass die eine oder mehrere Zusammensetzungen als Serum oder Gel verwendet werden.
D13) Eine oder mehrere Zusammensetzungen nach mindestens einen der vorangehenden Bestimmungen D1) bis D12) zur Behandlung von kapillararmen Schichten in einem künstlichen Gebärmutter-System.
D14) Eine oder mehrere Zusammensetzungen nach mindestens einen der vorangehenden Bestimmungen D1) bis D13) zur Behandlung von kapillararmen Schichten, insbesondere Amnion und Kornea, vor und nach der einer Transplantation.
F1) Eine oder mehrere Zusammensetzungen zur Anwendung in einem Verfahren zur Behandlung von kapillararmen Schichten mit einer Kapillardichte von 0 bis 50/mm² zur Behandlung von Krankheiten des bronchopulmonalen Systems und/oder der Atemwege eines menschlichen oder tierischen Körpers, dadurch gekennzeichnet, dass die eine der mehrere Zusammensetzungen zusammengenommen die folgenden Komponenten (pro 1000 ml)

| | |
|---|---|
| Natriumchlorid | 1-10 g |
| Natriumhydrogencarbonat | 0,001-3 g |
| Kaliumchlorid | 0-1 g |
| Natrium laktat | 0-2g |
| Calciumglukonat Monohydrat | 0-1 g |
| Magnesiumchlorid Hexahydrat | 0-1g |
| Dinatrium-β-glycerophosphat-pentahydrat | 0-1g |
| Zitronensäure Monohydrat | 0-0,5g |
| Harnstoff | 0-1g |
| Kupferchlorid Dihydrat | 0-2 mg |
| Natriumselenit Pentahydrat | 0-1 mg |
| Zinkchlorid | 0-10 mg |
| Hyaluronsäure | |
| und Salze der Hyaluronsäure | 0 bis 60 g |
| Surfactant | 0 - 120 g |
| sowie Wasser | |
| umfassen. | |

F2) Eine oder mehrere Zusammensetzungen nach Bestimmung F1) zur Behandlung von kapillararmen Schichten mit einer Kapillardichte von 0 bis 30/mm².
F3) Eine oder mehrere Zusammensetzungen nach Bestimmung F1) oder F2) zur Behandlung von kapillarlosen Schichten.
F4) Eine oder mehrere Zusammensetzungen nach mindestens einer der vorangehenden Bestimmungen F1) bis F3) zur Behandlung von Patienten mittels Inhalation, insbesondere zur Behandlung eines RDS-Syndroms.
F5) Eine oder mehrere Zusammensetzungen nach mindestens einer der vorangehenden Bestimmungen F1) bis F4), dadurch gekennzeichnet, dass mindestens eine der Zusammensetzungen Hyaluronsäure oder Salze der Hyaluronsäure enthält.
F6) Eine oder mehrere Zusammensetzungen nach mindestens einer der vorangehenden Bestimmungen F1) bis F5), dadurch gekennzeichnet, dass mindestens eine der Zusammensetzungen Surfactant enthält.
F7) Eine oder mehrere Zusammensetzungen nach mindestens einer der vorangehenden Bestimmungen F1) bis F6), dadurch gekennzeichnet, dass die Zusammensetzungen, bezogen auf das Gesamtgewicht aller Zusammensetzungen, zusammengenommen die folgenden Komponenten
∘ 120-175 mmol/l Natrium,
∘ 3,5-40 mmol/l Kalium,
∘ 0,5-2,5 mmol/l Calcium,
∘ 0-2 mmol/l Magnesium,
∘ 100-150 mmol/l Chlorid,
∘ 0-500 mg/l Harnstoff,
∘ 0-100 mg/l Harnsäure,
∘ 0-500 mg/l Phosphat,
∘ 0-8 g/l Kohlenhydrate,
∘ 0-8 g/l Glucose,
∘ 0-100 mmol/l Laktat,
∘ 0-100 mmol/l Gluconat,
∘ 0-100 mg/l Citrat,
∘ 5-100 mmol/l Hydrogencarbonat,
∘ 0-1.0 g/l Dinatrium-ß-glycerophosphat-pentahydrat
∘ 0-15 g/l Gesamt-Eiweiß,
∘ 0-15 g/l Albumin,
∘ 0-2 g/l Spurenelemente,
∘ 0-2 mg/l Kupfer,
∘ 0-1 mg/l Selen,
∘ 0-5 mg/l Eisen,
∘ 0-1,5 mg/l Chrom,
∘ 0-2 mg/l Aluminium,
∘ 0-0,1 mg/l Cobalt,
∘ 0-70 mg/l Nickel,
∘ 0-1,5 mg/l Mangan,
∘ 0-4,5 mg/l Vanadium,
∘ 0-0,1 mg/l Arsen,
∘ 0-0,2 mg/l Rubidium
∘ 0-0,35 mg/l Barium,
∘ 0-10 mg/l Zink,
∘ 0-20 g/l Vitamin C,
∘ 0-1500 mg/l Vitamin A,
∘ 0-75 g/l Vitamin E
∘ 0-100 mg/l Vitamin K,
∘ 0-20 mg/l Vitamin B1,
∘ 0-20 mg/l Vitamin B2,
∘ 0-90 mg/l Vitamin B3,
∘ 0-25 mg/l Vitamin B5,
∘ 0-5 mg/l Vitamin B7,
∘ 0-7 mg/l Vitamin B9,
∘ 0-5 mg/l Vitamin B12,
∘ 0-5 mg/l Vitamin D,
∘ 0-60 g/l Hyaluronsäure und Salze der Hyaluronsäure,
∘ 0-120 g/l Surfactant,
∘ 0-350 g/l Fettsäuren
∘ Wasser umfassen und
zusammen vorzugsweise einen pH im Bereich von 7 bis 8 aufweisen.
F8) Eine oder mehrere Zusammensetzungen nach mindestens einer der vorangehenden Bestimmungen F1) bis F7), dadurch gekennzeichnet, dass mindestens eine der Zusammensetzung Glucose, Dextrose, Phospholipide, Surfactant, Lipide, Aminosäuren, Wachstumsfaktoren, Albumin, Proteine, Spurenelemente und/oder Vitamine umfasst.
F9) Eine oder mehrere Zusammensetzungen nach mindestens einer der vorangehenden Bestimmungen F1) bis F8), dadurch gekennzeichnet, dass die eine oder mehrere Zusammensetzungen als Serum oder Gel verwendet werden.
G1) Eine oder mehrere Zusammensetzungen zur Anwendung in einem Verfahren zur Behandlung von kapillararmen Schichten mit einer Kapillardichte von 0 bis 50/mm² zur Behandlung von Hauterkrankungen eines menschlichen oder tierischen Körpers, dadurch gekennzeichnet, dass die eine der mehrere Zusammensetzungen zusammengenommen die folgenden Komponenten (pro 1000 ml)

| | |
|---|---|
| Natriumchlorid | 1-10 g |
| Natriumhydrogencarbonat | 0,001-3 g |
| Kaliumchlorid | 0-1 g |
| Natrium laktat | 0-2g |
| Calciumglukonat Monohydrat | 0-1 g |
| Magnesiumchlorid Hexahydrat | 0-1g |
| Dinatrium-β-glycerophosphat-pentahydrat | 0-1g |
| Zitronensäure Monohydrat | 0-0,5g |
| Harnstoff | 0-1g |
| Kupferchlorid Dihydrat | 0-2 mg |
| Natriumselenit Pentahydrat | 0-1 mg |
| Zinkchlorid | 0-10 mg |
| Hyaluronsäure | |
| und Salze der Hyaluronsäure | 0 bis 60 g |
| Surfactant | 0 - 120 g |
| sowie Wasser | |
| umfassen. | |

G2) Eine oder mehrere Zusammensetzungen nach Bestimmung G1) zur Behandlung von kapillararmen Schichten mit einer Kapillardichte von 0 bis 30/mm².
G3) Eine oder mehrere Zusammensetzungen nach Bestimmung G1) oder G2) zur Behandlung von kapillarlosen Schichten.
G4) Eine oder mehrere Zusammensetzungen nach mindestens einer der vorangehenden Bestimmungen G1) bis G3) zur Behandlung von Patienten durch Auftragung der einen oder mehreren Zusammensetzungen auf die zu behandelnde Haut.
G5) Eine oder mehrere Zusammensetzungen nach mindestens einer der vorangehenden Bestimmungen G1) bis G4) zur Versorgung von Haut und/oder Amnion-Transplantaten.
G6) Eine oder mehrere Zusammensetzungen nach mindestens einer der vorangehenden Bestimmungen G1) bis G5), dadurch gekennzeichnet, dass mindestens eine der Zusammensetzungen Hyaluronsäure oder Salze der Hyaluronsäure enthält.
G7) Eine oder mehrere Zusammensetzungen nach mindestens einer der vorangehenden Bestimmungen G1) bis G6), dadurch gekennzeichnet, dass mindestens eine der Zusammensetzungen Surfactant enthält.
G8) Eine oder mehrere Zusammensetzungen nach mindestens einer der vorangehenden Bestimmungen G1) bis G7), dadurch gekennzeichnet, dass die Zusammensetzungen, bezogen auf das Gesamtgewicht aller Zusammensetzungen, zusammengenommen die folgenden Komponenten
∘ 120-175 mmol/l Natrium,
∘ 3,5-40 mmol/l Kalium,
∘ 0,5-2,5 mmol/l Calcium,
∘ 0-2 mmol/l Magnesium,
∘ 100-150 mmol/l Chlorid,
∘ 0-500 mg/l Harnstoff,
∘ 0-100 mg/l Harnsäure,
∘ 0-500 mg/l Phosphat,
∘ 0-8 g/l Kohlenhydrate,
∘ 0-8 g/l Glucose,
∘ 0-100 mmol/l Laktat,
∘ 0-100 mmol/l Gluconat,
∘ 0-100 mg/l Citrat,
∘ 5-100 mmol/l Hydrogencarbonat,
∘ 0-1.0 g/l Dinatrium-ß-glycerophosphat-pentahydrat
∘ 0-15 g/l Gesamt-Eiweiß,
∘ 0-15 g/l Albumin,
∘ 0-2 g/l Spurenelemente,
∘ 0-2 mg/l Kupfer,
∘ 0-1 mg/l Selen,
∘ 0-5 mg/l Eisen,
∘ 0-1,5 mg/l Chrom,
∘ 0-2 mg/l Aluminium,
∘ 0-0,1 mg/l Cobalt,
∘ 0-70 mg/l Nickel,
∘ 0-1,5 mg/l Mangan,
∘ 0-4,5 mg/l Vanadium,
∘ 0-0,1 mg/l Arsen,
∘ 0-0,2 mg/l Rubidium
∘ 0-0,35 mg/l Barium,
∘ 0-10 mg/l Zink,
∘ 0-20 g/l Vitamin C,
∘ 0-1500 mg/l Vitamin A,
∘ 0-75 g/l Vitamin E
∘ 0-100 mg/l Vitamin K,
∘ 0-20 mg/l Vitamin B1,
∘ 0-20 mg/l Vitamin B2,
∘ 0-90 mg/l Vitamin B3,
∘ 0-25 mg/l Vitamin B5,
∘ 0-5 mg/l Vitamin B7,
∘ 0-7 mg/l Vitamin B9,
∘ 0-5 mg/l Vitamin B12,
∘ 0-5 mg/l Vitamin D,
∘ 0-60 g/l Hyaluronsäure und Salze der Hyaluronsäure,
∘ 0-120 g/l Surfactant,
∘ 0-350 g/l Fettsäuren
∘ Wasser umfassen und
zusammen vorzugsweise einen pH im Bereich von 7 bis 8 aufweisen.
G9) Eine oder mehrere Zusammensetzungen nach mindestens einer der vorangehenden Bestimmungen G1) bis G8), dadurch gekennzeichnet, dass mindestens eine der Zusammensetzung Glucose, Dextrose, Phospholipide, Surfactant, Lipide, Aminosäuren, Wachstumsfaktoren, Albumin, Proteine, Spurenelemente und/oder Vitamine umfasst.
G10)Eine oder mehrere Zusammensetzungen nach mindestens einer der vorangehenden Bestimmungen G1) bis G9), dadurch gekennzeichnet, dass die eine oder mehrere Zusammensetzungen als Serum oder Gel verwendet werden.

## Patentansprüche

1. Eine oder mehrere Zusammensetzungen zur Anwendung in einem Verfahren zur enteralen Ernährung, zur enteralen Ernährungsergänzung und/oder zur Behandlung von kapillararmen Schichten mit einer Kapillardichte von 0 bis 50/mm², **dadurch gekennzeichnet, dass** die eine der mehrere Zusammensetzungen zusammengenommen die folgenden Komponenten (pro 1000 ml)
| | |
|---|---|
| Natriumchlorid | 1-10 g |
| Natriumhydrogencarbonat | 0,001-3 g |
| Kaliumchlorid | 0-1 g |
| Natrium laktat | 0-2g |
| Calciumglukonat Monohydrat | 0-3 g |
| Magnesiumchlorid Hexahydrat | 0-1g |
| Dinatrium-β-glycerophosphat-pentahydrat | 0-1g |
| Zitronensäure Monohydrat | 0-0,5g |
| Harnstoff | 0-1g |
| Kupferchlorid Dihydrat | 0-2 mg |
| Natriumselenit Pentahydrat | 0-1 mg |
| Zinkchlorid | 0-10 mg |
| Hyaluronsäure | |
| und Salze der Hyaluronsäure | 0 bis 60 g |
| Surfactant | 0 - 120 g |
| sowie Wasser | |
| umfassen. | |

2. Eine oder mehrere Zusammensetzungen nach Anspruch 1 zur Behandlung von kapillararmen Schichten mit einer Kapillardichte von 0 bis 30/mm².

3. Eine oder mehrere Zusammensetzungen nach Anspruch 1 oder 2 zur Behandlung von kapillarlosen Schichten.

4. Eine oder mehrere Zusammensetzungen nach mindestens einen der vorangehenden Ansprüche, **dadurch gekennzeichnet, dass** mindestens eine der Zusammensetzungen Hyaluronsäure oder Salze der Hyaluronsäure enthält.

5. Eine oder mehrere Zusammensetzungen nach mindestens einen der vorangehenden Ansprüche, **dadurch gekennzeichnet, dass** mindestens eine der Zusammensetzungen Surfactant enthält.

6. Eine oder mehrere Zusammensetzungen nach mindestens einen der vorangehenden Ansprüche, **dadurch gekennzeichnet, dass** die Zusammensetzungen, bezogen auf das Gesamtgewicht aller Zusammensetzungen, zusammengenommen die folgenden Komponenten
∘ 120-175 mmol/l Natrium,
∘ 3,5-40 mmol/l Kalium,
∘ 0,5-2,5 mmol/l Calcium,
∘ 0-2 mmol/l Magnesium,
∘ 100-150 mmol/l Chlorid,
∘ 0-500 mg/l Harnstoff,
∘ 0-100 mg/l Harnsäure,
∘ 0-500 mg/l Phosphat,
∘ 0-8 g/l Kohlenhydrate,
∘ 0-8 g/l Glucose,
∘ 0-100 mmol/l Laktat,
∘ 0-100 mmol/l Gluconat,
∘ 0-100 mg/l Citrat,
∘ 5-100 mmol/l Hydrogencarbonat,
∘ 0-1.0 g/l Dinatrium-ß-glycerophosphat-pentahydrat
∘ 0-15 g/l Gesamt-Eiweiß,
∘ 0-15 g/l Albumin,
∘ 0-2 g/l Spurenelemente,
∘ 0-2 mg/l Kupfer,
∘ 0-1 mg/l Selen,
∘ 0-5 mg/l Eisen,
∘ 0-1,5 mg/l Chrom,
∘ 0-2 mg/l Aluminium,
∘ 0-0,1 mg/l Cobalt,
∘ 0-70 mg/l Nickel,
∘ 0-1,5 mg/l Mangan,
∘ 0-4,5 mg/l Vanadium,
∘ 0-0,1 mg/l Arsen,
∘ 0-0,2 mg/l Rubidium
∘ 0-0,35 mg/l Barium,
∘ 0-10 mg/l Zink,
∘ 0-20 g/l Vitamin C,
∘ 0-1500 mg/l Vitamin A,
∘ 0-75 g/l Vitamin E
∘ 0-100 mg/l Vitamin K,
∘ 0-20 mg/l Vitamin B1,
∘ 0-20 mg/l Vitamin B2,
∘ 0-90 mg/l Vitamin B3,
∘ 0-25 mg/l Vitamin B5,
∘ 0-5 mg/l Vitamin B7,
∘ 0-7 mg/l Vitamin B9,
∘ 0-5 mg/l Vitamin B12,
∘ 0-5 mg/l Vitamin D,
∘ 0-60 g/l Hyaluronsäure und Salze der Hyaluronsäure,
∘ 0-120 g/l Surfactant,
∘ 0-350 g/l Fettsäuren
∘ Wasser umfassen.

7. Eine oder mehrere Zusammensetzungen nach mindestens einen der vorangehenden Ansprüche, **dadurch gekennzeichnet, dass** mindestens eine der Zusammensetzung Glucose, Dextrose, Phospholipide, Surfactant, Lipide, Aminosäuren, Wachstumsfaktoren, Albumin, Proteine, Spurenelemente und/oder Vitamine umfasst.

8. Eine oder mehrere Zusammensetzungen nach mindestens einen der vorangehenden Ansprüche, **dadurch gekennzeichnet, dass** mindestens eine der Zusammensetzungen ein Antibiotikum umfasst.

9. Eine oder mehrere Zusammensetzungen nach Anspruch 8, **dadurch gekennzeichnet, dass** die Zusammensetzung ein β-Lactam-Antibiotikum, ein Glykopeptid, ein Polyketid, ein Tetracyclin, ein Glycylcyclin, ein Makrolid-Antibiotikum, ein Ketolid, ein Lincosamid, ein Aminoglykosid-Antibiotikum, ein Polypeptid-Antibiotikum, ein Lipopeptid-Antibiotikum, ein Epoxid-Antibiotikum, ein Chinolon-Antibiotikum, ein Streptogramin, ein Sulfonamid, ein Oxazolidinon, ein Ansamycin und/oder ein Nitroimidazol oder Azithromycin umfasst.

10. Eine oder mehrere Zusammensetzungen nach mindestens einen der vorangehenden Ansprüche, **dadurch gekennzeichnet, dass** die eine oder mehrere Zusammensetzungen als Serum oder Gel verwendet werden.

11. Eine oder mehrere Zusammensetzungen nach mindestens einen der vorangehenden Ansprüche zur Behandlung von Krankheiten des menschlichen oder tierischen Auges, insbesondere zur Behandlung von Patienten mit Sicca-Syndrom, einer Störung der Meibomdrüsen und/oder mit einer Störung infolge von Kontaktlinsentragen; zur enteralen Ernährung eines menschlichen oder tierischen Körpers; zur Anwendung bei der Amnioninfusion bei einem menschlichen oder tierischen Körper, bevorzugt zur Behandlung eines Oligo-Anhydramnion, insbesondere eines vorzeitigen Blasensprungs, vorzugsweise zur Anwendung bei einer kontinuierlichen Amnioninfusion; zur Behandlung von Krankheiten des bronchopulmonalen Systems und/oder der Atemwege eines menschlichen oder tierischen Körpers, bevorzugt zur Behandlung von Patienten mittels Inhalation, insbesondere zur Behandlung eines RDS-Syndroms; zur Behandlung von Hauterkrankungen eines menschlichen oder tierischen Körpers, vorzugsweise zur Behandlung von Patienten durch Auftragung der einen oder mehreren Zusammensetzungen auf die zu behandelnde Haut, insbesondere zur Versorgung von Amnion-Transplantaten.

12. Eine oder mehrere Zusammensetzungen nach mindestens einen der vorangehenden Ansprüche zur Behandlung von kapillararmen Schichten in einem künstlichen Gebärmutter-System oder Reservoir.

13. Eine oder mehrere Zusammensetzungen nach mindestens einen der vorangehenden Ansprüche zur Behandlung von kapillararmen oder kapillarlosen Schichten und Geweben, insbesondere Amnion und Kornea, vor und nach der einer Transplantation.
